# EUROPEAN PATENT APPLICATION

(11) **EP 4 810 492 A1**
(43) Date of publication of application: **23.09.2026**
(21) Application number: 24890764.4
(22) Date of filing: 14.11.2024
(51) Int. Cl.: C07D 471/04, C07D 491/08, C07D 519/00, A61K 31/444, A61K 31/5377, A61P 29/00, A61P 35/00

(54) **NMT INHIBITOR, PREPARATION METHOD THEREFOR, AND USE THEREOF**

(30) Priority: 16.11.2023 CN 202311530210
(71) Applicant: Nanjing Synnocare Pharmaceutical Technology Co., Ltd., Nanjing, Jiangsu 211122 (CN); Tianjin Synnocare Biomedical Technology Co., Ltd., Binhai New Area, Tianjin 300457 (CN)
(72) Inventor: FAN, Houxing, Nanjing, Jiangsu 211122 (CN); JIANG, Qiangqiang, Nanjing, Jiangsu 211122 (CN); WANG, Tiancai, Nanjing, Jiangsu 211122 (CN)
(74) Representative: Lavoix
(86) International application number: PCT/CN2024/132019
(87) International publication number: WO 2025/103409

(57) **Abstract**

The present invention provides an NMT inhibitor, a preparation method therefor, and a use thereof, and specifically relates to a compound as shown in formula (1) and a preparation method therefor, and a composition containing the compound as shown in formula (1) and/or a pharmaceutically acceptable salt thereof, a preparation method therefor and a use thereof as an NMT inhibitor in the preparation of a drug for NMT-mediated related diseases.

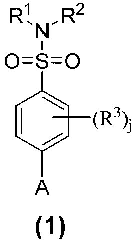

## Description

### FIELD OF THE INVENTION

The invention belongs to the field of medicinal chemistry, and more specifically, relates to a kind of NMT inhibitors as shown in Formula (1), and preparation methods therefor, as well as the use of these compounds for preparing pharmaceutial compositions for treating, regulating, and/or preventing diseases mediated by NMT.

### BACKGROUND

NMT (N-myristoyltransferase) is an enzyme that catalyzes the myristoylation of over 100 proteins in human cells and affects downstream related signaling pathways. In humans, protein myristoylation is mediated by two ubiquitously expressed *N*-myristoyltransferases, NMT1 and NMT2. Myristoylation is a co- and post-translational modification reaction in eukaryotes, where, through the action of NMT1 and NMT2, a myristoyl group is transferred to the *N*-terminal glycine of substrate proteins [Cell Death and Disease, 2018, 9 (12): 1143]. It acts like a "switch", triggering various reversible protein-membrane and protein-protein interactions.

NMT1 is highly expressed in various tumor tissues. In liver cancer, NMT1 is associated with poor prognosis; patients with high NMT1 expression have shorter survival times compared to those with low expression. The expression of NMT1 holds significant clinical importance. Detection in primary breast cancer tissues and adjacent tissues from 20 breast cancer patients showed that NMT1 expression was significantly higher in breast cancer tissues compared to adjacent tissues, particularly markedly elevated in triple-negative breast cancer tissues [Cell Death and Disease, 2018, 9 (12): 1143]. Elevated NMT1 is associated with tumor development and shorter patient survival. In colorectal cancer patients, NMT1 expression increased in peripheral blood and bone marrow and can serve as a diagnostic marker for colorectal cancer [J Transl Med. 2007, 5: 58]. High NMT1 expression is associated with poor prognosis in ovarian cancer and with the progression of liver cancer, brain cancer, etc. [Annals of medicine, 2023, 55 (1): 1422-1430].

NMT1 catalyzes the formation of myristoylated proteins from its substrate proteins, activating downstream signaling pathways such as NF-κB, C-Myc, and ERK, thereby promoting tumor cell proliferation, migration, and metastasis. Inhibiting NMT1 can induce cell cycle arrest and suppress cell proliferation and malignant growth [Annals of medicine, 2023, 55 (1): 1422-1430]. NMT1 is highly expressed in various tumors and is associated with tumor stage, prognosis, and survival, making it a potential target for precision cancer therapy. NMT inhibitors can significantly inhibit hematological tumor cells, including B lymphocyte activity, inhibit myristoylation in lymphoma cells, and suppress B-cell receptor signaling, leading to cell death and inhibiting tumor growth in mouse tumor models [Nature communications, 2020, 11 (1): 5348].

Although some small molecule NMT inhibitors have been disclosed, there remains a need to develop novel compounds with better activity and pharmacokinetic properties. The NMT inhibitor compounds of the present invention, having the structure represented by general formula (1), demonstrate excellent activity and profiles, potentially providing new options for precision cancer therapy.

### SUMMARY OF THE INVENTION

The objective of the present invention is to provide a novel NMT inhibitor with potent activity, as well as preparation therefor and uses thereof.

In the first aspect of the present invention, it provides a compound as shown in Formula (1), or an isomer, crystal form, pharmaceutically acceptable salt, hydrate, or solvate thereof: 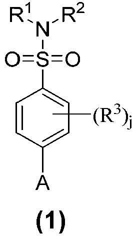 wherein:
A is selected from the structure consisting of: 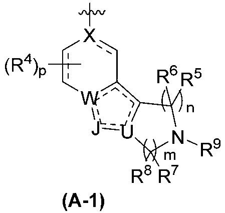 or 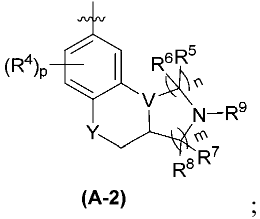
Wherein, the dashed line represents a chemical bond or absent; and 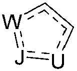 is an aromatic ring;
X is selected from the group consisting of: CH or N, when X is N, the dashed bond attached to X is absent;
U is selected from the group consisting of: CH or N;
W is selected from the group consisting of: CH or N;
J is selected from the group consisting of: O, S or NH;
V is selected from the group consisting of: CH or N;
Y is selected from the group consisting of: O, S, NH or N(C₁₋₃ alkyl);
m is 1 or 2;
n is 1 or 2;
p is 1, 2 or 3;
R⁴, R⁵, R⁶, R⁷ and R⁸ are each independently selected from the group consisting of: H, halogen, CN, C₁₋₆ alkyl, C₃₋₆ cycloalkyl, C₁₋₆ haloalkyl or C₁₋₆ alkoxyl;
R⁹ is selected from the group consisting of: H or C₁₋₃ alkyl;
j is 1, 2, 3 or 4;
R¹ is selected from the group consisting of: H, C₁₋₃ alkyl or C₁₋₃ haloalkyl;
R² represents a 5- to 7-membered heteroaryl, which may be further substituted by 1, 2, or 3 R^{2a}, wherein each R^{2a} is independently selected from the group consisting of: cyano, NO₂, - OR^{a}, -C(=O)R^{a}, -NR^{c}R^{d}, -C(=O)NR^{c}R^{d}, -NR^{b}C(=O)NR^{c}R^{d}, -NR^{b}C(=O)R^{a}, C₁₋₆ alkyl, C₂₋₆ alkenyl, C₂₋₆ alkynyl, C₃₋₆ cycloalkyl, or 4- to 7-membered heterocycloalkyl; wherein the alkyl, alkenyl, alkynyl, cycloalkyl, and heterocycloalkyl can be optionally substituted by one or more R^{2b};
each R^{2b} is independently selected from the group consisting of: halogen, CN, NO₂, OH, SH, -OR^{a}, -NR^{c}R^{d}, -C(=O)NR^{c}R^{d}, -NR^{b}C(=O)NR^{c}R^{d}, -NR^{b}C(=O)R^{a}, -NR^{b}S(=O)₂R^{a}, -C(=O)R^{a}, -OC(=O)R^{a}, -C(=O)OR^{b}, -NR^{b}C(=O)OR^{b}, -OC(=O)NR^{c}R^{d}, C₁₋₆ alkyl, C₂₋₆ alkenyl, C₂₋₆ alkynyl, C₁₋₆ alkoxyl, C₃₋₆ cycloalkyl or 4- to 7-membered heterocycloalkyl; wherein the alkyl, alkenyl, alkynyl, alkoxy, cycloalkyl and heterocycloalkyl can be optionally substituted by one or more R;
or two R^{2b} substituents on the same atom jointly form =O;
R^{a} is independently selected from the group consisting of: C₁₋₆ alkyl, C₂₋₆ alkenyl, C₂₋₆ alkynyl, C₁₋₆ alkoxyl, C₁₋₆ haloalkyl, hydroxy substituted C₁₋₆ alkyl, amino substituted C₁₋₆ alkyl, C₃₋₆ cycloalkyl or 4- to 7-membered heterocycloalkyl; wherein the alkyl, alkenyl, alkynyl, alkoxyl, cycloalkyl and heterocycloalkyl can be optionally substituted by one or more R;
R^{b} is independently selected from the group consisting of: H, C₁₋₆ alkyl, C₂₋₆ alkenyl, C₂₋₆ alkynyl, C₁₋₆ alkoxyl, C₁₋₆ haloalkyl, hydroxy substituted C₁₋₆ alkyl, amino substituted C₁₋₆ alkyl, C₃₋₆ cycloalkyl or 4- to 7-membered heterocycloalkyl; wherein the alkyl, alkenyl, alkynyl, alkoxyl, cycloalkyl and heterocycloalkyl can be optionally substituted by one or more R;
R^{c} and R^{d} are each independently selected from the group consisting of: H, C₁₋₆ alkyl, C₂₋₆ alkenyl, C₂₋₆ alkynyl, C₁₋₆ alkoxyl, C₁₋₆ haloalkyl, hydroxy substituted C₁₋₆ alkyl, amino substituted C₁₋₆ alkyl, C₃₋₆ cycloalkyl or 4- to 7-membered heterocycloalkyl; wherein the alkyl, alkenyl, alkynyl, alkoxyl, cycloalkyl and heterocycloalkyl can be optionally substituted by one or more R;
or R^{c} and R^{d} are taken together with the atoms to which they are bound to form a 4- to 7-membered heterocycloalkyl; wherein the heterocycloalkyl can be optionally substituted by one or more R;
each R is independently selected from the group consisting of: halogen, CN, OH, NH₂, NHCH₃, N(CH₃)₂, -C(=O)NH₂, -C(=O)NHCH₃, -C(=O)N(CH₃)₂, C₁₋₆ alkyl, C₁₋₆ alkoxyl, C₁₋₆ haloalkyl, hydroxy substituted C₁₋₆ alkyl, cyano substituted C₁₋₆ alkyl, amino substituted C₁₋₆ alkyl or C₃₋₆ cycloalkyl;
each R³ is independently selected from the group consisting of: H, halogen, CN, C₁₋₆ alkyl, C₃₋₆ cycloalkyl, C₁₋₆ haloalkyl or C₁₋₆ alkoxyl.

In another preferred embodiment, wherein the compound of formula (1) has a structure represented by the following (2-1), (2-2) or (2-3): 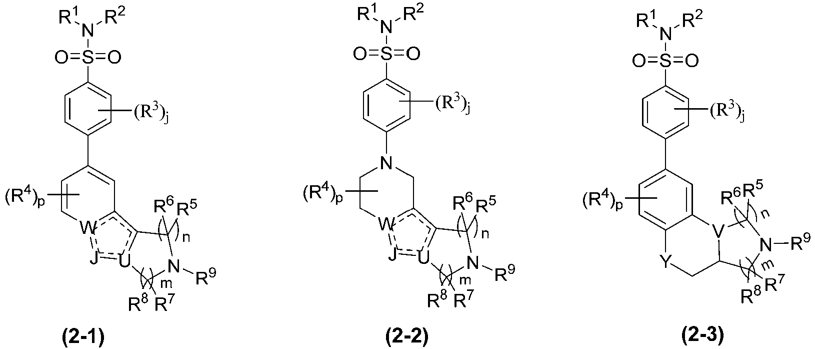 wherein, the definitions of each group are as described in the first aspect of the present invention.

In another preferred embodiment, wherein the compound of formula (1) has a structure represented by the following (3-1), (3-2) or (3-3):

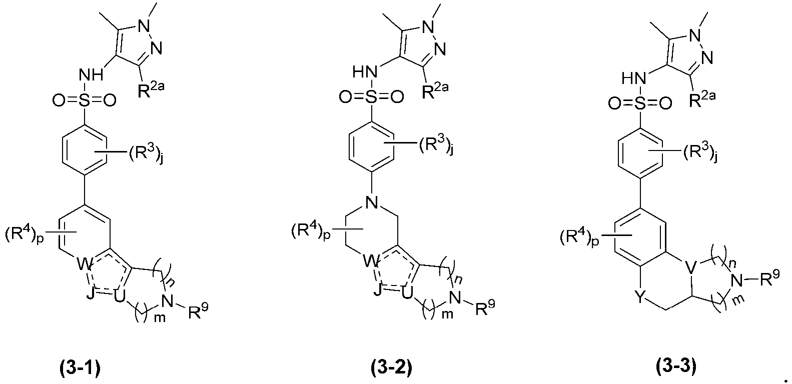

In another preferred embodiment, wherein 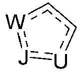 is selected from the group consisting of:

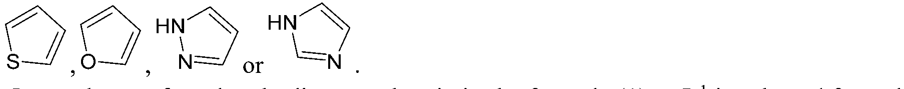

In another preferred embodiment, wherein in the formula (1), R¹ is selected from the group consisting of: H, Me, Et, ⁱ⁻Pr, CHF₂ or CH₂CF₃.

In another preferred embodiment, wherein in the formula (1), R² is 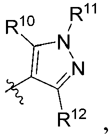 wherein, R¹⁰ is selected from H or C₁₋₆alkyl, R¹¹ is selected from H or C₁₋₆ alkyl, R¹² is R^{2a} as described in the first aspect of the present invention.

In another preferred embodiment, wherein in the formula (1), R² is 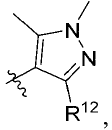 wherein, R¹² is R^{2a} as described in the first aspect of the present invention.

In another preferred embodiment, wherein R² is selected from the group consisting of:

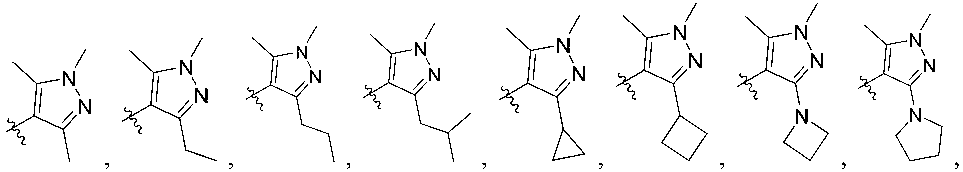

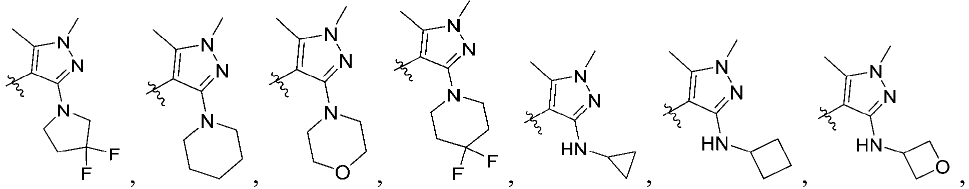

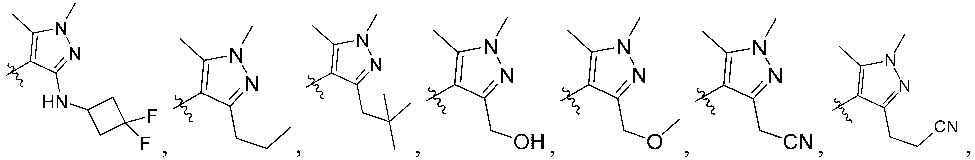

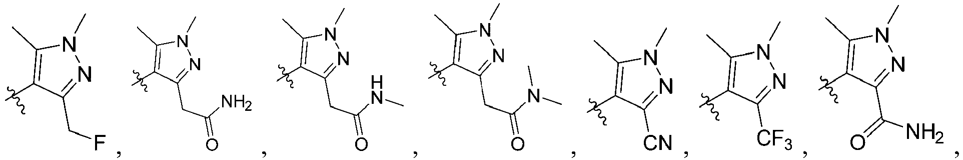

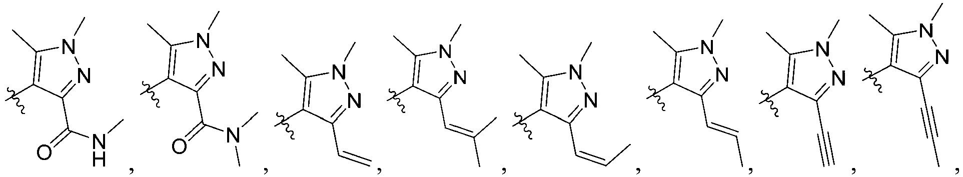

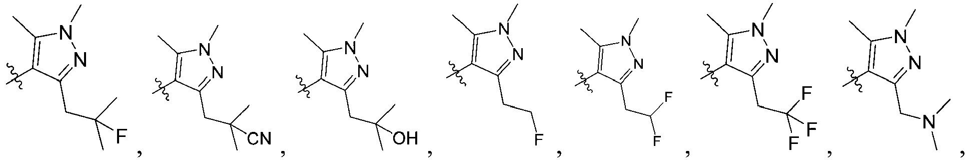

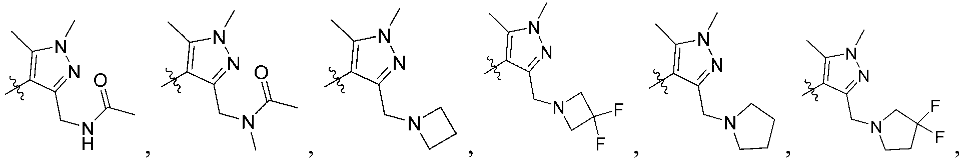

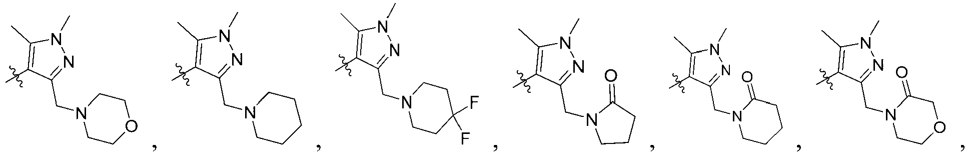

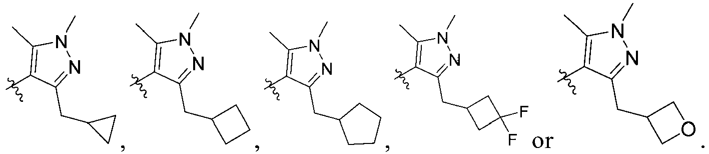

In another preferred embodiment, wherein in the formula (1), wherein R³, R⁴, R⁵, R⁶, R⁷ and R⁸ are each independently selected from the group consisting of: H, halogen, CN, Me, Et, CF₃, cyclopropyl, OMe, OEt or OCF₃.

In another preferred embodiment, wherein in the formula (1), wherein R⁹ is selected from the group consisting of: H or Me.

In another preferred embodiment, wherein the compound has a structure selected from the group consisting of:

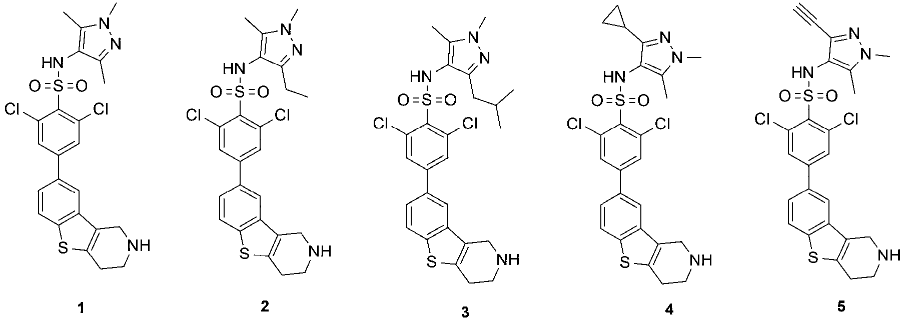

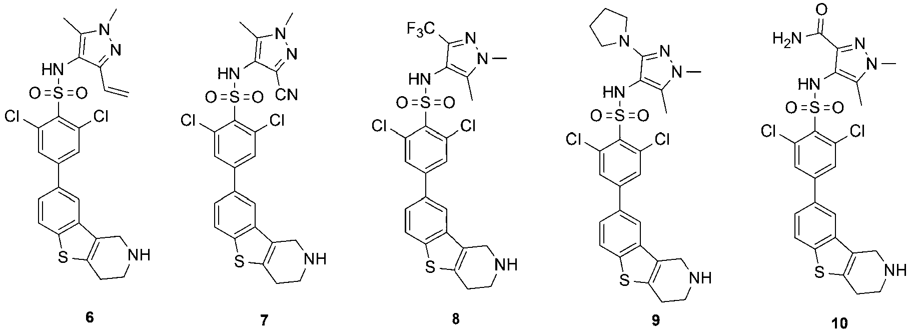

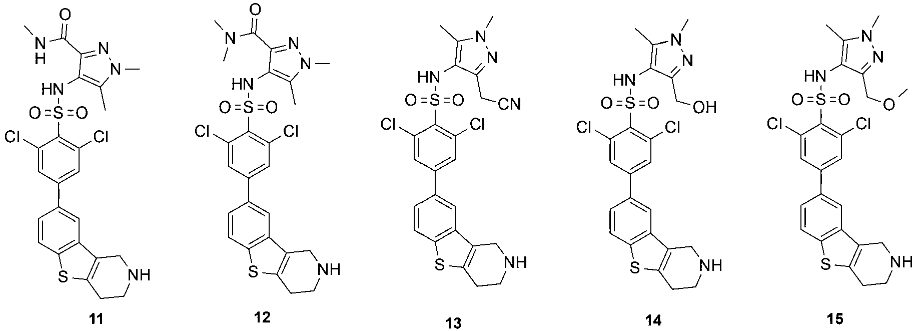

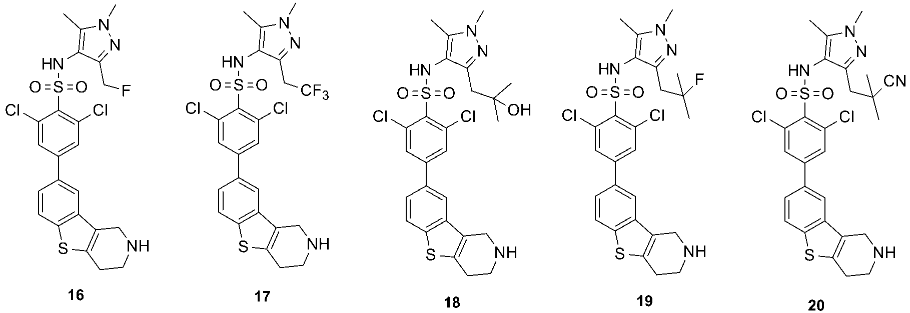

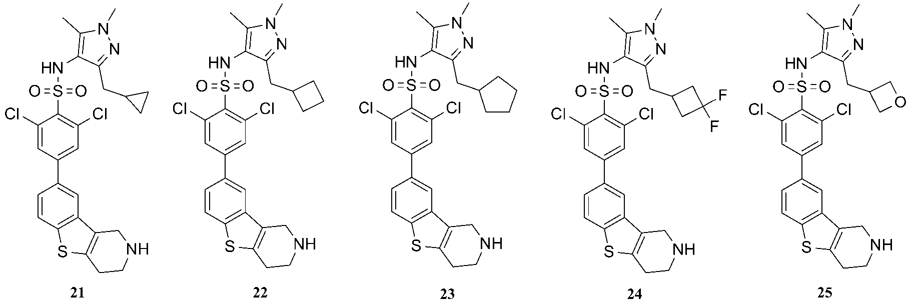

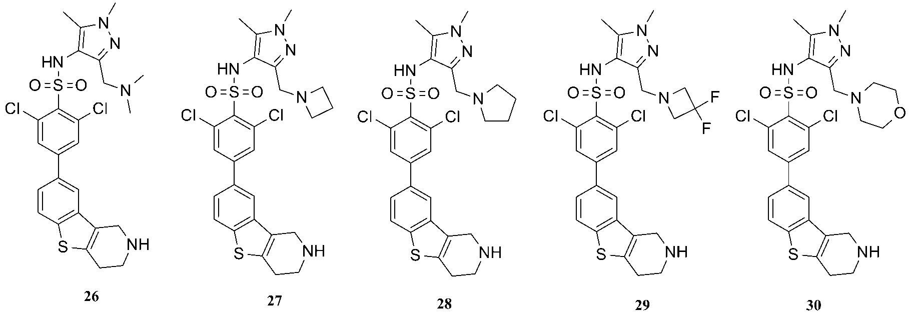

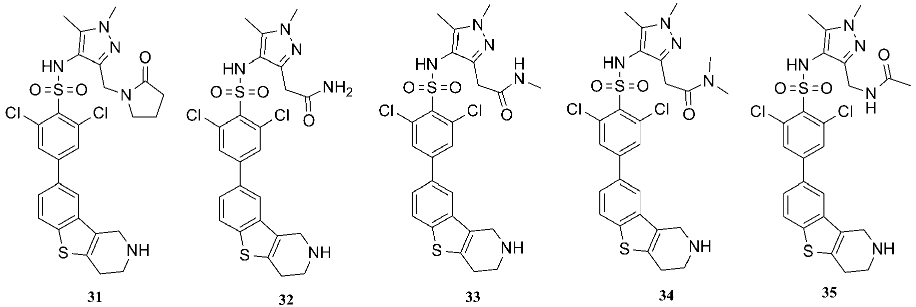

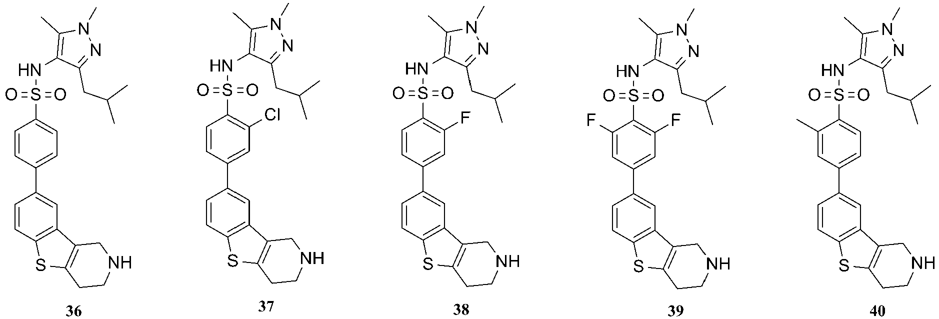

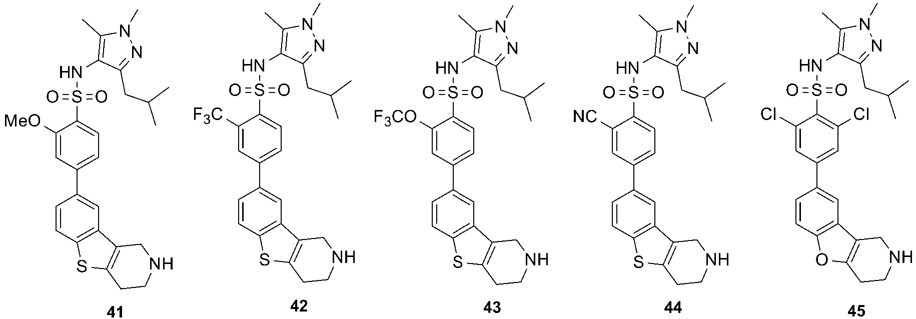

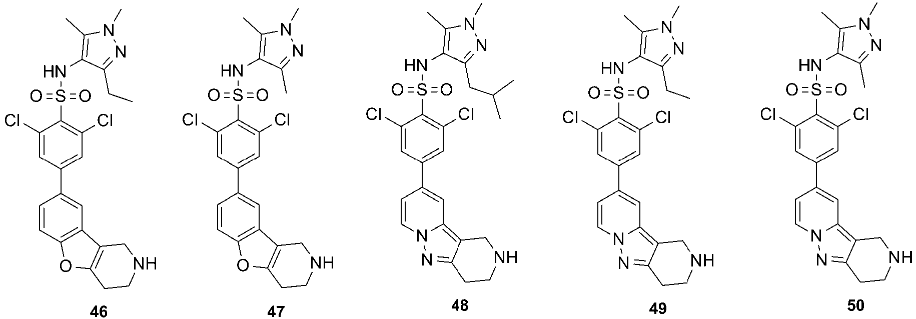

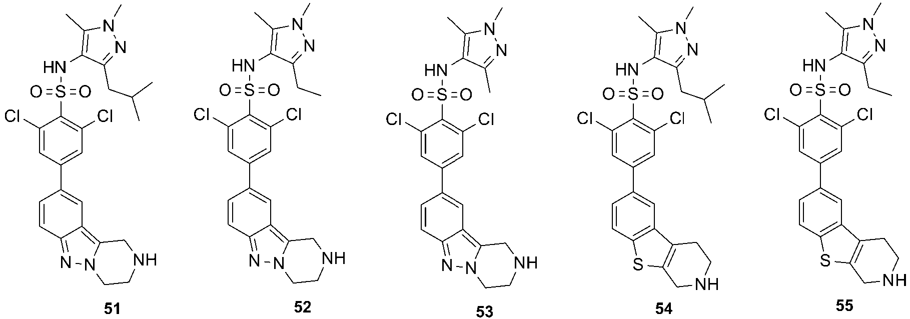

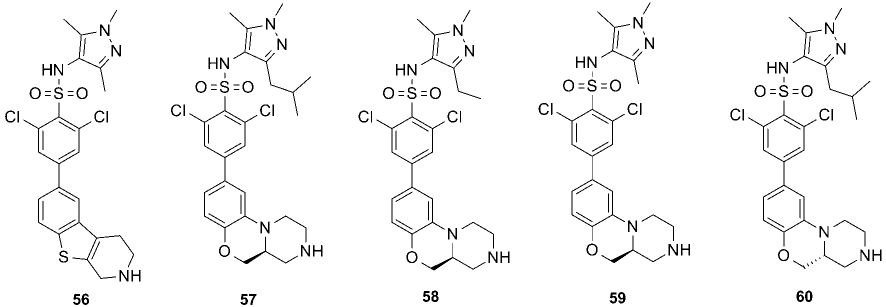

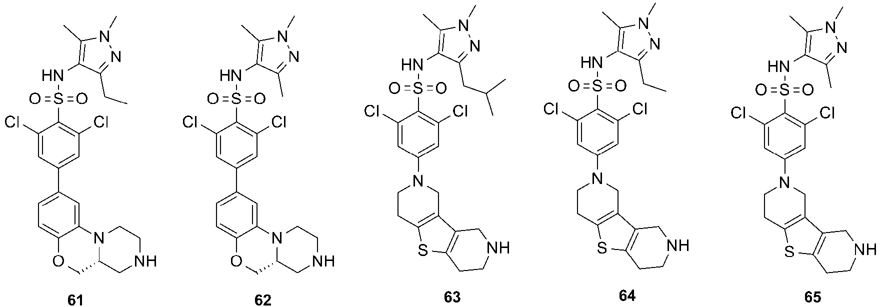

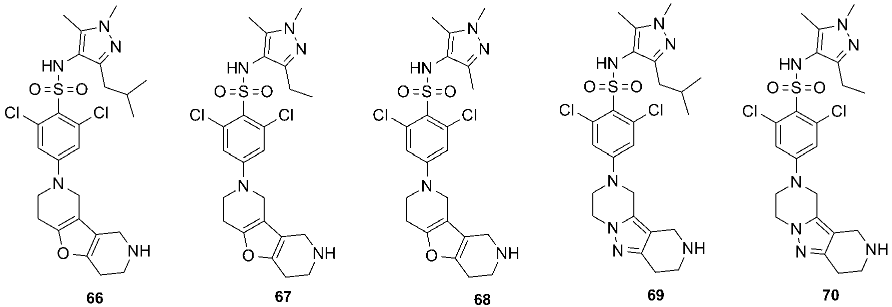

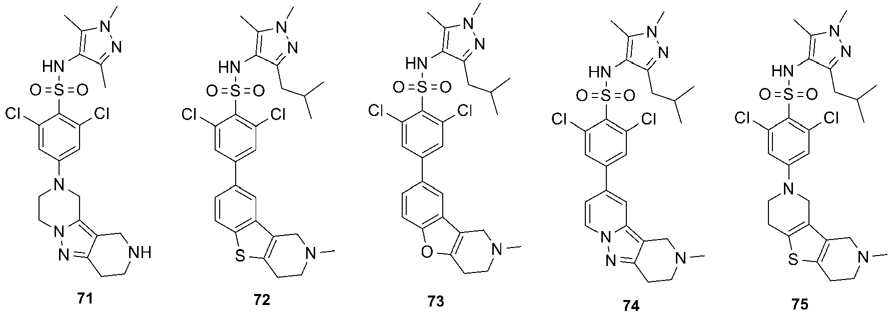

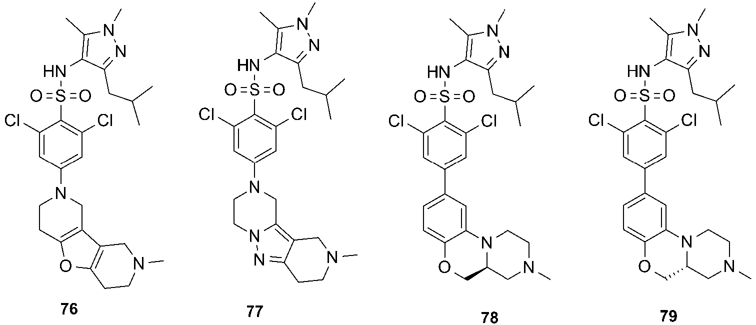

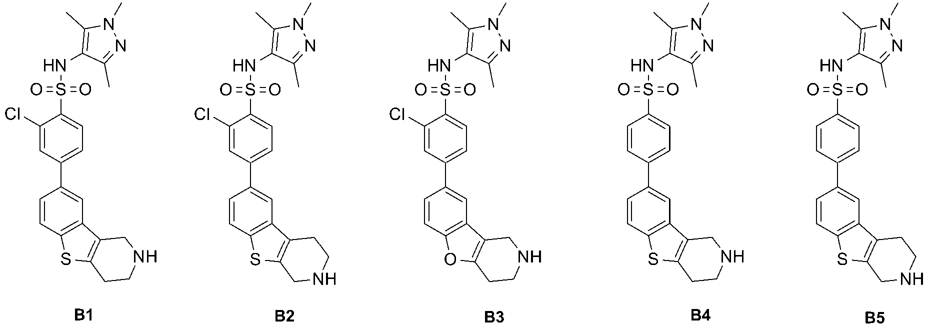

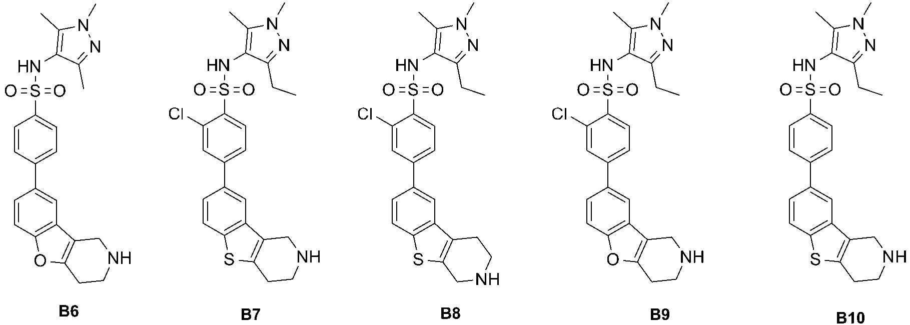

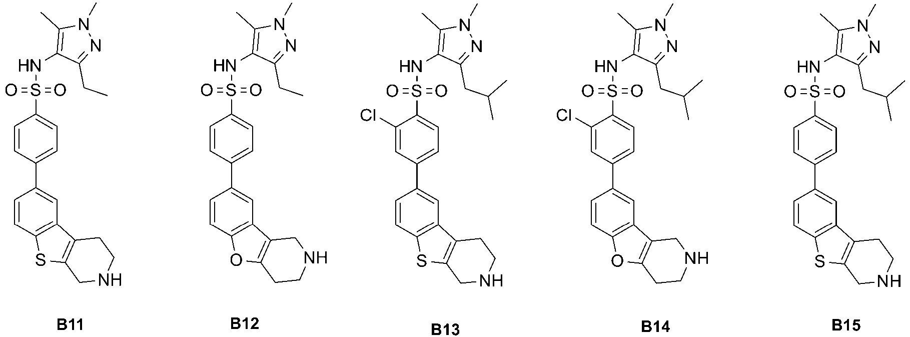

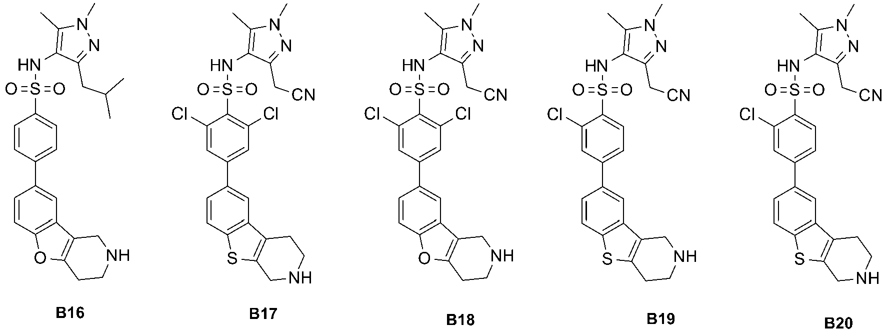

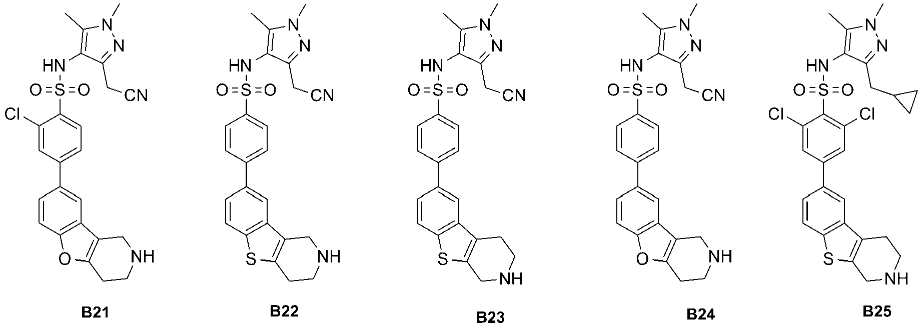

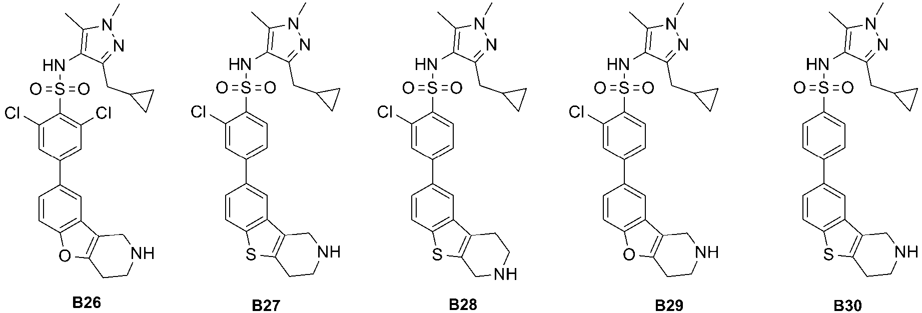

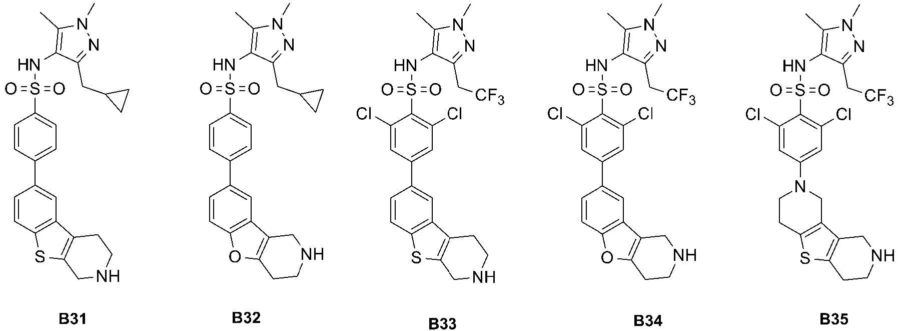

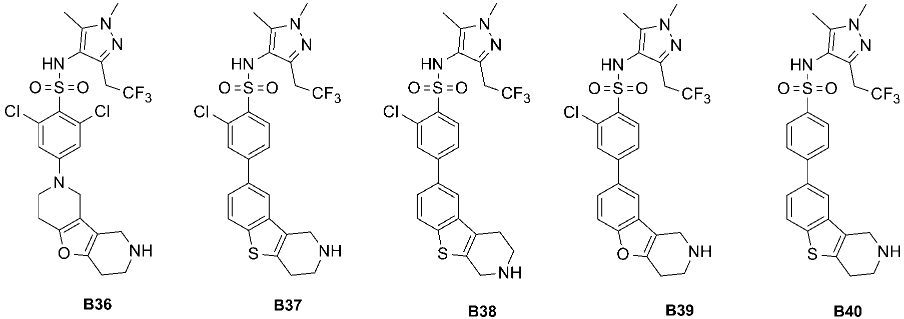

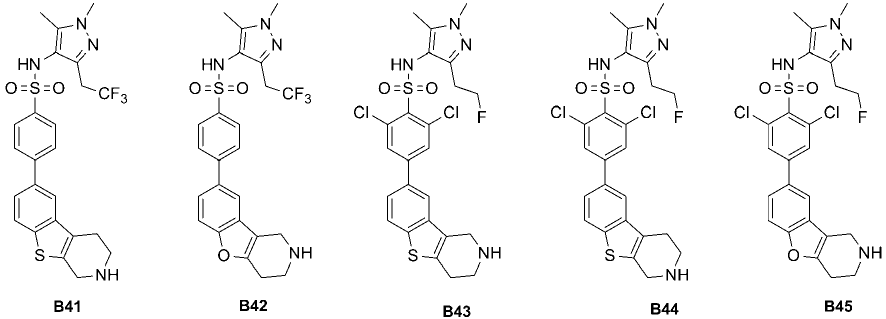

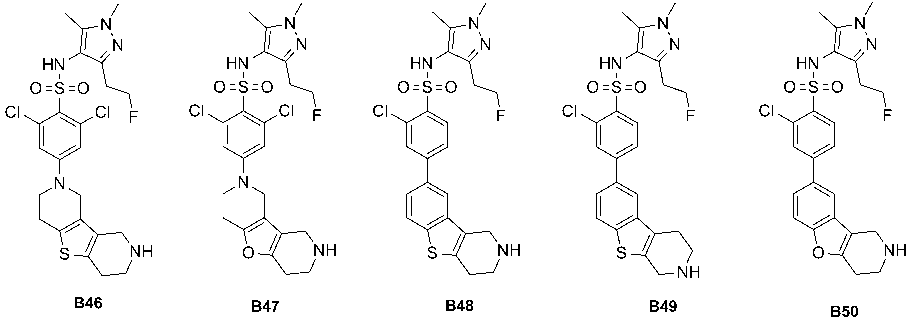

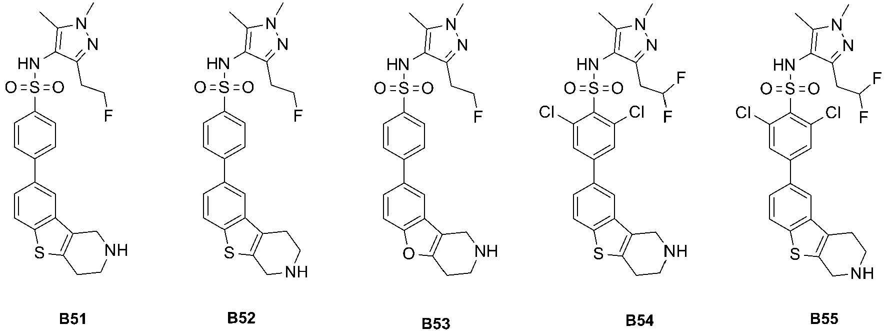

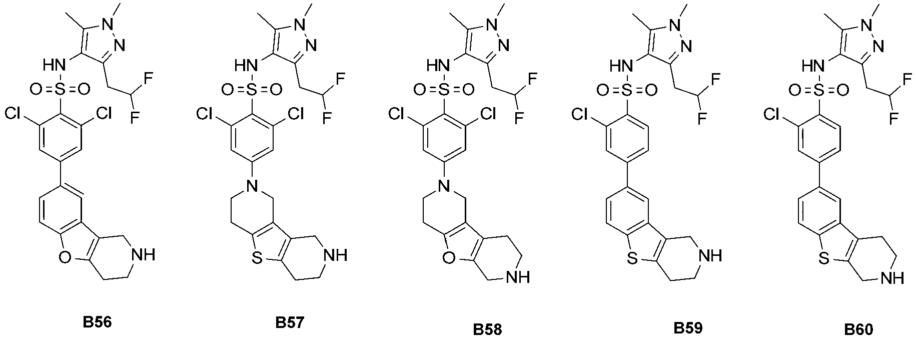

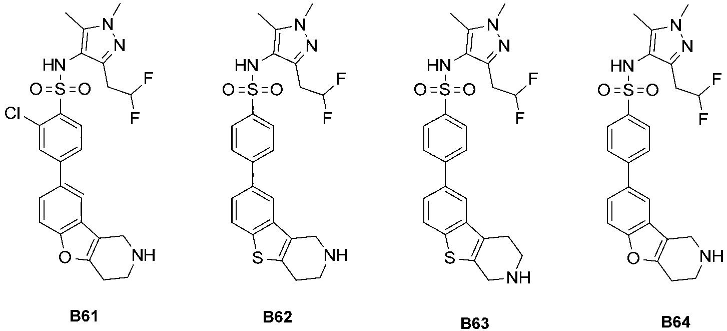

In the second aspect of the present invention, it provides a pharmaceutical composition for treating, regulating, and/or preventing a disease associated with NMT, wherein the pharmaceutical composition comprises:
(1) the compound described in the first aspect of the present invention as an active ingredient, or an isomer, crystal form, pharmaceutically acceptable salt, hydrate, or solvate thereof; and
optionally (2) a pharmaceutically acceptable excipient, or carrier.

In the third aspect of the present invention, it provides use of the compound as described in the first aspect of the present invention, or an isomer, crystal form, pharmaceutically acceptable salt, hydrate, or solvate thereof, or the pharmaceutical composition as described in the second aspect of the present invention for preparing a pharmaceutical composition for treating, regulating, and/or preventing a disease mediated by NMT.

In another preferred embodiment, wherein the related disease mediated by NMT include infectious disease or hyperproliferative disease.

In another preferred embodiment, wherein the infectious disease includes protozoan infection, such as malaria and leishmaniasis.

In another preferred embodiment, wherein the infectious disease includes viral infection, such as human rhinovirus infection and HIV infection.

In another preferred embodiment, wherein the hyperproliferative disease is selected from the following groups: lymphoma, leukemia, brain tumor, gastric tumor, liver cancer, lung cancer, intestinal cancer, pancreatic cancer, breast cancer, cervical cancer, ovarian cancer, endometrial cancer and prostate cancer.

In the fourth aspect of the present invention, it provides a method for treating, regulating, and/or preventing diseases mediated by NMT, characterized by comprising the steps of administering to an individual in need a compound as described in the first aspect of the present invention, or an isomer, polymorph, pharmaceutically acceptable salt, hydrate, or solvate thereof, or a pharmaceutical composition as described in the second aspect of the present invention

It should be understood that within the scope of the present invention, all the technical features of the present invention and those specifically described in the following text (e.g., examples) can be combined with each other to form new or preferred technical solutions. Due to the limited space, we will not enumerate them one by one here.

### FIRURE DESCRIPTION

Figure 1 shows the effects of compounds 2, 3, 13, and 45 on the body weight of MV-4-11 xenograft model mice.
Figure 2 shows the efficacy of compounds 2, 3, 13, and 45 on tumor volume in MV-4-11 xenograft model mice.

### DETAILED DESCRIPTION OF THE ILLUSTRATED EMBODIMENTS

The inventor, after extensive and intensive research, and through a large number of screenings and tests, first discovered a kind of compounds of Formula (1), which have a significant therapeutic effect on diseases mediated by NMT. The compounds of the present invention have potent inhibitory activity against NMT1 enzyme, as well as good inhibitory activity against cell proliferation of MV-4-11 cell line and antitumor activity in a murine MV-4-11 xenograft model. On this basis, the present invention was completed.

### THE COMPOUND OF THE PRESENT INVENTION AND ITS SYNTHESIS

The present invention provides an inhibitor of NMT, i.e., a compound of Formula (1), or an isomer, crystal form, pharmaceutically acceptable salt (inorganic salt or organic salt), hydrate, or solvate thereof. Preferably, the compound of the present invention is as described in the first aspect of the present invention.

The present invention also provides a method for preparing the compound of Formula (1) of the present invention. The following specifically describes the method for preparing the compound of Formula (1) of the present invention, but these specific methods do not constitute any limitation on the present invention.

In one aspect, the compounds described herein are prepared according to the well-known methods. However, the conditions of the process, such as reactants, solvents, bases, amounts of compounds used, reaction temperature, time required for the reactions, etc., are not limited to the following explanations. The compounds of the present invention may also be conveniently prepared, optionally in combination with various synthesis methods described in this specification or well-known in the art, and such combinations are readily carried out by those skilled technicians in the art. On the other aspect, the present invention also provides the preparation methods of the compounds represented by the general formula (1), which is prepared by the following general scheme 1:

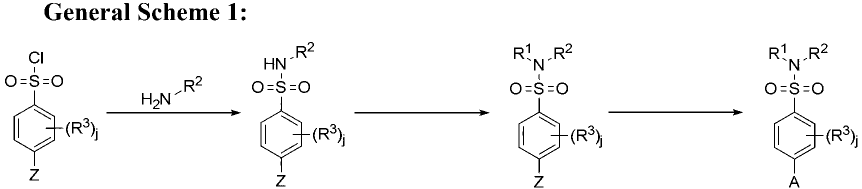

The embodiments of the compound of Formula (1) can be prepared according to the general scheme 1, wherein Z is a halogen, OTf, a boronic acid group or boronic ester group, and R¹, R², R³, j and A are as defined above.

### Related definitions

Unless otherwise specified, the following terms and phrases used herein are intended to have the following meanings. A specific term or phrase should not be considered indefinite in the absence of a particular definition, but should be understood according to its ordinary meaning. When a trade name appears herein, it is intended to refer to its corresponding commercial product or active ingredient thereof.

The term "pharmaceutically acceptable" used herein in terms of those compounds, materials, compositions, and/or dosage forms, which are suitable for use in contact with human and animal tissues within the scope of reliable medical judgment, without excessive toxicity, irritation, allergic reactions or other problems or complications, commensurate with a reasonable benefit/risk ratio.

The term "pharmaceutically acceptable salt" refers to a salt of the compound of the present disclosure that is prepared by reacting the compound having a specific substituent of the present invention with a relatively non-toxic acid or base. When the compound of the present disclosure contains a relatively acidic functional group, a base addition salt can be obtained by contacting the compound with a sufficient amount of a base in a pure solution or a suitable inert solvent. The pharmaceutically acceptable base addition salts include a salt of sodium, potassium, calcium, ammonium, organic amine or magnesium, or similar salts. When the compound of the present disclosure contains a relatively basic functional group, an acid addition salt can be obtained by contacting the compound with a sufficient amount of acid in a pure solution or a suitable inert solvent. Examples of pharmaceutically acceptable acid addition salts include an inorganic acid salt, wherein the inorganic acid include, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphinic acid and the like; and an organic acid salt, wherein the organic acid includes, for example, acetic acid, propionic acid, isobutyric acid, maleic acid, malonic acid, benzoic acid, succinic acid, octanedioic acid, trans-butenedioic acid, lactic acid, mandelic acid, phthalic acid, benzenesulfonic acid, p-toluenesulfonic acid, citric acid, tartaric acid, and methanesulfonic acid, and the like; and salts of amino acids (such as arginine and the like), and a salt of organic acids such as glucuronic acid and the like. Certain specific compounds of the present invention contain both basic and acidic functional groups, thus can be converted to any base or acid addition salt.

The pharmaceutically acceptable salt of the present disclosure can be prepared from the parent compound that contains an acid or basic moiety by conventional chemical method. Generally, such salt can be prepared by reacting the free acid or base form of the compound with a stoichiometric amount of an appropriate base or acid in water or an organic solvent or a mixture thereof.

The compounds of the present disclosure may exist in specific geometric or stereoisomeric forms. The present disclosure contemplates all such compounds, including cis and trans isomers, (-)- and (+)-enantiomers, (*R*)- and (*S*)-enantiomers, diastereoisomers, (*D*)-isomers, (*L*)-isomers, and racemic and other mixtures thereof, such as enantiomers or diastereomer enriched mixtures, all of which are within the scope of the present disclosure. Additional asymmetric carbon atoms may be present in substituents such as alkyl. All these isomers and their mixtures are encompassed within the scope of the present disclosure.

Unless otherwise specified, the term "enantiomer" or "optical isomer" refers to stereoisomers that are mirror images of each other.

Unless otherwise specified, the term "cis-trans isomer" or "geometric isomer" is caused by the inability to rotate freely double bonds or single bonds of ring -forming carbon atoms.

Unless otherwise specified, the term "diastereomer" refers to a stereoisomer in which a molecule has two or more chiral centers and the relationship between the molecules is not mirror images.

Unless otherwise specified, "(*D*)" or " (+)" refers to dextrorotation, "(*L*)" or " (-)" refers to levorotation, and "(*DL*)" or " (±)" refers to racemic.

Unless otherwise specified, the absolute configuration of a stereogenic center is represented by a wedged solid bond ( 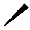 ) and a wedged dashed bond ( 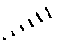 ), and the relative configuration of a stereogenic center is represented by a straight solid bond ( 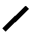 ) and a straight dashed bond ( 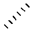 ), a wave line ( 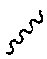 ) is used to represent a wedged solid bond ( 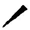 ) or a wedged dashed bond ( 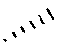 ), or the wave line ( 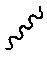 ) is used to represent a straight solid bond ( 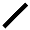 ) or a straight dashed bond ( 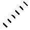 ).

Unless otherwise specified, the term "isomer excess" or "enantiomeric excess" refers to the difference between the relative percentages of two isomers or two enantiomers. For example, if the content of one isomer or enantiomer is 90%, and the content of the other isomer or enantiomer is 10%, then the isomer or enantiomer excess (ee value) is 80%.

Optically active (*R*)- and (*S*)-isomers, or *D* and *L* isomer can be prepared using chiral synthesis or chiral reagents or other conventional techniques. If one kind of enantiomer of certain compound of the present disclosure is to be obtained, the pure desired enantiomer can be obtained by asymmetric synthesis or derivative action of chiral auxiliary followed by separating the resulting diastereomeric mixture and cleaving the auxiliary group. Alternatively, when the molecule contains a basic functional group (such as an amino) or an acidic functional group (such as a carboxyl), the compound reacts with an appropriate optically active acid or base to form a salt of the diastereomeric isomer which is then subjected to diastereomeric separation through the conventional method in the art to give the pure enantiomer. In additional, the enantiomers and diastereoisomer are generally isolated through chromatography which uses a chiral stationary phase and optionally combines with a chemical derivative method (such as carbamate generated from amine).

Compounds disclosed herein may contain unnatural proportions of atomic isotopes at one or more of the atoms that make up the compounds. For example, a compound may be labeled with a radioisotope such as tritium (³H), iodine-125 (¹²⁵I) or C-14 (¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs usually have the advantages of reduced toxic side effects, increased drug stability, enhanced efficacy, and prolonged biological half-life of the drug. All changes in the isotopic compositions of compounds disclosed herein, regardless of radioactivity are included within the scope of the present disclosure.

The term "optional" or "optionally" means that the subsequent event or condition may occur but not requisite, that the term includes the instance in which the event or condition occurs and the instance in which the event or condition does not occur.

The term "substituted" refers to that one or more than one hydrogen atoms on a specific atom are substituted by a substituent, including deuterium and hydrogen variants, as long as the valence of the specific atom is normal and the substituted compound is stable. When the substituent is oxo (i.e., =O), it means that two hydrogen atoms are substituted. Positions on an aromatic ring cannot be substituted by oxo. The term "optionally substituted" means an atom can be substituted with a substituent or not, unless otherwise specified, the type and the number of the substituent may be arbitrary so long as being chemically achievable.

When any variable (e.g., R) occurs in the constitution or structure of the compound more than once, the definition of the variable at each occurrence is independent. Thus, for example, if a group is substituted by 0-2 R, the group can be optionally substituted by up to two R, wherein the option of R at each occurrence is independent. Moreover, the combination of the substituent and/or the variant thereof is allowed only when the combination results in a stable compound.

When the number of a linking group is 0, such as -(CH₂)₀-, it means that the linking group is a single bond.

When one of the variables is a single bond, it means that the two groups linked by the single bond are connected directly. For example, when L in X-L-Y represents a single bond, the structure of X-L-Y is actually X-Y.

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; Similarly, n membered to n+m membered indicates the number of atoms on a ring is n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, etc.

Unless otherwise specified, "C₁₋₆ alkyl" refers to a linear or branched saturated hydrocarbon group composed of 1 to 6 carbon atoms. The C₁₋₆ alkyl includes C₁₋₂, C₁₋₃, C₁₋₄, C₁₋₅, C₂₋₄, C₂₋₆, C₃₋₅, C₅ and C₆ alkyl and the like; it can be monovalent (such as methyl), divalent (such as methylene), or multivalent (such as methine). Examples of C₁₋₆ alkyl include but not limited to methyl, ethyl, propyl, n-propyl, isopropyl, n-butyl, isobutyl, tert butyl, sec butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2,3-dimethylbutyl, 2-ethylbutyl, 2-methylpentyl, 3-methylpentyl, 4-methylpentyl, and their various branched isomers and the like.

Unless otherwise specified, "C₁₋₃ alkyl" refers to a linear or branched saturated hydrocarbon group composed of 1 to 3 carbon atoms. The C₁₋₃ alkyl includes C₁₋₂ and C₂₋₃ alkyl and the like, it can be monovalent (such as methyl), divalent (such as methylene), or multivalent (such as methine). Examples of C₁₋₃ alkyl include but not limited to methyl, ethyl, propyl, n-propyl, isopropyl and the like.

Unless otherwise specified, "C₁₋₃ alkoxy" refers to an alkyl containing 1 to 3 carbon atoms that are connected to the rest of the molecule through an oxygen atom. The C₁₋₃ alkoxy includes C₁₋₂, C₂, and C₃ alkoxy and the like. Examples of C₁₋₃ alkoxy include but are not limited to methoxy, ethoxy, propoxy, n-propyloxy, isopropyl and the like.

The term "halo" or "halogen" refers to fluorine, chlorine, bromine and iodine atom.

The term "hydroxyl" refers to -OH.

The term "cyano" refers to -CN.

### SPECIFIC PHARMACEUTICAL AND MEDICAL TERMS

The term "acceptable," as used herein, means that a prescription component or active ingredient does not have excessively harmful effects on the health of subject.

As used herein, the term "treatment," "course of treatment" or "therapy" includes alleviating, inhibiting or improving symptoms or conditions of diseases; inhibiting the occurrence of complications; improving or preventing potential metabolic syndrome; suppressing the occurrence of diseases or symptoms, such as controlling the development of diseases or conditions; alleviating diseases or symptoms; reducing disease or symptoms; alleviating complications caused by diseases or symptoms, or preventing or treating symptoms caused by diseases or symptoms. As used herein, a certain compound or pharmaceutical composition, which can improve a certain disease, symptom or condition after administration, especially reduce its severity, delay the onset, slow down the progress of the disease, or shorten the duration of the disease. Whether fixed administration or temporary administration, continuous administration or intermittent administration, which can be attributed to related administration.

The term "active ingredient" refers to the compounds represented by the general formula (1) and the pharmaceutically acceptable inorganic or organic salts of the compounds of the general formula (1). Compounds of the present invention may contain one or more asymmetric centers, and thus appear in the form of racemates, racemic mixtures, single enantiomers, diastereomeric compounds or single diastereomers. The asymmetric centers that can exist depend on the properties of various substituents on the molecule. Each such asymmetric center will independently produce two optical isomers, and all possible optical isomers, diastereomer mixtures and pure or partially pure compounds are included in the scope of the present disclosure. The present disclosure is meant to include all such isomeric forms of these compounds.

The terms such as "compound," "composition," "agent" or "medicine or medicament" can be used interchangeably here, and all refer to a compound or composition which can induce the desired pharmaceutical and/or physiological response through local and/or systemic action when administered to a person (human or animal).

The term "administered, administering or administration" here refers to the direct administration of the compounds or compositions, or the administration of prodrugs, derivatives or analogs of the active compounds.

Although the numerical ranges and parameters used to define the wide range of the present invention are approximate values, the relevant values in the specific embodiments have been presented here as accurately as possible. However, any numerical value inevitably contains standard deviation caused by individual test methods. Here, "about" usually means that the actual value is within plus or minus 10%, 5%, 1% or 0.5% of a specific value or range. Alternatively, the word "about" means that the actual value falls within the acceptable standard error of the average value, depending on the consideration of those skilled in the art. Except for experimental examples, or unless otherwise specified, it is understood that all ranges, quantities, values and percentages used herein (for example, to describe the amount of materials, the length of time, the temperatures, the operating conditions, the proportions of quantities and other similar ones) are modified by "about". Therefore, unless otherwise stated, the numerical parameters disclosed in this specification and the appended claims are approximate values, and can be changed as required. At least these numerical parameters should be understood as the indicated effective digits and the numerical values obtained by applying the general carry method.

Unless otherwise defined in this specification, the meanings of scientific and technical terms used herein are the same as those understood and used by those skilled in the art. In addition, the singular noun used in this specification covers the plural form of the noun without conflict with the context; the plural nouns used also cover the singular form of the noun.

### ROUTE OF ADMINISTRATION

The compound of the present invention and its pharmaceutically acceptable salts can be made into various preparations, which contain the compound of the present invention or its pharmaceutically acceptable salts and pharmaceutically acceptable excipients or carriers in a safe and effective amount range. Among them, "safe and effective amount" means that the amount of the compound is capable of obviously improving the condition without causing serious side effects. The safe and effective dose of the compound is determined according to the age, illness, course of treatment and other specific conditions of the subject.

"Pharmaceutically acceptable excipient or carrier" refers to one or more compatible solid or liquid fillers or gel substances, which are suitable for human use and must have sufficient purity and low toxicity. "Compatibility" here means that each component in the composition can be mixed with the compounds of the present invention and between them, without significantly reducing the efficacy of the compound. Examples of pharmaceutically acceptable excipients or carriers include cellulose and its derivatives (such as sodium carboxymethyl cellulose, sodium ethyl cellulose, cellulose acetate, etc.), gelatin, talc, solid lubricants (such as stearic acid and magnesium stearate), calcium sulfate, vegetable oils (such as soybean oil, sesame oil, peanut oil, olive oil, etc.), polyols (such as propylene glycol, glycerol, mannitol, sorbitol, etc.), emulsifiers (such as Tween^{®}), wetting agent (such as sodium dodecyl sulfate), coloring agent, flavoring agent, stabilizer, antioxidant, preservative, pyrogen-free water, etc.

The compounds of the present invention can be administered orally, rectally, parenterally (intravenously, intramuscularly or subcutaneously) or topically.

Solid dosage forms for oral administration include capsules, tablets, pills, powders and granules. In these solid dosage forms, the active compound is mixed with at least one conventional inert excipient (or carrier), such as sodium citrate or dicalcium phosphate, or with: (a) a filler or compatibilizer, such as starch, lactose, sucrose, glucose, mannitol, and silicic acid; (b) binders such as hydroxymethyl cellulose, alginates, gelatin, polyvinyl pyrrolidone, sucrose and acacia; (c) humectant, such as glycerol; (d) disintegrant such as agar, calcium carbonate, potato or tapioca starch, alginic acid, certain complex silicates, and sodium carbonate; (e) slow solvents, such as paraffin; (f) an absorption accelerator, such as a quaternary amine compound; (g) wetting agents, such as cetyl alcohol and glyceryl monostearate; (h) adsorbent, such as kaolin; and (i) a lubricant such as talc, calcium stearate, magnesium stearate, solid polyethylene glycol, sodium lauryl sulfate, or mixtures thereof. In capsule, tablets and pill, the dosage form may also contain a buffer.

Solid dosage forms such as tablets, sugar pills, capsules, pills and granules may be prepared using coatings and shell materials such as casings and other materials well-known in the art. They may comprise an opacifying agent and the release of the active compound or compound in such a composition may be released in a delayed manner in a portion of the digestive tract. Examples of embedding components that may be used are polymeric substances and waxes. If desired, the active compound may also form microcapsules with one or more of the above excipients.

Liquid dosage forms for oral administration include pharmaceutically acceptable emulsions, solutions, suspensions, syrups or tinctures. In addition to the active compound, the liquid dosage form may comprise inert diluents conventionally used in the art, such as water or other solvents, solubilizers and emulsifiers, for example ethanol, isopropanol, ethyl carbonate, ethyl acetate, propylene glycol, 1,3-butanediol, dimethylformamide and oils, particularly cottonseed oil, peanut oil, corn germ oil, olive oil, castor oil, sesame oil or mixtures of these and the like.

In addition to these inert diluents, the composition may also contain adjuvants such as wetting agents, emulsifiers and suspending agents, sweeteners, flavoring agents and flavorants.

In addition to the active compounds, the suspension may comprise suspending agents such as ethoxylated isostearyl alcohol, polyoxyethylene sorbitol and sorbitan esters, microcrystalline cellulose, aluminum methoxide, agar or mixtures of these and the like.

The composition for parenteral injection may comprise a physiologically acceptable sterile aqueous or non-aqueous solution, dispersion, suspension or emulsion, and a sterile powder for reconstitution into a sterile injectable solution or dispersion. Suitable aqueous and non-aqueous carriers, diluents, solvents or excipients include water, ethanol, polyols and suitable mixtures thereof.

Dosage forms of the compounds of the present invention for topical administration include ointments, powders, patches, sprays and inhalants. The active ingredient is mixed under sterile conditions with a physiologically acceptable carrier and any preservatives, buffers, or propellants as may be required.

The compound of the present invention may be administered alone or in combination with other pharmaceutically acceptable compounds.

When a pharmaceutical composition is used, a safe and effective amount of a compound of the present invention is applied to a mammal (e.g., a human) in need of treatment, wherein the dose at the time of administration is a pharmaceutically acceptable effective dose, and for a human of 60 kg body weight, the daily dose is generally 1 to 2000 mg, preferably 50 to 1000 mg. Of course, specific dose should also take into account factors such as route of administration, patient health, etc., which are within the skill scope of a skilled physician.

The main advantages of the present disclosure include:
(a) Unexpectedly, the compound of Formula (1) of the present disclosure showed excellent *in vitro* activity, including potent inhibitory activity against NMT1 enzyme and good cell proliferation inhibition activity against MV-4-11 cell line, making it more suitable for development as a drug.
(b) The compound of the present disclosure showed excellent oral absorption and strong tumor inhibitory activity in mice
(c) The compound of the present disclosure showed good druggablility.

The above features mentioned in the present disclosure, or the features mentioned in the examples, may be combined at random. All of the features disclosed in this specification may be used in any composition form and the various features disclosed in the specification may be replaced with any alternative feature that provides the same, equivalent, or similar purpose. Thus, unless otherwise specified, the disclosed features are merely generic examples of equivalent or similar features.

Various specific aspects, features and advantages of the above compounds, methods, and pharmaceutical compositions will be set forth in detail as following. It is to be understood that the following detailed description and examples describe specific embodiments for reference only. Various changes or modifications may occur to those skilled in the art after reading the description of the invention, and such equivalents fall within the scope of the application.

The structures of compounds disclosed herein can be confirmed by conventional methods well known to those skilled in the art. If the present disclosure relates to an absolute configuration of a compound, the absolute configuration can be confirmed by conventional techniques in the field. For example, single crystal X-ray diffraction (SXRD), the diffraction intensity data of the cultivated single crystal is collected using a Bruker D8 venture diffractometer with a light source of CuKα radiation in a scanning mode of φ/ω scan. After collecting the relevant data, the crystal structure is further analyzed by the direct method (Shelxs97) to conform the absolute configuration.

The solvent used in the present invention can be obtained commercially. The compounds of the present disclosure are named according to the conventional naming principles in the art or by ChemDraw^{®} software, and the commercially available compounds use the supplier catalog names.

In all examples, ¹H-NMR was recorded with a Varian Mercury 400 NMR spectrometer and that chemical shift was expressed as δ (ppm); Silica gel for separation is 200-300 mesh without specific statement, and the ratio of eluents is volume ratio.
Abbreviations: (Boc)₂O represents di-tert-butyl dicarbonate; ACN (CH₃CN) represents acetonitrile; °C represents degree celsius; Cs₂CO₃ represents caesium carbonate; CD₃OD represents Methanol-*d*₄; EA (EtOAc) represents ethyl acetate; DCM represents Dichloromethane; DIEA (DIPEA) represents *N*,*N*-diisopropylethylamine; Dioxane represents 1,4-dioxane; DMF represents *N*,*N-*dimethylformamide; h represents hour; HATU represents *O-*(7-Azabenzotriazol-1-yl)-*N*,*N*,*N',N'*-tetramethyl uronium hexa fluorophosphate; H₂O₂ represents hydrogen peroxide; K₂CO₃ represents potassium carbonate; K₃PO₄ represents potassium phosphate tribasic; LC-MS represents liquid chromatography mass spectrometry; LiAlH₄ represents lithium aluminum hydride; MS represents mass spectrometry; MsCl represents methanesulfonyl chloride; NaBH₃CN represents sodium cyanoborohydride; NaHCO₃ represents sodium bicarbonate; NaOH represents sodium hydroxide; NIS represents N-iodosuccinimide; NMR represents nuclear magnetic resonance; Pd₂(dba)₃ represents tris(dibenzylideneacetone)dipalladium; Pd(dppf)₂Cl₂ represents [1,1'-Bis-(diphenylphosphino)ferrocene]dichloropalladium(II); PE represents petroleum ether; POCl₃ represents phosphorus oxychloride; TEA represents triethylamine_{∘}

The present invention is further described through the following examples. It should be understood that these examples are only used to illustrate the invention, but not intended to impose any limitations on the present invention. The experimental methods not specified in the following examples are usually carried out under conventional conditions, or according to the conditions recommended by the manufacturer. Unless otherwise stated, percentages and parts are weight percentages and weight parts.

### Preparation Example 1: Synthesis of 3-ethyl-1,5-dimethyl-1H-pyrazol-4-amine (Intermediate 1)

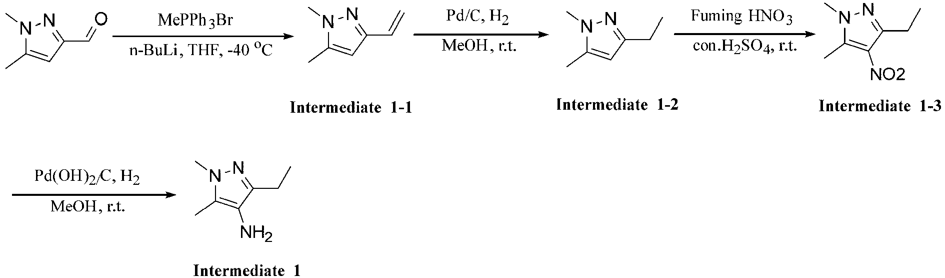

### Step 1: Synthesis of Intermediate 1-1

Methyltriphenylphosphonium bromide (8.63 g, 24.17 mmol) was dissolved in THF (100 mL). ⁿ⁻BuLi (2.5 M in hexane, 9.67 mL, 24.17 mmol) was added at -40 °C, and the mixture was stirred at -40 °C for 1 h. A solution of 1,5-dimethylpyrazole-3-carbaldehyde (2 g, 16.11 mmol) in THF was then added, and the reaction mixture was allowed to warm slowly to room temperature. The reaction was monitored by LC-MS until the starting material was consumed. Saturated aqueous ammonium chloride was added to quench the reaction, and the mixture was extracted with EA (50 mL*3). The combined organic phases were dried, concentrated, and purified by column chromatography (PE/EA = 20:1 to 5:1) to afford **intermediate 1-1** (1.65 g, 84% yield) as a yellow liquid. ESI-MS m/z: 123.1 [M+H]⁺.

### Step 2: Synthesis of Intermediate 1-2

**Intermediate 1-1** (1.65 g, 13.52 mmol) was dissolved in MeOH (20 mL). Pd/C (165 mg, 10%) was added, and the reaction vessel was purged with hydrogen gas. The mixture was stirred at room temperature under a hydrogen atmosphere, and the reaction was monitored by LC-MS until the starting material was consumed. The mixture was directly filtered by suction, and the filtrate was dried and concentrated to afford **intermediate 1-2** (1.64 g, 98% yield) as a pale yellow liquid. ESI-MS m/z: 125.1 [M+H]⁺.

### Step 3: Synthesis of Intermediate 1-3

**Intermediate 1-2** (1.64 g, 13.23 mmol) was dissolved in concentrated sulfuric acid (5 mL). Fuming nitric acid (15 mL) was added slowly at 0 °C, and the mixture was stirred at room temperature. The reaction was monitored by LC-MS until the starting material was consumed. The reaction mixture was poured slowly into water (100 mL) and neutralized to weakly basic with saturated aqueous sodium bicarbonate. The mixture was extracted with EA (50 mL*3). The combined organic phases were dried, concentrated, and purified by column chromatography (PE/EA = 20:1 to 5:1) to afford **intermediate 1-3** (1.41 g, 63% yield) as a pale yellow oil. ESI-MS m/z: 170.1 [M+H]⁺.

### Step 4: Synthesis of Intermediate 1

**Intermediate 1-3** (1.41 g, 8.34 mmol) was dissolved in MeOH (20 mL). Pd(OH)₂/C (141 mg, 10%) was added, and the reaction vessel was purged with hydrogen gas. The mixture was stirred at room temperature under a hydrogen atmosphere, and the reaction was monitored by LC-MS until the starting material was consumed. The mixture was filtered directly by suction, and the filtrate was dried and concentrated to afford **intermediate 1** (1.09 g, 94% yield) as a pale yellow oil. ESI-MS m/z: 140.1 [M+H]⁺.

Using a synthetic approach similar to that described in **preparation example 1, intermediate 2** can be prepared.

| Compound | Structure | Name | ESI-MS: [M+H]⁺ |
|---|---|---|---|
| **Intermediate 2** | 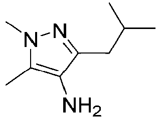 | 3-Isobutyl-1,5-dimethyl-1*H-*pyrazol-4-amine | 168.1 |

### Preparation Example 2: Synthesis of 1,5-Dimethyl-3-vinyl-1H-pyrazol-4-amine (Intermediate 3)

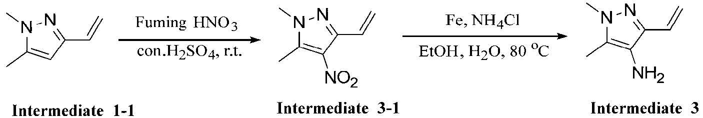

### Step 1: Synthesis of Intermediate 3-1

**Intermediate 1-1** (1.0 g, 8.13 mmol) was dissolved in concentrated sulfuric acid (5 mL). Fuming nitric acid (15 mL) was added slowly at 0 °C, and the mixture was stirred at room temperature. The reaction was monitored by LC-MS until the starting material was consumed. The reaction mixture was poured slowly into water (100 mL) and neutralized to weakly basic with saturated aqueous sodium bicarbonate. The mixture was extracted with EA (50 mL*3). The combined organic phases were dried, concentrated, and purified by column chromatography (PE/EA = 20:1 to 5:1) to afford **intermediate 3-1** (653 mg, 48% yield) as a pale yellow oil. ESI-MS m/z: 168.1 [M+H]⁺.

### Step 2: Synthesis of Intermediate 3

**Intermediate 3-1** (653 mg, 3.91 mmol) was dissolved in EtOH/H₂O (10/2 mL). Fe powder (1.09 g, 19.55 mmol) and NH₄Cl (1.05 g, 19.55 mmol) were added, and the mixture was stirred at 80 °C. The reaction was monitored by LC-MS until the starting material was consumed. The mixture was filtered directly by suction, and the filtrate was extracted with EA (30 mL*3). The combined organic phases were dried, concentrated, and purified by column chromatography (PE/EA = 10:1 to 1:1) to afford **intermediate 3** (348 mg, 65% yield) as a pale yellow oil. ESI-MS m/z: 138.1 [M+H]⁺.

### Preparation Example 3: Synthesis of 3-Cyclopropyl-1,5-dimethyl-1H-pyrazol-4-amine (Intermediate 4)

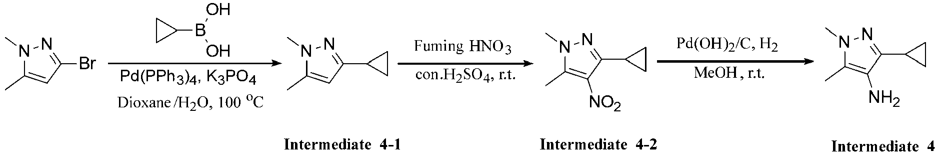

### Step 1: Synthesis of Intermediate 4-1

3-Bromo-1,5-dimethylpyrazole (1 g, 5.71 mmol) and cyclopropylboronic acid (589 mg, 6.86 mmol) were dissolved in 1,4-dioxane/H₂O (30/3 mL). Pd(PPh₃)₄ (330 mg, 0.29 mmol) and K₃PO₄ (2.42 g, 11.42 mmol) were added, and the mixture was stirred at 100 °C. The reaction was monitored by LC-MS until the starting material was consumed. The mixture was concentrated directly to remove 1,4-dioxane, poured into water (50 mL), and extracted with EA (30 mL*3). The combined organic phases were dried, concentrated, and purified by column chromatography (PE/EA = 20:1 to 5:1) to afford **intermediate 4-1** as a yellow liquid (404 mg, 52% yield). ESI-MS m/z: 137.1 [M+H]⁺.

Subsequently, using a synthetic method similar to that described in **preparation example 1, Intermediate 4** can be obtained.

| Compound | Structure | Name | ESI-MS: [M+H]⁺ |
|---|---|---|---|
| **Intermediate 4** | 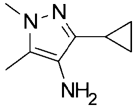 | 3-Cyclopropyl-1,5-dimethyl-1*H-*pyrazol-4-amine | 152.1 |

### Preparation Example 4: Synthesis of 3-Ethynyl-1,5-dimethyl-1H-pyrazol-4-amine (Intermediate 5)

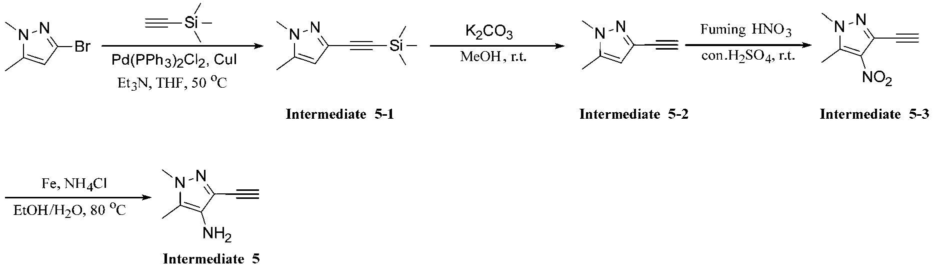

### Step 1: Synthesis of Intermediate 5-1

3-Bromo-1,5-dimethylpyrazole (1 g, 5.71 mmol) and trimethylsilylacetylene (1.68 g, 17.13 mmol) were dissolved in THF (30 mL). Pd(PPh₃)₂Cl₂ (400 mg, 0.57 mmol), CuI (109 mg, 0.57 mmol), and TEA (1.73 g, 17.13 mmol) were added, and the mixture was stirred at 50 °C. The reaction was monitored by LC-MS until the starting material was consumed. The mixture was poured into water (50 mL) and extracted with EA (30 mL*3). The combined organic phases were dried, concentrated, and purified by column chromatography (PE/EA = 20:1 to 5:1) to afford **intermediate 5-1** (680 mg, 62% yield) as a yellow liquid. ESI-MS m/z: 193.1 [M+H]⁺.

### Step 2: Synthesis of Intermediate 5-2

**Intermediate 5-1** (680 mg, 3.54 mmol) was dissolved in MeOH (20 mL). K₂CO₃ (979 mg, 7.08 mmol) was added, and the mixture was stirred at room temperature. The reaction was monitored by LC-MS until the starting material was consumed. The mixture was poured into water (50 mL) and extracted with EA (30 mL*3). The combined organic phases were dried, concentrated, and purified by column chromatography (PE/EA = 20:1 to 5:1) to afford **intermediate 5-2** (306 mg, 72% yield) as a yellow liquid. ESI-MS m/z: 121.1 [M+H]⁺.

### Step 3: Synthesis of Intermediate 5-3

**Intermediate 5-2** (306 mg, 2.55 mmol) was dissolved in concentrated sulfuric acid (2 mL). Fuming nitric acid (6 mL) was added slowly at 0 °C, and the mixture was stirred at room temperature. The reaction was monitored by LC-MS until the starting material was consumed. The reaction mixture was poured slowly into water (50 mL) and neutralized to weakly basic with saturated aqueous sodium bicarbonate. The mixture was extracted with EA (30 mL*3). The combined organic phases were dried, concentrated, and purified by column chromatography (PE/EA = 20:1 to 10:1) to afford **intermediate 5-3** (286 mg, 68% yield) as a pale yellow oil. ESI-MS m/z: 166.0 [M+H]⁺.

### Step 4: Synthesis of Intermediate 5

**Intermediate 5-3** (286 mg, 1.73 mmol) was dissolved in EtOH/H₂O (10/2 mL). Fe powder (484 mg, 8.65 mmol) and NH₄Cl (463 mg, 8.65 mmol) were added, and the mixture was stirred at 80 °C. The reaction was monitored by LC-MS until the starting material was consumed. The mixture was filtered directly by suction, and the filtrate was extracted with EA (30 mL*3). The combined organic phases were dried, concentrated, and purified by column chromatography (PE/EA = 10:1 to 1:1) to afford **intermediate 5** (187 mg, 80% yield) as a pale yellow oil. ESI-MS m/z: 136.1 [M+H]⁺.

### Preparation Example 5: Synthesis of 3-Cyano-1,5-dimethyl-1H-pyrazol-4-amine (Intermediate 6)

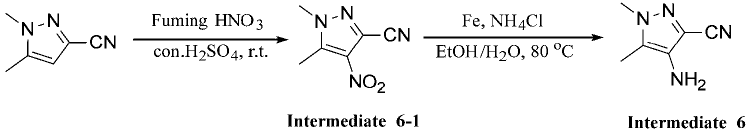

Using 3-Cyano-1,5-dimethyl-1*H*-pyrazole as the raw material, **intermediate 6** can be synthesized by a synthetic approach similar to that described in **preparation example 2.**

| Compound | Structure | Name | ESI-MS: [M+H]⁺ |
|---|---|---|---|
| **Intermediate 6** | 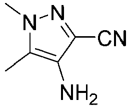 | 3-Cyano-1,5-dimethyl-1*H-*pyrazol-4-amine | 137.1 |

### Preparation Example 6: Synthesis of 3-(Trifluoromethyl)-1,5-dimethyl-1H-pyrazol-4-amine (Intermediate 7)

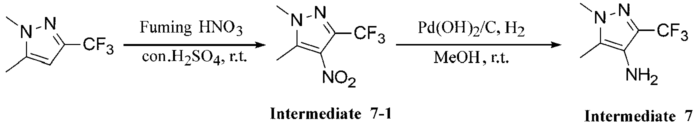

Using the corresponding substituted pyrazoles as starting materials and following the synthetic route of **intermediate 7, intermediate 7-9** can be synthesized by a synthetic approach similar to that described in **preparation example 1.**

| Compound | Structure | Name | ESI-MS: [M+H]⁺ |
|---|---|---|---|
| **Intermediate 7** | 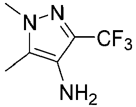 | 3-(Trifluoromethyl)-1,5-dimethyl-1*H*-pyrazol-4-amine | 180.1 |
| **Intermediate 8** | 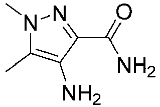 | 4-Amino-1,5-dimethyl-1*H-*pyrazole-3-carboxamide | 155.1 |
| **Intermediate 9** | 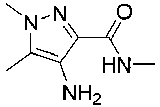 | 4-Amino-*N*,1,5-trimethyl-1*H-*pyrazole-3-carboxamide | 169.1 |

### Preparation Example 7: Synthesis of 1,5-Dimethyl-3-(pyrrolidin-1-yl)-1H-pyrazol-4-amine (Intermediate 10)

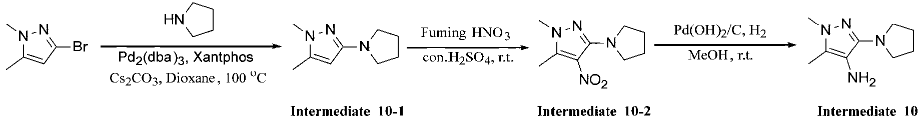

### Step 1: Synthesis of Intermediate 10-1

3-Bromo-1,5-dimethylpyrazole (1 g, 5.71 mmol) and pyrrolidine (487 mg, 6.85 mmol) were dissolved in 1,4-dioxane (30 mL). Pd₂(dba)₃ (265 mg, 0.29 mmol), Xantphos (330 mg, 0.57 mmol), and Cs₂CO₃ (3.72 g, 11.42 mmol) were added, and the mixture was stirred at 100 °C. The reaction was monitored by LC-MS until the starting material was consumed. The mixture was concentrated directly to remove 1,4-dioxane, poured into water (50 mL), and extracted with EA (30 mL*3). The combined organic phases were dried, concentrated, and purified by column chromatography (PE/EA = 20:1 to 5:1) to afford **intermediate 10-1** (490 mg, 52% yield) as a yellow solid. ESI-MS m/z: 166.1 [M+H]⁺.

Subsequently, using a synthetic method similar to that described in **preparation example 1, Intermediate 10** can be obtained.

| Compound | Structure | Name | ESI-MS: [M+H]⁺ |
|---|---|---|---|
| **Intermediate 10** | 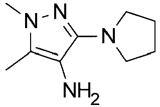 | 1,5-Dimethyl-3-(pyrrolidin-1-yl)-1*H*-pyrazol-4-amine | 181.1 |

### Preparation Example 8: Synthesis of 4-Amino-N,N,1,5-tetramethyl-1H-pyrazole-3-carboxamide (Intermediate 11)

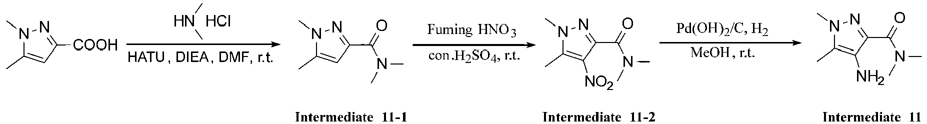

### Step 1: Synthesis of Intermediate 11-1

3-Carboxy-1,5-dimethylpyrazole (1 g, 7.14 mmol) and dimethylamine hydrochloride (582 mg, 7.14 mmol) were dissolved in DMF (30 mL). HATU (4.07 g, 10.71 mmol) and DIEA (2.76 g, 21.42 mmol) were added, and the mixture was stirred at room temperature. The reaction was monitored by LC-MS until the starting material was consumed. The mixture was poured into water (50 mL) and extracted with EA (30 mL*3). The combined organic phases were dried, concentrated, and purified by column chromatography (PE/EA = 10:1 to 2:1) to afford **intermediate 11-1** (1.03 g, 86% yield) as a yellow solid. ESI-MS m/z: 168.1 [M+H]⁺.

Subsequently, using a synthetic method similar to that described in **preparation example 1, Intermediate 11** can be obtained.

| Compound | Structure | Name | ESI-MS: [M+H]⁺ |
|---|---|---|---|
| **Intermediate 11** | 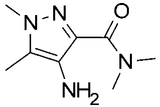 | 4-Amino-*N*,*N*,1,5-tetramethyl-1*H*-pyrazole-3-carboxamide | 183.1 |

### Preparation Example 9: Synthesis of 2-(4-Amino-1,5-dimethyl-1H-pyrazol-3-yl)acetonitrile (Intermediate 12)

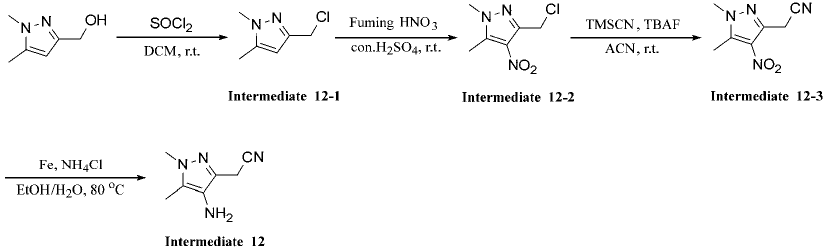

### Step 1: Synthesis of Intermediate 12-1

(1,5-dimethylpyrazol-3-yl)methanol (2 g, 15.87 mmol) was dissolved in DCM (30 mL). SOCl₂ (5.67 g, 47.62 mmol) was added slowly, and the mixture was stirred at room temperature. The reaction was monitored by LC-MS until the starting material was consumed. The mixture was concentrated directly to afford **intermediate 12-1** (2.4 g, quantitative yield) as a white solid. ESI-MS m/z: 145.0 [M+H]⁺.

### Step 2: Synthesis of Intermediate 12-2

**Intermediate 12-1** (1 g, 6.94 mmol) was dissolved in concentrated sulfuric acid (5 mL). Fuming nitric acid (15 mL) was added slowly at 0 °C, and the mixture was stirred at room temperature. The reaction was monitored by LC-MS until the starting material was consumed. The reaction mixture was poured slowly into water (100 mL) and neutralized to weakly basic with saturated aqueous sodium bicarbonate. The mixture was extracted with EA (50 mL*3). The combined organic phases were dried, concentrated, and purified by column chromatography (PE/EA = 10:1 to 2:1) to afford **intermediate 12-2** (720 mg, 55% yield) as a pale yellow solid. ESI-MS m/z: 190.0 [M+H]⁺.

### Step 3: Synthesis of Intermediate 12-3

**Intermediate 12-2** (720 mg, 3.81 mmol) was dissolved in ACN (20 mL). TMSCN (756 mg, 7.62 mmol) and TBAF (1.99 g, 7.62 mmol) were added, and the mixture was stirred at room temperature. The reaction was monitored by LC-MS until the starting material was consumed. The mixture was extracted with EA (30 mL* 3). The combined organic phases were dried, concentrated, and purified by column chromatography (PE/EA = 10:1 to 5:1) to afford **intermediate 12-3** (328 mg, 48% yield) as a pale yellow solid. ESI-MS m/z: 181.1 [M+H]⁺.

### Step 4: Synthesis of Intermediate 12

**Intermediate 12-3** (328 mg, 1.82 mmol) was dissolved in EtOH/H₂O (10/2 mL). Fe powder (510 mg, 9.11 mmol) and NH₄Cl (487 mg, 9.11 mmol) were added, and the mixture was stirred at 80 °C. The reaction was monitored by LC-MS until the starting material was consumed. The mixture was filtered directly by suction, and the filtrate was extracted with EA (30 mL*3). The combined organic phases were dried, concentrated, and purified by column chromatography (PE/EA = 10:1 to 1:1) to afford **intermediate 12** (205 mg, 75% yield) as a pale yellow oil. ESI-MS m/z: 151.1 [M+H]⁺.

### Preparation Example 10: Synthesis of 3-(((tert-Butyldimethylsilyl)oxy)methyl)-1,5-dimethyl-1H-pyrazol-4-amine (Intermediate 13)

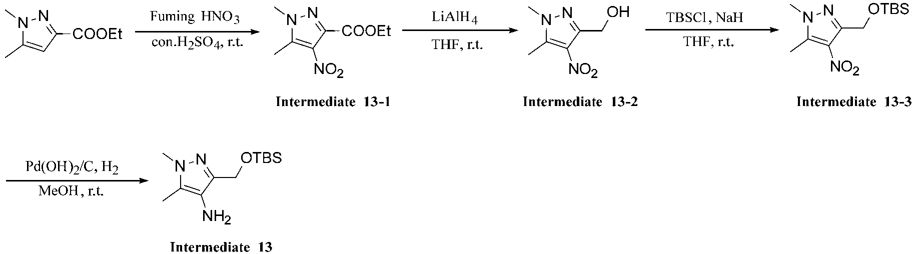

### Step 1: Synthesis of Intermediate 13-1

Ethyl 1,5-dimethyl-1*H*-pyrazole-3-carboxylate (1 g, 5.95 mmol) was dissolved in concentrated sulfuric acid (5 mL). Fuming nitric acid (15 mL) was added slowly at 0 °C, and the mixture was stirred at room temperature. The reaction was monitored by LC-MS until the starting material was consumed. The reaction mixture was poured slowly into water (100 mL) and neutralized to weakly basic with saturated aqueous sodium bicarbonate. The mixture was extracted with EA (50 mL*3). The combined organic phases were dried, concentrated, and purified by column chromatography (PE/EA = 10:1 to 2:1) to afford **intermediate 13-1** (672 mg, 53% yield) as a pale yellow solid. ESI-MS m/z: 214.1 [M+H]⁺.

### Step 2: Synthesis of Intermediate 13-2

**Intermediate 13-1** (672 mg, 3.15 mmol) was dissolved in THF (20 mL). LiAlH₄ (179 mg, 4.73 mmol) was added slowly, and the mixture was stirred at room temperature. The reaction was monitored by LC-MS until the starting material was consumed. The reaction was quenched with saturated aqueous ammonium chloride, filtered by suction, and extracted with EA (30 mL*3). The combined organic phases were dried and concentrated to afford **intermediate 13-2** (479 mg, 89% yield) as a pale yellow liquid. ESI-MS m/z: 172.1 [M+H]⁺.

### Step 3: Synthesis of Intermediate 13-3

**Intermediate 13-2** (200 mg, 1.17 mmol) was dissolved in THF (20 mL). NaH (94 mg, 2.34 mmol, 60% dispersion in mineral oil) was added slowly, and the mixture was stirred at room temperature for 10 min. TBSCl (212 mg, 1.40 mmol) was then added, and the reaction was monitored by LC-MS until the starting material was consumed. The reaction was quenched with saturated aqueous ammonium chloride, and the mixture was extracted with EA (30 mL*3). The combined organic phases were dried, concentrated, and purified by column chromatography (PE/EA = 20:1 to 5:1) to afford **intermediate 13-3** (303 mg, 91% yield) as a pale yellow liquid. ESI-MS m/z: 286.1 [M+H]⁺.

### Step 4: Synthesis of Intermediate 13

**Intermediate 13-3** (303 mg, 1.06 mmol) was dissolved in MeOH (20 mL). Pd(OH)₂/C (30 mg, 10%) was added, and the reaction vessel was purged with hydrogen gas. The mixture was stirred at room temperature under a hydrogen atmosphere, and the reaction was monitored by LC-MS until the starting material was consumed. The mixture was filtered directly by suction, and the filtrate was dried and concentrated to afford **intermediate 13** (249 mg, 92% yield) as a pale yellow oil. ESI-MS m/z: 256.2 [M+H]⁺.

Using a synthetic approach similar to that described in **preparation example 10, Intermediate 14** can be prepared.

| Compound | Structure | Name | ESI-MS: [M+H]⁺ |
|---|---|---|---|
| **Intermediate 14** | 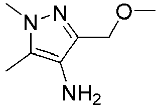 | 3-(Methoxymethyl)-1,5-dimethyl-1*H*-pyrazol-4-amine | 156.1 |

### Preparation Example 11: Synthesis of 3-(Fluoromethyl)-1,5-dimethyl-1H-pyrazol-4-amine (Intermediate 15)

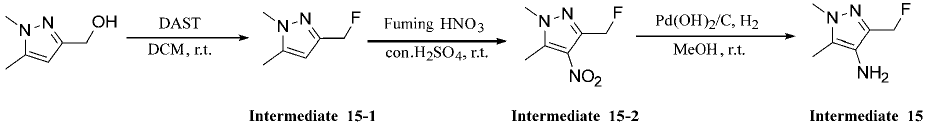

### Step 1: Synthesis of Intermediate 15-1

(1,5-Dimethyl-1*H*-pyrazol-3-yl)methanol (1 g, 7.94 mmol) was dissolved in DCM (20 mL). DAST (1.92 g, 11.90 mmol) was added, and the mixture was stirred at room temperature. The reaction was monitored by LC-MS until the starting material was consumed. The reaction was quenched by the addition of water, and the mixture was extracted with EA (30 mL*3). The combined organic phases were dried, concentrated, and purified by column chromatography (PE/EA = 20:1 to 5:1) to afford **intermediate 15-1** (823 mg, 81% yield) as a pale yellow liquid. ESI-MS m/z: 129.1 [M+H]⁺.

Subsequently, using a synthetic method similar to that described in **preparation example 1, Intermediate 15** can be obtained.

| Compound | Structure | Name | ESI-MS: [M+H]⁺ |
|---|---|---|---|
| **Intermediate 15** | 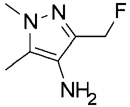 | 3-(Fluoromethyl)-1,5-dimethyl-1*H*-pyrazol-4-amine | 144.1 |

### Preparation Example 12: Synthesis of 1,5-Dimethyl-3-(2,2,2-trifluoroethyl)-1H-pyrazol-4-amine (Intermediate 16)

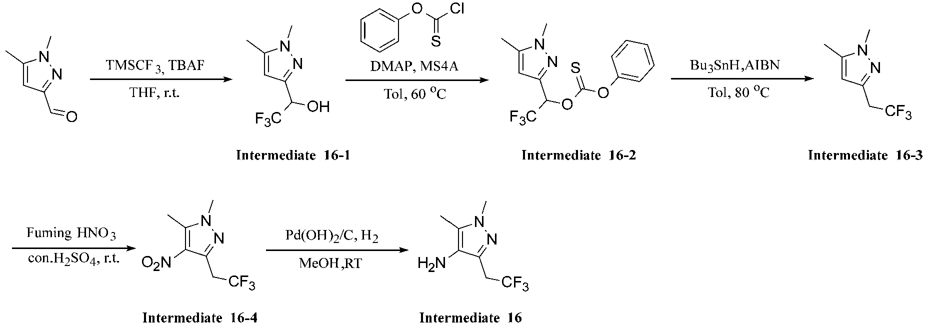

### Step 1: Synthesis of Intermediate 16-1

1,5-Dimethyl-1*H*-pyrazole-3-carbaldehyde (3.8 g, 30.61 mmol) was dissolved in THF (50 mL). TMSCF₃ (7.4 g, 52.00 mmol) and TBAF (1 M in THF, 6.1 mL) were added at room temperature, and the mixture was stirred at room temperature. The reaction was monitored by LC-MS until the starting material was consumed. The mixture was extracted with ES (50 mL *3). The combined organic phases were dried, concentrated, and purified by column chromatography (PE/EA = 10:1 to 1:1) to afford **intermediate 16-1** (5.1 g, 86% yield) as a yellow solid. ESI-MS m/z: 195.1 [M+H]⁺.

### Step 2: Synthesis of Intermediate 16-2

**Intermediate 16-1** (2 g, 10.31 mmol) was dissolved in toluene (40 mL). Phenyl chlorothionoformate (3.56 g, 20.62 mmol), 4Å molecular sieves (2 g), and DMAP (2.52 g, 20.62 mmol) were added, and the mixture was stirred at 60 °C. The reaction was monitored by LC-MS until the starting material was consumed. The mixture was extracted with EA (50 mL*3). The combined organic phases were dried, concentrated, and purified by column chromatography (PE/EA = 10:1 to 2:1) to afford **intermediate 16-2** (2.8 g, 82% yield) as a yellow liquid. ESI-MS m/z: 331.1 [M+H]⁺.

### Step 3: Synthesis of Intermediate 16-3

**Intermediate 16-2** (2.8 g, 8.48 mmol) was dissolved in toluene (50 mL). Tributyltin Hydride (9.88 g, 33.94 mmol) and AIBN (834 mg, 5.08 mmol) were added, and the mixture was stirred at 80 °C. The reaction was monitored by LC-MS until the starting material was consumed. The mixture was extracted with EA (50 mL*3). The combined organic phases were dried, concentrated, and purified by column chromatography (PE/EA = 10:1 to 1:1) to afford **intermediate 16-3** (1.1 g, 73% yield) as a yellow liquid. ESI-MS m/z: 179.1 [M+H]⁺.

Subsequently, using a synthetic method similar to that described in **preparation example 1, Intermediate 16** can be obtained.

| Compound | Structure | Name | ESI-MS: [M+H]⁺ |
|---|---|---|---|
| **Intermediate 16** | 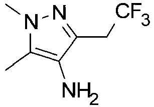 | 1,5-Dimethyl-3-(2,2,2-trifluoroethyl)-1*H*-pyrazol-4-amine | 194.1 |

### Preparation Example 13: Synthesis of 3-(2-((tert-Butyldimethylsilyl)oxy)-2-methylpropyl)-1,5-dimethyl-1H-pyrazol-4-amine (Intermediate 17)

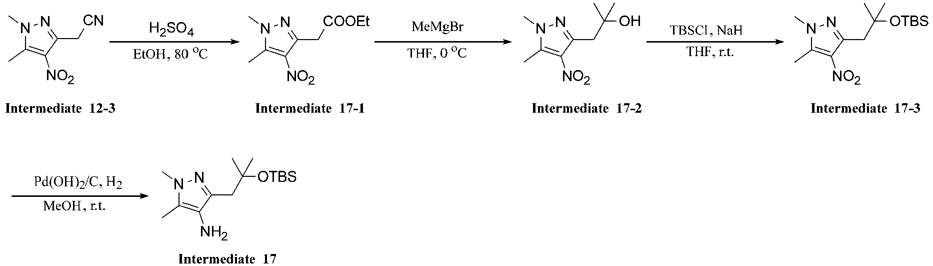

### Step 1: Synthesis of Intermediate 17-1

Intermediate 12-3 (1 g, 5.56 mmol) was dissolved in EtOH (20 mL). Concentrated sulfuric acid (5 mL) was added slowly at 0 °C, and the mixture was stirred at 80 °C. The reaction was monitored by LC-MS until the starting material was consumed. The reaction mixture was poured slowly into water (50 mL) and neutralized to weakly basic with saturated aqueous sodium bicarbonate. The mixture was extracted with EA (50 mL*3). The combined organic phases were dried, concentrated, and purified by column chromatography (PE/EA = 10:1 to 2:1) to afford **intermediate 17-1** (1.05 g, 83% yield) as a pale yellow solid. ESI-MS m/z: 228.1 [M+H]⁺.

### Step 2: Synthesis of Intermediate 17-2

**Intermediate 17-1** (1.05 g, 4.63 mmol) was dissolved in THF (20 mL). Methylmagnesium bromide (1 M in THF, 13.89 mL) was added slowly at 0 °C, and the mixture was stirred at 0 °C. The reaction was monitored by LC-MS until the starting material was consumed. The reaction was quenched with saturated aqueous ammonium chloride, filtered by suction, and extracted with EA (30 mL*3). The combined organic phases were dried, concentrated, and purified by column chromatography (PE/EA = 10:1 to 1:1) to afford **intermediate 17-2** (601 mg, 61% yield) as a pale yellow liquid. ESI-MS m/z: 214.1 [M+H]⁺.

Subsequently, using a synthetic method similar to that described in **preparation example 10, Intermediate 17** can be obtained.

| Compound | Structure | Name | ESI-MS: [M+H]⁺ |
|---|---|---|---|
| **Intermediate 17** | 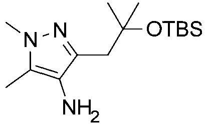 | 3-(2-((tertButyldimethylsilyl)oxy)-2-methylpropyl)-1,5-dimethyl-1*H-*pyrazol-4-amine | 298.2 |

### Preparation Example 14: Synthesis of 3-(2-Fluoro-2-methylpropyl)-1,5-dimethyl-1H-pyrazol-4-amine (Intermediate 18)

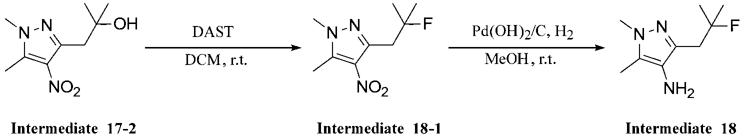

### Step 1: Synthesis of Intermediate 18-1

**Intermediate 17-2** (300 mg, 1.41 mmol) was dissolved in DCM (20 mL). DAST (341 mg, 2.11 mmol) was added, and the mixture was stirred at room temperature. The reaction was monitored by LC-MS until the starting material was consumed. The mixture was extracted with EA (30 mL*3). The combined organic phases were dried, concentrated, and purified by column chromatography (PE/EA = 20:1 to 5:1) to afford **intermediate 18-1** (212 mg, 70% yield) as a pale yellow liquid. ESI-MS m/z: 216.1 [M+H]⁺.

Subsequently, using a synthetic method similar to that described in **preparation example 1, Intermediate 18** can be obtained.

| Compound | Structure | Name | ESI-MS: [M+H]⁺ |
|---|---|---|---|
| **Intermediate 18** | 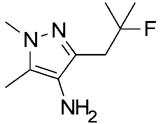 | 3-(2-Fluoro-2-methylpropyl)-1,5-dimethyl-1*H*-pyrazol-4-amine | 186.1 |

### Preparation Example 15: Synthesis of 3-(4-Amino-1,5-dimethyl-1H-pyrazol-3-yl)-2,2-dimethylpropanenitrile (Intermediate 19)

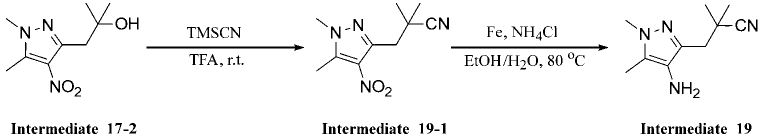

### Step 1: Synthesis of Intermediate 19-1

**Intermediate 17-2** (300 mg, 1.41 mmol) was dissolved in TFA (10 mL). TMSCN (420 mg, 4.23 mmol) was added, and the mixture was stirred at room temperature. The reaction was monitored by LC-MS until the starting material was consumed. The mixture was neutralized to weakly basic with saturated aqueous sodium bicarbonate and extracted with EA (30 mL*3). The combined organic phases were dried, concentrated, and purified by column chromatography (PE/EA = 20:1 to 5:1) to afford **intermediate 19-1** (172 mg, 55% yield) as a pale yellow liquid. ESI-MS m/z: 223.1 [M+H]⁺.

Subsequently, using a synthetic method similar to that described in **preparation example 2, Intermediate 19** can be obtained.

| Compound | Structure | Name | ESI-MS: [M+H]⁺ |
|---|---|---|---|
| **Intermediate 19** | 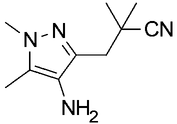 | 3-(4-Amino-1,5-dimethyl-1*H-*pyrazol-3-yl)-2,2-dimethylpropanenitrile | 193.1 |

### Preparation Example 16: Synthesis of 3-(Cyclopropylmethyl)-1,5-dimethyl-1H-pyrazol-4-amine (Intermediate 20)

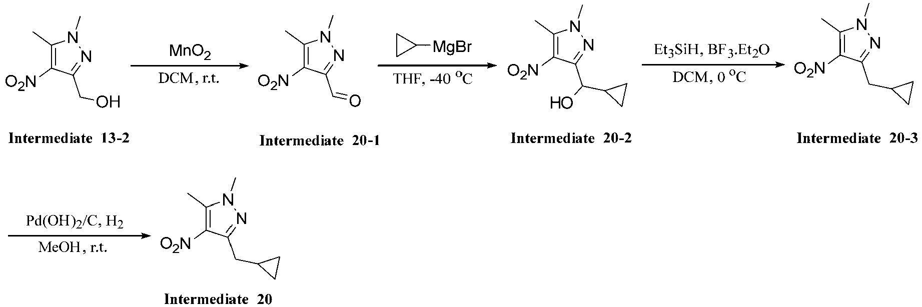

### Step 1: Synthesis of Intermediate 20-1

**Intermediate 13-2** (1 g, 5.99 mmol) was dissolved in DCM (20 mL). Activated manganese dioxide (5.21 g, 59.88 mmol) was added, and the mixture was stirred at room temperature. The reaction was monitored by LC-MS until the starting material was consumed. The mixture was filtered directly by suction and concentrated to afford **intermediate 20-1** (950 mg, 94% yield) as a pale yellow solid. ESI-MS m/z: 170.0 [M+H]⁺.

### Step 2: Synthesis of Intermediate 20-2

**Intermediate 20-1** (950 mg, 5.62 mmol) was dissolved in THF (20 mL). Cyclopropylmagnesium bromide (1 M in THF, 11.24 mL) was added slowly at -40 °C, and the mixture was stirred at -40 °C. The reaction was monitored by LC-MS until the starting material was consumed. The reaction was quenched with saturated aqueous ammonium chloride, filtered by suction, and extracted with EA (30 mL*3). The combined organic phases were dried, concentrated, and purified by column chromatography (PE/EA = 10:1 to 1:1) to afford **intermediate 20-2** (620 mg, 53% yield) as a pale yellow liquid. ESI-MS m/z: 212.1 [M+H]⁺.

### Step 3: Synthesis of Intermediate 20-3

**Intermediate 20-2** (620 mg, 2.94 mmol) was dissolved in DCM (20 mL). Boron trifluoride diethyl etherate (1.25 g, 8.82 mmol) and triethylsilane (1.02 g, 8.82 mmol) were added slowly at 0 °C, and the mixture was stirred at 0 °C. The reaction was monitored by LC-MS until the starting material was consumed. The reaction was quenched by the addition of an appropriate amount of water, filtered by suction, and extracted with DCM (30 mL*3). The combined organic phases were dried, concentrated, and purified by column chromatography (PE/EA = 10:1 to 1:1) to afford **intermediate 20-3** (220 mg, 38% yield) as a pale yellow liquid. ESI-MS m/z: 196.1 [M+H]⁺.

Subsequently, using a synthetic method similar to that described in **preparation example 1, Intermediate 20** can be obtained.

| Compound | Structure | Name | ESI-MS: [M+H]⁺ |
|---|---|---|---|
| **Intermediate 20** | 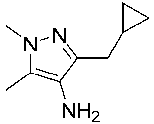 | 3-(Cyclopropylmethyl)-1,5-dimethyl-1*H*-pyrazol-4-amine | 166.1 |

Using a synthetic approach similar to that described in **preparation example 16, Intermediate 21-22** can be obtained.

| Compound | Structure | Name | ESI-MS: [M+H]⁺ |
|---|---|---|---|
| **Intermediate 21** | 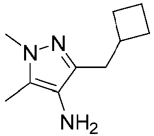 | 3-(Cyclobutylmethyl)-1,5-dimethyl-1*H*-pyrazol-4-amine | 180.1 |
| **Intermediate 22** | 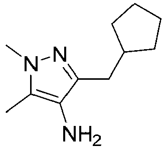 | 3-(Cyclopentylmethyl)-1,5-dimethyl-1*H*-pyrazol-4-amine | 194.2 |

### Preparation Example 17: Synthesis of 3-((3,3-Difluorocyclobutyl)methyl)-1,5-dimethyl-1H-pyrazol-4-amine (Intermediate 23)

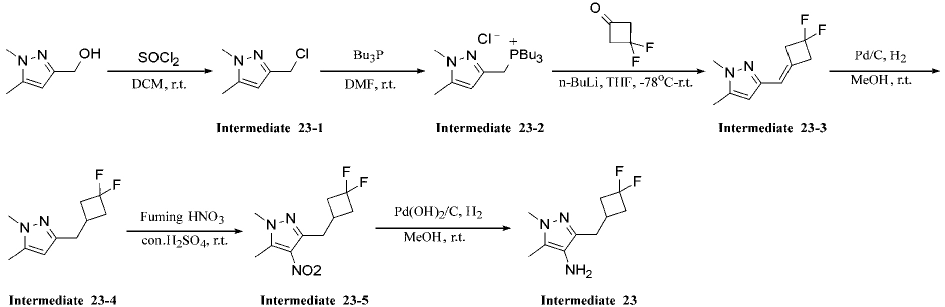

### Step 1: Synthesis of Intermediate 23-1

(1,5-Dimethyl-1*H*-pyrazol-3-yl)methanol (1 g, 7.94 mmol) was dissolved in DCM (20 mL). SOCl₂ (1.42 g, 11.90 mmol) was added, and the mixture was stirred at room temperature. The reaction was monitored by LC-MS until the starting material was consumed. The mixture was concentrated directly to afford **intermediate 23-1** (1.09 g, 95% yield) as a white solid. ESI-MS m/z: 145.0 [M+H]⁺.

### Step 2: Synthesis of Intermediate 23-2

**Intermediate 23-1** (1.09 g, 7.57 mmol) was dissolved in DMF (20 mL). Tributylphosphine (2.29 g, 11.36 mmol) was added, and the mixture was stirred at room temperature. The reaction was monitored by LC-MS until the starting material was consumed. The mixture was concentrated directly and purified by column chromatography (DCM/MeOH = 20:1 to 10:1) to afford **intermediate 23-2** (2.18 g, 83% yield) as a white solid. ESI-MS m/z: 312.3 [M+H]⁺.

### Step 3: Synthesis of Intermediate 23-3

**Intermediate 23-2** (2.18 g, 6.28 mmol) was dissolved in THF (30 mL). ⁿ⁻BuLi (2.5 M in hexane, 3 mL, 7.54 mmol) was added at -78 °C, and the mixture was stirred at 0 °C for 1 h. The mixture was cooled back to -78 °C, and a solution of 3,3-difluorocyclobutanone (799 mg, 7.54 mmol) in THF was added. The reaction mixture was allowed to warm slowly to room temperature and monitored by LC-MS until the starting material was consumed. Saturated aqueous ammonium chloride was added to quench the reaction, and the mixture was extracted with EA (50 mL*3). The combined organic phases were dried, concentrated, and purified by column chromatography (PE/EA = 20:1 to 5:1) to afford **intermediate 23-3** (435 mg, 35% yield) as a yellow liquid. ESI-MS m/z: 199.1 [M+H]⁺.

Subsequently, using a synthetic method similar to that described in **preparation example 1, Intermediate 23** can be obtained.

| Compound | Structure | Name | ESI-MS: [M+H]⁺ |
|---|---|---|---|
| **Intermediate 23** | 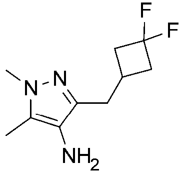 | 3-((3,3-Difluorocyclobutyl)methyl)-1,5-dimethyl-1*H*-pyrazol-4-amine | 216.1 |

Using a synthetic approach similar to that described in **preparation example 17, Intermediate 24** can be prepared.

| Compound | Structure | Name | ESI-MS: [M+H]⁺ |
|---|---|---|---|
| **Intermediate 24** | 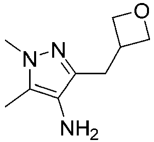 | 1,5-Dimethyl-3-(oxetan-3-ylmethyl)-1*H*-pyrazol-4-amine | 182.1 |

### Preparation Example 18: Synthesis of 3-((Dimethylamino)methyl)-1,5-dimethyl-1H-pyrazol-4-amine (Intermediate 25)

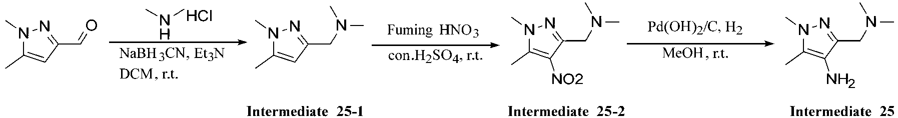

### Step 1: Synthesis of Intermediate 25-1

1,5-Dimethylpyrazole-3-carbaldehyde (1 g, 8.06 mmol) and dimethylamine hydrochloride (1.31 g, 16.13 mmol) were dissolved in DCM (20 mL). TEA (2.44 g, 24.18 mmol) and NaBH₃CN (1.52 g, 24.18 mmol) were added, and the mixture was stirred at room temperature. The reaction was monitored by LC-MS until the starting material was consumed. The mixture was extracted with DCM (30 mL*3). The combined organic phases were dried, concentrated, and purified by column chromatography (DCM/MeOH = 20:1 to 10:1) to afford **intermediate 25-1** (521 mg, 42% yield) as a yellow liquid. ESI-MS m/z: 154.1 [M+H]⁺.

Subsequently, using a synthetic method similar to that described in **preparation example 1, Intermediate 25** can be obtained.

| Compound | Structure | Name | ESI-MS: [M+H]⁺ |
|---|---|---|---|
| **Intermediate 25** | 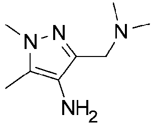 | 3-((Dimethylamino)methyl)-1,5-dimethyl-1*H*-pyrazol-4-amine | 169.1 |

Using a synthetic approach similar to that described in **preparation example 18, Intermediate 26-29** can be obtained.

| Compound | Structure | Name | ESI-MS: [M+H]⁺ |
|---|---|---|---|
| **Intermediate 26** | 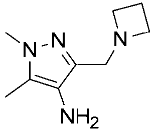 | 3-(Azetidin-1-ylmethyl)-1,5-dimethyl-1*H*-pyrazol-4-amine | 181.1 |
| **Intermediate 27** | 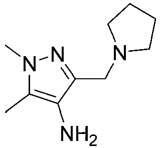 | 1,5-Dimethyl-3-(pyrrolidin-1-ylmethyl)-1*H*-pyrazol-4-amine | 195.1 |
| **Intermediate 28** | 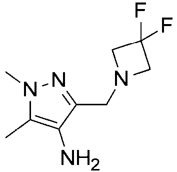 | 3-[(3,3-difluoroazetidin-1-yl)methyl]-1,5-dimethylpyrazol-4-amine | 217.1 |
| **Intermediate 29** | 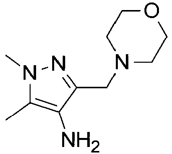 | 1,5-dimethyl-3-(1,4-oxazinan-4-ylmethyl)pyrazol-4-amine | 211.1 |

### Preparation Example 19: Synthesis of 1-((4-Amino-1,5-dimethyl-1H-pyrazol-3-yl)methyl)pyrrolidin-2-one (Intermediate 30)

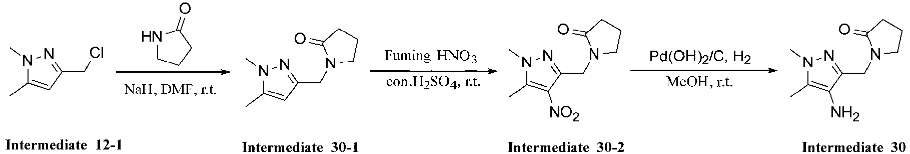

### Step 1: Synthesis of Intermediate 30-1

2-Pyrrolidinone (155 mg, 1.82 mmol) was dissolved in DMF (20 mL). NaH (73 mg, 1.82 mmol, 60% dispersion in mineral oil) was added under ice-bath cooling, and the mixture was stirred at room temperature for 10 min. A solution of Intermediate 12-1 (300 mg, 1.52 mmol) in DMF was then added, and the mixture was stirred at room temperature. The reaction was monitored by LC-MS until the starting material was consumed. Saturated aqueous ammonium chloride was added to quench the reaction, and the mixture was extracted with EA (30 mL*3). The combined organic phases were dried, concentrated, and purified by column chromatography (PE/EA = 10:1 to 2:1) to afford **intermediate 30-1** (223 mg, 76% yield) as a yellow solid. ESI-MS m/z: 194.1 [M+H]⁺.

Subsequently, using a synthetic method similar to that described in **preparation example 1, Intermediate 30** can be obtained.

| Compound | Structure | Name | ESI-MS: [M+H]⁺ |
|---|---|---|---|
| **Intermediate 30** | 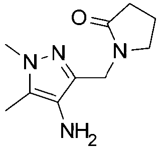 | 1-((4-Amino-1,5-dimethyl-1*H-*pyrazol-3-yl)methyl)pyrrolidin-2-one | 209.1 |

### Preparation Example 20: Synthesis of 2-(4-Amino-1,5-dimethyl-1H-pyrazol-3-yl)acetamide (Intermediate 31)

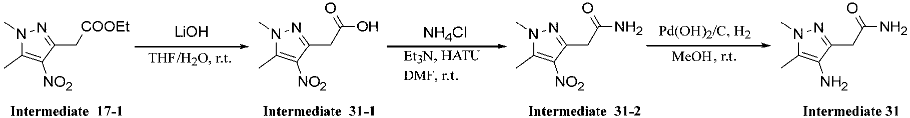

### Step 1: Synthesis of Intermediate 31-1

**Intermediate 17-1** (500 mg, 2.20 mmol) was dissolved in THF/H₂O (20/4 mL). LiOH (106 mg, 4.40 mmol) was added, and the mixture was stirred at room temperature. The reaction was monitored by LC-MS until the starting material was consumed. The mixture was neutralized to weakly acidic with 1 M hydrochloric acid and extracted with EA (50 mL*3). The combined organic phases were dried and concentrated to afford **intermediate 31-1** (398 mg, 91% yield) as a pale yellow solid. ESI-MS m/z: 200.1 [M+H]⁺.

### Step 2: Synthesis of Intermediate 31-2

**Intermediate 31-1** (398 mg, 2.00 mmol) was dissolved in DMF (20 mL). NH₄Cl (321 mg, 6.00 mmol), HATU (1.14 g, 3.00 mmol), and TEA (606 mg, 6.00 mmol) were added, and the mixture was stirred at room temperature. The reaction was monitored by LC-MS until the starting material was consumed. The mixture was extracted with EA (30 mL*3). The combined organic phases were dried, concentrated, and purified by column chromatography (PE/EA = 10:1 to 1:1) to afford **intermediate 31-2** (318 mg, 80% yield) as a pale yellow solid. ESI-MS m/z: 199.1 [M+H]⁺.

Subsequently, using a synthetic method similar to that described in **preparation example 1, Intermediate 31** can be obtained.

| Compound | Structure | Name | ESI-MS: [M+H]⁺ |
|---|---|---|---|
| **Intermediate 31** | 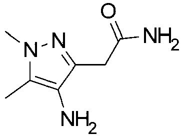 | 2-(4-Amino-1,5-dimethyl-1*H-*pyrazol-3 -yl)acetamide | 169.1 |

Using a synthetic approach similar to that described in **preparation example 20, Intermediate 32-33** can be prepared.

| Compound | Structure | Name | ESI-MS: [M+H]⁺ |
|---|---|---|---|
| **Intermediate 32** | 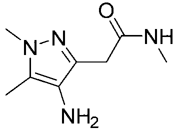 | 2-(4-Amino-1,5-dimethyl-1*H-*pyrazol-3-yl)-*N-*methylacetamide | 183.1 |
| **Intermediate 33** | 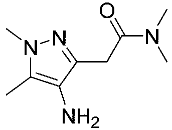 | 2-(4-Amino-1,5-dimethyl-1*H-*pyrazol-3-yl)-*N*,*N-*dimethylacetamide | 197.1 |

### Preparation Example 21: Synthesis of N-((4-Amino-1,5-dimethyl-1H-pyrazol-3-yl)methyl)acetamide (Intermediate 34)

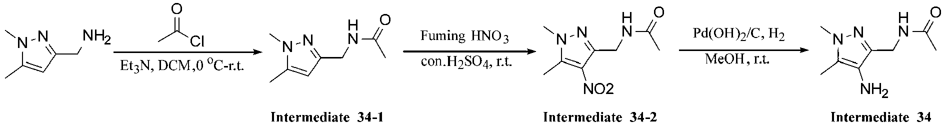

### Step 1: Synthesis of Intermediate 34-1

(1,5-Dimethyl-1*H*-pyrazol-3-yl)methanamine (300 mg, 2.40 mmol) and TEA (485 mg, 4.80 mmol) were dissolved in DCM (20 mL). A solution of acetyl chloride (198 mg, 2.52 mmol) in DCM was added at 0 °C, and the mixture was stirred at room temperature. The reaction was monitored by LC-MS until the starting material was consumed. The mixture was extracted with DCM (30 mL*3). The combined organic phases were dried, concentrated, and purified by column chromatography (PE/EA = 10:1 to 3:1) to afford **intermediate 34-1** (353 mg, 88% yield) as a yellow solid. ESI-MS m/z: 168.1 [M+H]⁺.

Subsequently, using a synthetic method similar to that described in **preparation example 1, Intermediate 34** can be obtained.

| Compound | Structure | Name | ESI-MS: [M+H]⁺ |
|---|---|---|---|
| **Intermediate 34** | 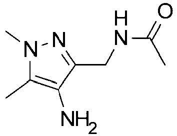 | *N*-((4-Amino-1,5-dimethyl-1*H-*pyrazol-3-yl)methyl)acetamide | 183.1 |

### Preparation Example 22: Synthesis of 3-(2-Fluoroethyl)-1,5-dimethyl-1H-pyrazol-4-amine (Intermediate 35)

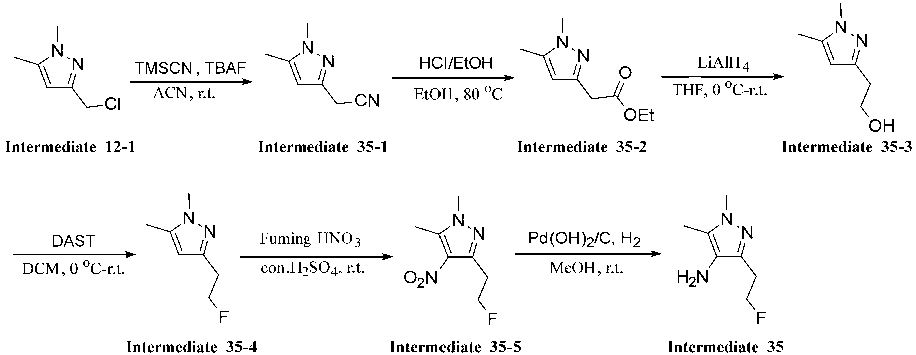

### Step 1: Synthesis of Intermediate 35-1

**Intermediate 12-1** (2 g, 13.89 mmol) was dissolved in acetonitrile (50 mL). TMSCN (2.76 g, 27.78 mmol) and TBAF (1 M in THF, 27.78 mL) were added at room temperature, and the mixture was stirred at room temperature. The reaction was monitored by LC-MS until the starting material was consumed. The mixture was extracted with EA (50 mL*3). The combined organic phases were dried, concentrated, and purified by column chromatography (PE/EA = 10:1 to 2:1) to afford **intermediate 35-1** (1.1 g, 59% yield) as a yellow solid. ESI-MS m/z: 136.1 [M+H]⁺.

### Step 2: Synthesis of Intermediate 35-2

**Intermediate 35-1** (1.1 g, 8.15 mmol) was dissolved in EtOH (20 mL). HCl/EtOH (2 M, 20.4 mL) was added, and the mixture was stirred at 80 °C. The reaction was monitored by LC-MS until the starting material was consumed. The mixture was concentrated to remove ethanol, neutralized to weakly basic with saturated aqueous sodium bicarbonate, and extracted with EA (50 mL*3). The combined organic phases were dried, concentrated, and purified by column chromatography (PE/EA = 10:1 to 2:1) to afford **intermediate 35-2** (1.15 g, 78% yield) as a pale yellow solid. ESI-MS m/z: 183.1 [M+H]⁺.

### Step 3: Synthesis of Intermediate 35-3

**Intermediate 35-2** (1.15 g, 6.32 mmol) was dissolved in THF (20 mL). LiAlH₄ (360 mg, 9.48 mmol) was added slowly under ice-bath cooling, and the mixture was stirred at room temperature. The reaction was monitored by LC-MS until the starting material was consumed. The reaction was quenched with saturated aqueous ammonium chloride, filtered by suction, and extracted with EA (30 mL*3). The combined organic phases were dried and concentrated to afford **intermediate 35-3** (580 mg, 66% yield) as a pale yellow liquid. ESI-MS m/z: 141.1 [M+H]⁺.

### Step 4: Synthesis of Intermediate 35-4

**Intermediate 35-3** (580 mg, 4.14 mmol) was dissolved in DCM (20 mL). DAST (1.00 g, 6.21 mmol) was added under ice-bath cooling, and the mixture was then stirred at room temperature. The reaction was monitored by LC-MS until the starting material was consumed. The mixture was extracted with DCM (30 mL*3). The combined organic phases were dried, concentrated, and purified by column chromatography (PE/EA = 10:1 to 1:1) to afford **intermediate 35-4** (260 mg, 44% yield) as a pale yellow liquid. ESI-MS m/z: 143.1 [M+H]⁺.

Subsequently, using a synthetic method similar to that described in **preparation example 1, Intermediate 35** can be obtained.

| Compound | Structure | Name | ESI-MS: [M+H]⁺ |
|---|---|---|---|
| **Intermediate 35** | 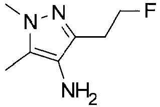 | 3-(2-Fluoroethyl)-1,5-dimethyl-1*H*-pyrazol-4-amine | 158.1 |

### Preparation Example 23: Synthesis of 3-(2,2-Difluoroethyl)-1,5-dimethyl-1H-pyrazol-4-amine (Intermediate 36)

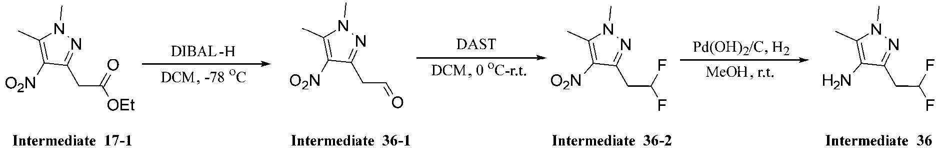

### Step 1: Synthesis of Intermediate 36-1

**Intermediate 17-1** (1 g, 4.41 mmol) was dissolved in DCM (20 mL). DIBAL-H (1 M in toluene, 8.82 mL) was added at -78 °C, and the mixture was stirred at -78 °C. The reaction was monitored by LC-MS until the starting material was consumed. The reaction was quenched with saturated aqueous ammonium chloride and extracted with EA (30 mL*3). The combined organic phases were dried, concentrated, and purified by column chromatography (PE/EA = 10:1 to 2:1) to afford **intermediate 36-1** (610 mg, 76% yield) as a pale yellow liquid. ESI-MS m/z: 184.1 [M+H]⁺.

Subsequently, using a synthetic method similar to that described in **preparation example 14, Intermediate 36** can be obtained.

| Compound | Structure | Name | ESI-MS: [M+H]⁺ |
|---|---|---|---|
| **Intermediate 36** | 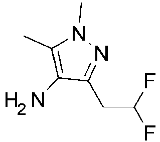 | 3-(2,2-Difluoroethyl)-1,5-dimethyl-1*H*-pyrazol-4-amine | 176.1 |

### Preparation Example 24: Synthesis of tert-Butyl 8-bromo-3,4-dihydrobenzo[4,5]thieno[3,2-c]pyridine-2(1H)-carboxylate (Intermediate 37) and tert-Butyl 6-bromo-3,4-dihydrobenzo[4,5]thieno[2,3-c]pyridine-2(1H)-carboxylate (Intermediate 38)

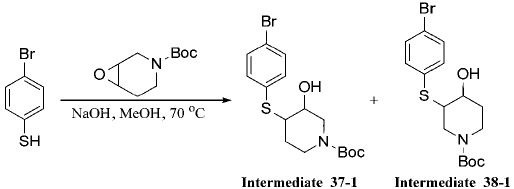

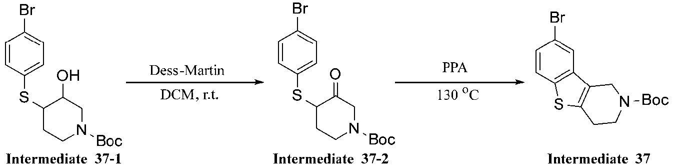

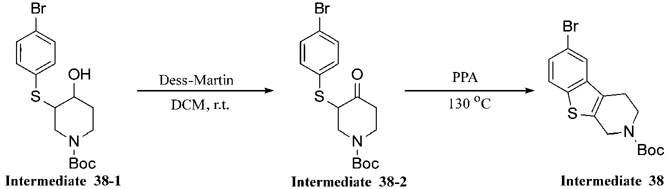

### Step 1: Synthesis of Intermediate 37-1 and Intermediate 38-1

4-Bromothiophenol (5 g, 26.46 mmol) was dissolved in methanol (80 mL). NaOH (1.06 g, 26.46 mmol) and *tert*-butyl 7-oxa-3-azabicyclo[4.1.0]heptane-3-carboxylate (5.27 g, 26.46 mmol) were added, and the mixture was stirred at 70 °C. The reaction was monitored by LC-MS until the starting material was consumed. The mixture was extracted with EA (50 mL*3). The combined organic phases were dried, concentrated, and purified by column chromatography (PE/EA = 10:1 to 2:1) to afford **intermediate 37-1** (8.0 g, 78% yield) as a colorless oil, ESI-MS m/z: 388.0 [M+H]⁺, and **intermediate 38-1** (1.6 g, 16% yield) as a colorless oil, ESI-MS m/z: 388.0 [M+H]⁺.

### Step 2: Synthesis of Intermediate 37-2

**Intermediate 37-1** (8 g, 20.62 mmol) was dissolved in DCM (100 mL). Dess-Martin periodinane (11.37 g, 26.80 mmol) was added, and the mixture was stirred at room temperature. The reaction was monitored by LC-MS until the starting material was consumed. The reaction was quenched with saturated aqueous sodium sulfite, and the mixture was extracted with DCM (50 mL*3). The combined organic phases were dried and concentrated to afford **intermediate 37-2** (7.5 g, 94% yield) as a pale yellow oil. ESI-MS m/z: 386.0 [M+H]⁺.

### Step 3: Synthesis of Intermediate 37

**Intermediate 37-2** (7 g, 18.13 mmol) was dissolved in polyphosphoric acid (50 mL). The mixture was stirred at 130 °C, and the reaction was monitored by LC-MS until the starting material was consumed. The mixture was diluted with THF and methanol, neutralized to weakly basic with 50% aqueous sodium hydroxide, and Boc₂O (7.91 g, 36.26 mmol) was added and stirred at room temperature for 1 h. The mixture extracted with EA (50 mL*3), the combined organic phases were dried, concentrated, and purified by column chromatography (PE/EA = 10:1 to 1:1) to afford **intermediate 37** (1.4 g, 21% yield) as a yellow solid. ESI-MS m/z: 368.0 [M+H]⁺.

**Intermediate 38** can be synthesized by the sane synthetic approach.

| Compound | Structure | Name | ESI-MS: [M+H]⁺ |
|---|---|---|---|
| **Intermediate 38** | 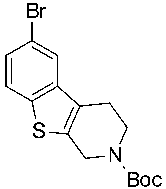 | *tert-*Butyl 6-bromo-3,4-dihydrobenzo[4,5]thieno[2,3-c]pyridine-2(1*H*)-carboxylate | 368.0 |

### Preparation Example 25: Synthesis of tert-Butyl 8-bromo-3,4-dihydrobenzofuro[3,2-c]pyridine-2(1H)-carboxylate (Intermediate 39)

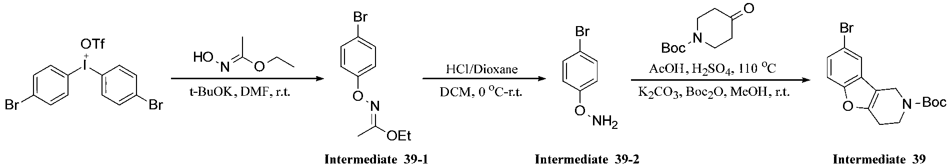

### Step 1: Synthesis of Intermediate 39-1

Ethyl acetohydroxamate (526 mg, 5.10 mmol) was dissolved in DMF (30 mL), and potassium *tert*-butoxide (572 mg, 5.10 mmol) was added. After stirring at room temperature for 5 minutes, bis(4-bromophenyl)iodonium trifluoromethanesulfonate (2 g, 3.40 mmol) was added. The reaction mixture was stirred at room temperature and monitored by LC-MS until the starting material was consumed. The mixture was extracted with EA (50 mL* 3), and the combined organic phases were dried, concentrated, and purified by column chromatography (PE/EA = 10/1 to 2/1) to afford **intermediate 39-1** (700 mg, 80% yield) as a colorless oil. ESI-MS m/z: 258.0 [M+H]⁺.

### Step 2: Synthesis of Intermediate 39-2

**Intermediate 39-1** (700 mg, 2.72 mmol) was dissolved in DCM (20 mL), and HCl/dioxane (4 M, 5 mL) was added at 0 °C. The reaction mixture was stirred at room temperature and monitored by LC-MS until the starting material was consumed. The mixture was concentrated directly to give **intermediate 39-2** (505 mg, 99% yield) as a white solid. ESI-MS m/z: 188.0 [M+H]⁺.

### Step 3: Synthesis of Intermediate 39

**Intermediate 39-2** (505 mg, 2.70 mmol) and *N*-tert-butyloxycarbonylpiperidin-4-one (592 mg, 2.97 mmol) was dissolved in acetic acid/concentrated sulfuric acid (10/2 mL), and the mixture was stirred at 110 °C. The reaction was monitored by LC-MS until the starting material was consumed. The mixture was concentrated directly, neutralized to slightly basic with saturated aqueous sodium bicarbonate, and extracted with EA (30 mL*3). The combined organic phases were dried, concentrated, and the residue was dissolved in MeOH (20 mL). K₂CO₃ (745 mg, 5.40 mmol) and Boc₂O (1.18 g, 5.40 mmol) were added, and the mixture was stirred at room temperature for 1 h. The mixture was then extracted with EA (50 mL*3), and the combined organic phases were dried, concentrated, and purified by column chromatography (PE/EA = 10/1 to 2/1) to afford **intermediate 39** (768 mg, 81% yield) as a yellow oil. ESI-MS m/z: 352.0 [M+H]⁺.

### Preparation Example 26: Synthesis of tert-Butyl 9-bromo-3,4-dihydropyrazolo[1,5-a:4,3-c']dipyridine-2(1H)-carboxylate (Intermediate 40)

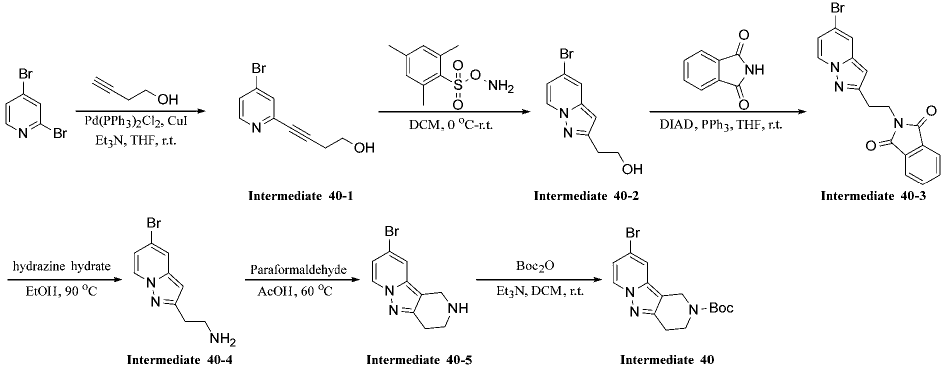

### Step 1: Synthesis of Intermediate 40-1

2,4-Dibromopyridine (15.00 g, 63.30 mmol) and 3-butyn-1-ol (4.40 g, 63.30 mmol) were dissolved in THF (150 mL). Pd(PPh₃)₂Cl₂ (2.22 g, 3.17 mmol), CuI (1.21 g, 6.33 mmol), and TEA (12.83 g, 127.00 mmol) were added. The reaction mixture was stirred at room temperature and monitored by LC-MS until the starting materials were consumed. The mixture was extracted with EA (100 mL*3), and the combined organic phases were dried, concentrated, and purified by column chromatography (PE/EA = 5/1 to 0/1) to afford **intermediate 40-1** (12.00 g, 85% yield) as a yellow solid. ESI-MS m/z: 226.0 [M+H]⁺.

### Step 2: Synthesis of Intermediate 40-2

**Intermediate 40-1** (7.00 g, 30.95 mmol) was dissolved in DCM (120 mL), and 2,4,6-trimethylbenzenesulfonyl hydroxylamine (17.50 g, 81.51 mmol) was added at 0 °C. The reaction mixture was stirred at room temperature and monitored by LC-MS until the starting material was consumed. The mixture was extracted with EA (50 mL*3), and the combined organic phases were dried, concentrated, and purified by column chromatography (PE/EA = 5/1 to 0/1) to afford **intermediate 40-2** (5.50 g, 74% yield) as a yellow solid. ESI-MS m/z: 241.0 [M+H]⁺.

### Step 3: Synthesis of Intermediate 40-3

**Intermediate 40-2** (4.00 g, 16.60 mmol) and phthalimide (2.90 g, 19.90 mmol) were dissolved in THF (100 mL). DIAD (4.00 g, 19.90 mmol) and PPh₃ (5.20 g, 19.90 mmol) were added, and the reaction mixture was stirred at room temperature. The reaction was monitored by LC-MS until the starting material was consumed. The mixture was extracted with EA (50 mL*3), and the combined organic phases were dried, concentrated, and purified by column chromatography (PE/EA = 5/1 to 0/1) to afford **intermediate 40-3** (5.20 g, 85% yield) as a yellow solid. ESI-MS m/z: 370.0 [M+H]⁺.

### Step 4: Synthesis of Intermediate 40-4

**Intermediate 40-3** (5.20 g, 14.09 mmol) was dissolved in EtOH (100 mL), and hydrazine hydrate (4.09 g, 85%) was added. The reaction was stirred at 90 °C and monitored by LC-MS until the starting material was consumed. The mixture was filtered by suction, extracted with EA (50 mL*3), and the combined organic phases were dried, concentrated, and purified by column chromatography (DCM/MeOH = 20/1 to 10/1) to afford **intermediate 40-4** (2.53 g, 75% yield) as a yellow liquid. ESI-MS m/z: 240.0 [M+H]⁺.

### Step 5: Synthesis of Intermediate 40-5

**Intermediate 40-4** (1.05 g, 4.37 mmol) was dissolved in AcOH (20 mL), and paraformaldehyde (787 mg, 8.75 mmol) was added. The reaction was stirred at 60 °C and monitored by LC-MS until the starting material was consumed. The mixture was concentrated directly to give **intermediate 40-5** (1.85 g, crude) as a white solid. ESI-MS m/z: 252.0 [M+H]⁺.

### Step 6: Synthesis of Intermediate 40

**Intermediate 40-5** (1.85 g, 4.37 mmol) was dissolved in DCM (30 mL), and TEA (2.21 g, 21.85 mmol) and Boc₂O (1.91 g, 8.74 mmol) were added. The reaction mixture was stirred at room temperature and monitored by LC-MS until the starting material was consumed. The mixture was extracted with DCM (50 mL*3), and the combined organic phases were dried, concentrated, and purified by column chromatography (PE/EA = 10/1 to 2/1) to afford **intermediate 40** (936 mg, 61% yield) as a white solid. ESI-MS m/z: 352.1 [M+H]⁺.

### Preparation Example 27: Synthesis of tert-Butyl 9-bromo-3,4-dihydropyrazino[1,2-b]indazole-2(1H)-carboxylate (Intermediate 41)

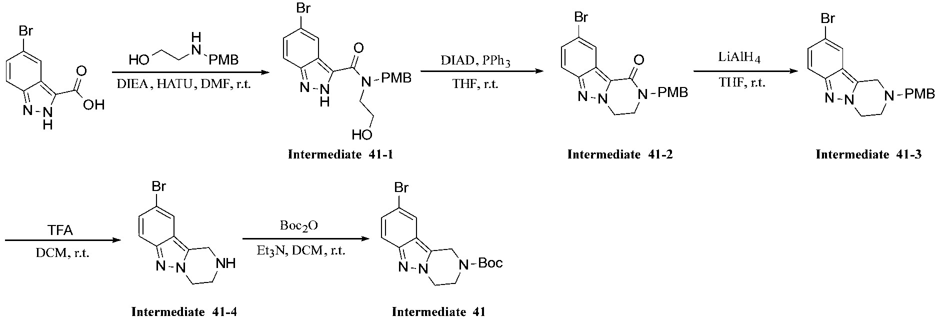

### Step 1: Synthesis of Intermediate 41-1

5-Bromoindazole-3-carboxylic acid (2.20 g, 9.20 mmol) and 2-((4-methoxybenzyl)amino)ethanol (2.00 g, 11.04 mmol) were dissolved in DMF (30 mL). DIEA (7.10 g, 54.76 mmol) and HATU (3.50 g, 9.20 mmol) were added, and the reaction mixture was stirred at room temperature. The reaction was monitored by LC-MS until the starting materials were consumed. The mixture was extracted with EA (50 mL*3), and the combined organic phases were dried, concentrated, and purified by column chromatography (PE/EA = 10/1 to 1/1) to afford **intermediate 41-1** (2.50 g, 67% yield) as a yellow solid. ESI-MS m/z: 404.1 [M+H]⁺.

### Step 2: Synthesis of Intermediate 41-2

**Intermediate 41-1** (2.45 g, 6.26 mmol) was dissolved in THF (50 mL). DIAD (1.58 g, 9.10 mmol) and PPh₃ (2.39 g, 9.10 mmol) were added, and the reaction mixture was stirred at room temperature. The reaction was monitored by LC-MS until the starting material was consumed. The mixture was extracted with EA (50 mL*3), and the combined organic phases were dried, concentrated, and purified by column chromatography (PE/EA = 5/1 to 1/1) to afford **intermediate 41-2** (2.10 g, 88% yield) as a white solid. ESI-MS m/z: 386.0 [M+H]⁺.

### Step 3: Synthesis of Intermediate 41-3

**Intermediate 41-2** (2.10 g, 5.45 mmol) was dissolved in THF (40 mL). LiAlH₄ (412 mg, 10.91 mmol) was added, and the reaction mixture was stirred at room temperature. The reaction was monitored by LC-MS until the starting material was consumed. The mixture was quenched with saturated ammonium chloride, filtered by suction, and extracted with EA (50 mL*3). The combined organic phases were dried, concentrated, and purified by column chromatography (PE/EA = 10/1 to 1/1) to afford **intermediate 41-3** (1.52 g, 75% yield) as a yellow liquid. ESI-MS m/z: 372.1 [M+H]⁺.

### Step 4: Synthesis of Intermediate 41-4

**Intermediate 41-3** (1.52 g, 4.10 mmol) was dissolved in DCM (20 mL), and TFA (5 mL) was added. The reaction mixture was stirred at room temperature and monitored by LC-MS until the starting material was consumed. The mixture was concentrated directly to afford **intermediate 41-4** (1.65 g, crude) as a yellow oil. ESI-MS m/z: 252.0 [M+H]⁺.

### Step 5: Synthesis of Intermediate 41

**Intermediate 41-4** (1.65 g, 4.10 mmol) was dissolved in DCM (30 mL). TEA (1.24 g, 12.30 mmol) and Boc₂O (1.79 g, 8.20 mmol) were added, and the reaction mixture was stirred at room temperature. The reaction was monitored by LC-MS until the starting material was consumed. The mixture was extracted with DCM (50 mL*3), and the combined organic phases were dried, concentrated, and purified by column chromatography (PE/EA = 10/1 to 2/1) to afford **intermediate 41** (750 mg, 52% yield) as a white solid. ESI-MS m/z: 352.1 [M+H]⁺.

### Preparation Example 28: Synthesis of tert-Butyl (S)-9-bromo-1,2,4a,5-tetrahydrobenzo[b]pyrazino[1,2-d][1,4]oxazine-3(4H)-carboxylate (Intermediate 42)

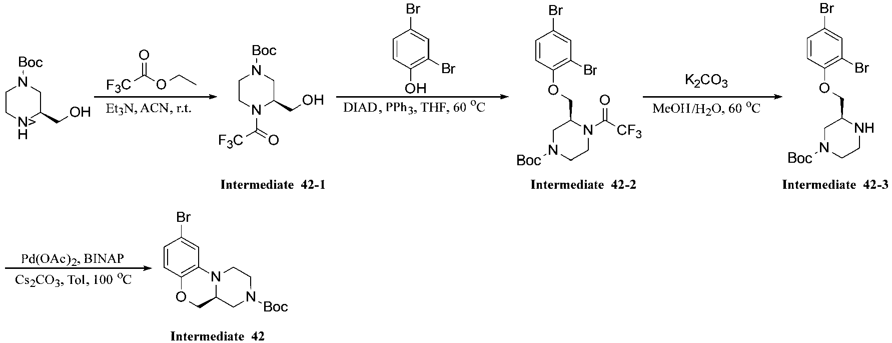

### Step 1: Synthesis of Intermediate 42-1

(*S*)-1-Boc-3-(hydroxymethyl)piperazine (5.00 g, 23.15 mmol) was dissolved in ACN (50 mL). TEA (1.54 g, 15.28 mmol) and ethyl trifluoroacetate (3.29 g, 23.15 mmol) were added, and the reaction mixture was stirred at room temperature. The reaction was monitored by LC-MS until the starting material was consumed. The mixture was extracted with EA (50 mL*3), and the combined organic phases were dried, concentrated, and purified by column chromatography (PE/EA = 10/1 to 3/1) to afford **intermediate 42-1** (6.50 g, 90% yield) as a yellow oil. ESI-MS m/z: 313.1 [M+H]⁺.

### Step 2: Synthesis of Intermediate 42-2

**Intermediate 42-1** (6.50 g, 20.83 mmol) and 2,4-dibromophenol (5.25 g, 20.83 mmol) were dissolved in THF (80 mL). DIAD (8.42 g, 41.66 mmol) and PPh₃ (10.91 g, 41.66 mmol) were added, and the reaction mixture was stirred at 60 °C. The reaction was monitored by LC-MS until the starting material was consumed. The mixture was extracted with EA (100 mL*3), and the combined organic phases were dried, concentrated, and purified by column chromatography (PE/EA = 10/1 to 3/1) to afford **intermediate 42-2** (8.64 g, 76% yield) as a yellow solid. ESI-MS m/z: 547.0 [M+H]⁺.

### Step 3: Synthesis of Intermediate 42-3

**Intermediate 42-2** (8.64 g, 15.82 mmol) was dissolved in MeOH/water (100/20 mL). K₂CO₃ (6.55 g, 47.46 mmol) was added, and the reaction mixture was stirred at 60 °C. The reaction was monitored by LC-MS until the starting material was consumed. The mixture was extracted with EA (50 mL*3), and the combined organic phases were dried, concentrated, and purified by column chromatography (PE/EA = 10/1 to 1/1) to afford **intermediate 42-3** (6.48 g, 91% yield) as a yellow solid. ESI-MS m/z: 451.0 [M+H]⁺.

### Step 4: Synthesis of Intermediate 42

**Intermediate 42-3** (6.48 g, 14.40 mmol) was dissolved in toluene (100 mL). Pd(OAc)₂ (323 mg, 1.44 mmol), BINAP (1.79 g, 2.88 mmol), and Cs₂CO₃ (9.38 g, 28.80 mmol) were added, and the reaction mixture was stirred at 100 °C. The reaction was monitored by LC-MS until the starting material was consumed. The mixture was extracted with EA (50 mL*3), and the combined organic phases were dried, concentrated, and purified by column chromatography (PE/EA = 10/1 to 1/1) to afford **intermediate 42** (1.32 g, 25% yield) as a yellow solid. ESI-MS m/z: 369.1 [M+H]⁺.

Using a synthetic approach similar to that described in **preparation example 28, Intermediate 43** can be prepared.

| Compound | Structure | Name | ESI-MS: [M+H]⁺ |
|---|---|---|---|
| **Intermediate 43** | 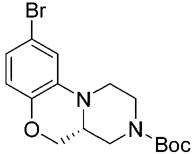 | *tert-*Butyl (*R*)-9-bromo-1,2,4*a*,5-tetrahydrobenzo[*b*]pyrazino[1,2-*d*][1,4]oxazine-3(4*H*)-carboxylate | 369.1 |

### Preparation Example 29: Synthesis of tert-Butyl 3,4,6,7,8,9-hexahydrothieno[3,2-c:4,5-c']dipyridine-2(1H)-carboxylate (Intermediate 44)

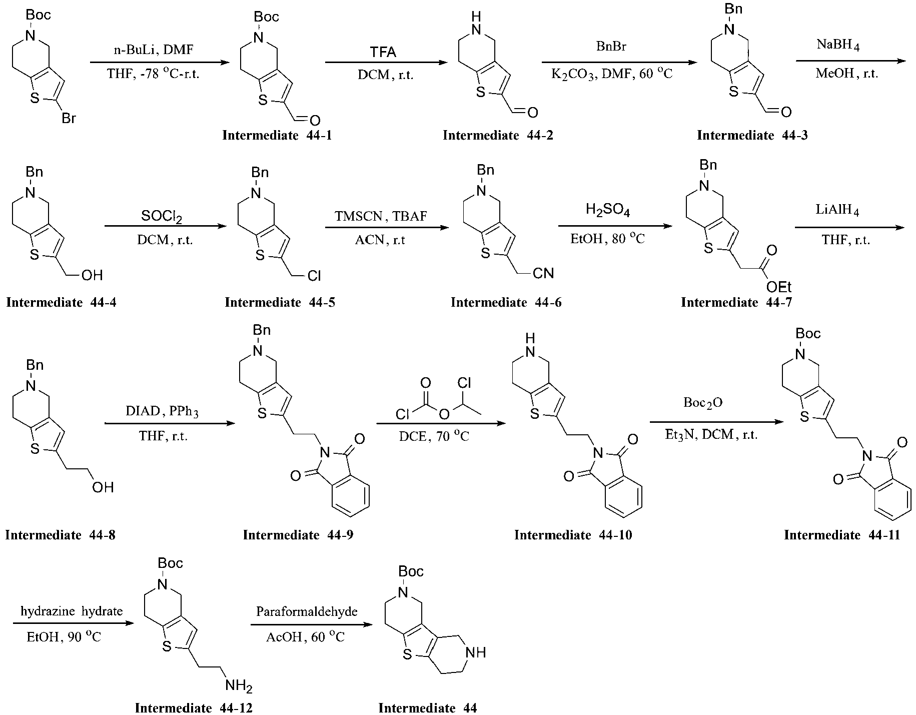

### Step 1: Synthesis of Intermediate 44-1

tert-Butyl 2-bromo-6,7-dihydrothieno[3,2-c]pyridine-5(4*H*)-carboxylate (20.00 g, 62.85 mmol) and DMF (9.20 g, 125.70 mmol) were dissolved in THF (300 mL). ⁿ⁻BuLi (2.5 M, 38 mL) was added at -78 °C, and the mixture was slowly warmed to room temperature and stirred. The reaction was monitored by LC-MS until the starting material was mostly consumed. The mixture was quenched with saturated ammonium chloride, extracted with EA (100 mL*3), and the combined organic phases were dried, concentrated, and purified by column chromatography (PE/EA = 20/1 to 5/1) to afford **intermediate 44-1** (12.00 g, 71% yield) as a yellow solid. ESI-MS m/z: 268.1 [M+H]⁺.

### Step 2: Synthesis of Intermediate 44-2

**Intermediate 44-1** (12.00 g, 44.94 mmol) was dissolved in DCM (120 mL), and TFA (20 mL) was added. The reaction mixture was stirred at room temperature and monitored by LC-MS until the starting material was consumed. The mixture was concentrated directly to afford **intermediate 44-2** (10.15 g, yield >100%) as a yellow oil. ESI-MS m/z: 168.0 [M+H]⁺.

### Step 3: Synthesis of Intermediate 44-3

**Intermediate 44-2** (10.15 g, 44.94 mmol) was dissolved in DMF (100 mL). K₂CO₃ (18.61 g, 134.82 mmol) and BnBr (7.68 g, 44.94 mmol) was added, and the reaction mixture was stirred at 60 °C. The reaction was monitored by LC-MS until the starting material was consumed. The mixture was extracted with EA (50 mL*3), and the combined organic phases were dried, concentrated, and purified by column chromatography (PE/EA = 10/1 to 2/1) to afford **intermediate 44-3** (9.82 g, 85% yield) as a yellow oil. ESI-MS m/z: 258.1 [M+H]⁺.

### Step 4: Synthesis of Intermediate 44-4

**Intermediate 44-3** (5.00 g, 19.46 mmol) was dissolved in MeOH (50 mL). NaBH₄ (1.47 g, 38.91 mmol) was added, and the reaction mixture was stirred at room temperature. The reaction was monitored by LC-MS until the starting material was consumed. The mixture was quenched with saturated ammonium chloride, filtered by suction, and extracted with EA (50 mL*3). The combined organic phases were dried and concentrated to afford **intermediate 44-4** (4.84 g, 96% yield) as a yellow liquid. ESI-MS m/z: 260.1 [M+H]⁺.

### Step 5: Synthesis of Intermediate 44-5

**Intermediate 44-4** (4.84 g, 18.69 mmol) was dissolved in DCM (50 mL). Thionyl chloride (3.33 g, 28.03 mmol) was slowly added, and the reaction mixture was stirred at room temperature. The reaction was monitored by LC-MS until the starting material was consumed. The mixture was concentrated directly to afford **intermediate 44-5** (5.13 g, 99% yield) as a white solid. ESI-MS m/z: 278.1 [M+H]⁺.

### Step 6: Synthesis of Intermediate 44-6

**Intermediate 44-5** (5.13 g, 18.52 mmol) was dissolved in ACN (80 mL). TMSCN (3.67 g, 37.04 mmol) and TBAF (9.68 g, 37.04 mmol) were added, and the reaction mixture was stirred at room temperature. The reaction was monitored by LC-MS until the starting material was consumed. The mixture was extracted with EA (30 mL*3), and the combined organic phases were dried, concentrated, and purified by column chromatography (PE/EA = 10/1 to 5/1) to afford **intermediate 44-6** (3.92 g, 79% yield) as a pale yellow solid. ESI-MS m/z: 269.1 [M+H]⁺.

### Step 7: Synthesis of Intermediate 44-7

**Intermediate 44-6** (3.92 g, 14.63 mmol) was dissolved in EtOH (50 mL). Concentrated sulfuric acid (5 mL) was slowly added, and the reaction mixture was stirred at 80 °C. The reaction was monitored by LC-MS until the starting material was consumed. The mixture was poured into ice water, neutralized to slightly basic with saturated sodium bicarbonate, and extracted with EA (50 mL*3). The combined organic phases were dried, concentrated, and purified by column chromatography (PE/EA = 10/1 to 3/1) to afford **intermediate 44-7** (4.06 g, 88% yield) as a pale yellow oil. ESI-MS m/z: 316.1 [M+H]⁺.

### Step 8: Synthesis of Intermediate 44-8

**Intermediate 44-7** (4.06 g, 12.89 mmol) was dissolved in THF (40 mL). LiAlH₄ (731 mg, 19.33 mmol) was added, and the reaction mixture was stirred at room temperature. The reaction was monitored by LC-MS until the starting material was consumed. The mixture was quenched with saturated ammonium chloride, filtered by suction, and extracted with EA (50 mL*3). The combined organic phases were dried, concentrated, and purified by column chromatography (PE/EA = 10/1 to 1/1) to afford **intermediate 44-8** (2.64 g, 75% yield) as a yellow liquid. ESI-MS m/z: 274.1 [M+H]⁺.

### Step 9: Synthesis of Intermediate 44-9

**Intermediate 44-8** (2.64 g, 9.67 mmol) and phthalimide (1.42 g, 9.67 mmol) were dissolved in THF (50 mL). DIAD (3.91 g, 19.34 mmol) and PPh₃ (5.07 g, 19.34 mmol) were added, and the reaction mixture was stirred at room temperature. The reaction was monitored by LC-MS until the starting material was consumed. The mixture was extracted with EA (50 mL*3), and the combined organic phases were dried, concentrated, and purified by column chromatography (PE/EA = 5/1 to 1/1) to afford **intermediate 44-9** (3.34 g, 86% yield) as a white solid. ESI-MS m/z: 403.1 [M+H]⁺.

### Step 10: Synthesis of Intermediate 44-10

**Intermediate 44-9** (3.92 g, 8.31 mmol) was dissolved in DCE (50 mL). 1-Chloroethyl chloroformate (4.75 g, 33.24 mmol) was added, and the reaction mixture was stirred at 70 °C. The reaction was monitored by LC-MS until the starting material was consumed. The mixture was neutralized to slightly basic with saturated sodium bicarbonate and extracted with EA (50 mL*3). The combined organic phases were dried and concentrated to afford **intermediate 44-10** (2.33 g, 90% yield) as a pale yellow oil. ESI-MS m/z: 313.1 [M+H]⁺.

### Step 11: Synthesis of Intermediate 44-11

**Intermediate 44-10** (2.33 g, 7.47 mmol) was dissolved in DCM (30 mL). TEA (2.26 g, 22.40 mmol) and Boc₂O (3.26 g, 14.94 mmol) were added, and the reaction mixture was stirred at room temperature. The reaction was monitored by LC-MS until the starting material was consumed. The mixture was extracted with DCM (50 mL*3), and the combined organic phases were dried, concentrated, and purified by column chromatography (PE/EA = 10/1 to 3/1) to afford **intermediate 44-11** (2.43 g, 79% yield) as a white solid. ESI-MS m/z: 413.1 [M+H]⁺.

Subsequently, using a synthetic method similar to that described in **preparation example 26, Intermediate 44** can be obtained, ESI-MS m/z: 295.1 [M+H]⁺.

Using a synthetic approach similar to that described in **preparation example 29, Intermediate 45** can be ontained.

| Compound | Structure | Name | ESI-MS: [M+H]⁺ |
|---|---|---|---|
| **Intermediate 45** | 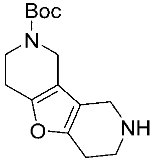 | *tert*-Butyl 3,4,6,7,8,9-hexahydrofuro[3,2-c:4,5-*c*']dipyridine-2(1*H*)-carboxylate | 279.2 |

### Preparation Example 30: Synthesis of 2-(4-methoxybenzyl)-1,2,3,4,7,8,9,10-octahydropyrido[4',3':3,4]pyrazolo[1,5-a]pyrazine (Intermediate 46)

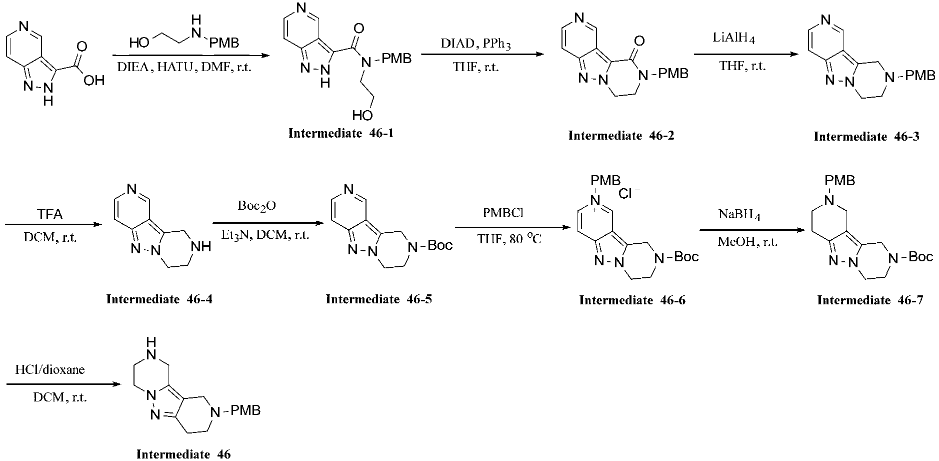

Using a synthetic approach similar to that described in **preparation example 27, Intermediate 46-5** can be ontained, ESI-MS m/z: 275.1 [M+H]⁺.

### Step 1: Synthesis of Intermediate 46-6

**Intermediate 46-5** (1.00 g, 3.65 mmol) and 4-methoxybenzyl chloride (572 mg, 3.65 mmol) were dissolved in THF (20 mL), and the mixture was stirred at 80 °C. The reaction was monitored by LC-MS until the starting material was consumed. The mixture was concentrated directly to afford **intermediate 46-6** (1.65 g, crude) as a yellow solid. ESI-MS m/z: 396.2 [M+H]⁺.

### Step 2: Synthesis of Intermediate 46-7

**Intermediate 46-6** (1.65 g, 3.65 mmol) was dissolved in MeOH (30 mL). NaBH₄ (414 mg, 10.95 mmol) was added, and the reaction mixture was stirred at room temperature. The reaction was monitored by LC-MS until the starting material was consumed. The mixture was extracted with EA (50 mL*3), and the combined organic phases were dried, concentrated, and purified by column chromatography (PE/EA = 5/1 to 0/1) to afford **intermediate 46-7** (799 mg, 55% yield) as a yellow solid. ESI-MS m/z: 399.2 [M+H]⁺.

### Step 3: Synthesis of Intermediate 46

**Intermediate 46-7** (799 mg, 2.01 mmol) was dissolved in DCM (20 mL), and HCl/dioxane (4 M, 5 mL) was added. The reaction mixture was stirred at room temperature and monitored by LC-MS until the starting material was consumed. The mixture was concentrated directly to afford **intermediate 46** (740 mg, yield >100%) as a white solid. ESI-MS m/z: 299.2 [M+H]⁺.

### Example 1: Synthesis of 2,6-dichloro-4-(1,2,3,4-tetrahydrobenzo[4,5]thieno[3,2-c]pyridin-8-yl)-N-(1,3,5-trimethyl-1H-pyrazol-4-yl)benzenesulfonamide (Compound 1)

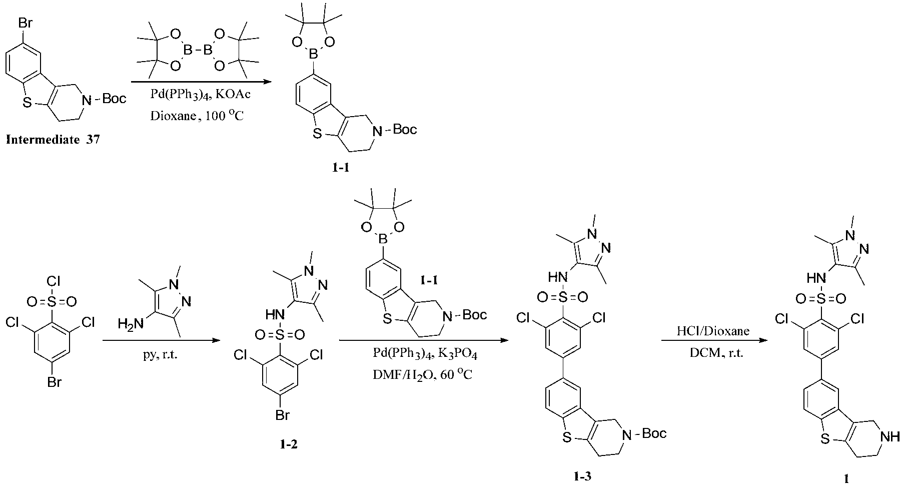

### Step 1: Synthesis of 1-1

**Intermediate 37** (1 g, 2.72 mmol) was dissolved in Dioxane (20 mL). Bis(pinacolato)diboron (690 mg, 2.72 mmol), Pd(PPh₃)₄ (312 mg, 0.27 mmol), and AcOK (530 mg, 5.44 mmol) were added. The mixture was stirred at 110 °C and monitored by LC-MS until the starting material was consumed. The mixture was extracted with EA (30 mL*3), and the combined organic phases were dried, concentrated, and purified by column chromatography (PE/EA = 10/1 to 3/1) to afford **1-1** (1.00 g, 89% yield) as a colorless oil. ESI-MS m/z: 416.2 [M+H]⁺.

### Step 2: Synthesis of 1-2

1,3,5-Trimethyl-1*H*-pyrazol-4-amine (1 g, 7.99 mmol) was dissolved in pyridine (20 mL). 4-Bromo-2,6-dichlorobenzenesulfonyl chloride (2.59 g, 7.99 mmol) was added, and the reaction mixture was stirred at room temperature. The reaction was monitored by LC-MS until the starting material was consumed. Pyridine was removed by concentration, water (30 mL) was added, and the mixture was extracted with EA (30 mL*3). The combined organic phases were dried, concentrated, and purified by column chromatography (PE/EA = 10/1 to 3/1) to afford **1-2** (3.00 g, 91% yield) as a pale yellow solid. ESI-MS m/z: 411.9 [M+H]⁺.

### Step 3: Synthesis of 1-3

**1-2** (100 mg, 0.24 mmol) and **1-1** (100 mg, 0.24 mmol) were dissolved in DMF/H₂O (5/0.5 mL). Pd(PPh₃)₄ (28 mg, 0.02 mmol) and K₃PO₄ (102 mg, 0.48 mmol) were added, and the mixture was stirred at 60 °C. The reaction was monitored by LC-MS until the starting materials were consumed. Water (30 mL) was added, and the mixture was extracted with EA (30 mL*3). The combined organic phases were dried, concentrated, and purified by column chromatography (PE/EA = 10/1 to 2/1) to afford **1-3** (90 mg, 60% yield) as a yellow solid. ESI-MS m/z: 621.1 [M+H]⁺.

### Step 4: Synthesis of 1

**1-3** (90 mg, 0.14 mmol) was dissolved in DCM (5 mL). HCl/dioxane (4 M, 1 mL) was added, and the reaction mixture was stirred at room temperature. The reaction was monitored by LC-MS until the starting material was consumed. The mixture was neutralized to slightly basic with saturated aqueous sodium bicarbonate and extracted with EA (20 mL*3). The combined organic phases were dried, concentrated, and purified by prep-HPLC to afford 1 (51 mg, 68% yield) as a yellow solid.

¹H NMR (400 MHz, DMSO-*d*₆): δ 8.24 (s, 1H), 8.12 (s, 1H), 8.07 (s, 2H), 8.03 (d, *J =* 8.4 Hz, 1H), 7.77 (dd, *J =* 8.4, 1.6 Hz, 1H), 4.09 (s, 2H), 3.57 (s, 3H), 3.12 (t, *J =* 5.4 Hz, 2H), 2.85 (d, *J =* 5.5 Hz, 2H), 1.96 (s, 3H), 1.75 (s, 3H); ESI-MS m/z: 521.1 [M+H]⁺.

### Example 2-35: Synthesis of Compound 2-35

Using **intermediate 1-34** as the starting material, a substitution reaction with the corresponding substituted benzenesulfonyl chloride is carried out, followed by coupling with **1-1.** The target **compounds 2-35** can be obtained by a synthetic method similar to that described in **Example 1.**

| Compound | Structure | ESI-MS: [M+H]⁺ | Compound | Structure | ESI-MS: [M+H]⁺ |
|---|---|---|---|---|---|
| **2** | 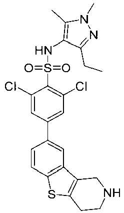 | 535.1 | **3** | 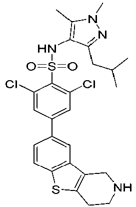 | 563.1 |
| **4** | 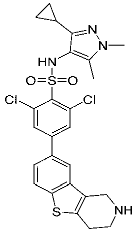 | 547.1 | **5** | 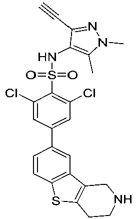 | 531.0 |
| **6** | 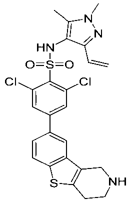 | 533.1 | **7** | 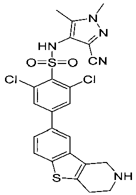 | 532.0 |
| **8** | 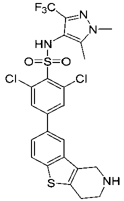 | 575.0 | **9** | 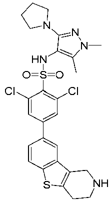 | 576.1 |
| **10** | 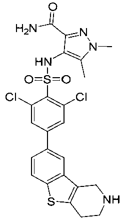 | 550.1 | **11** | 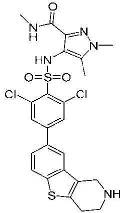 | 564.1 |
| **12** | 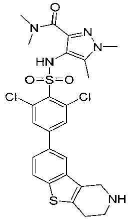 | 578.1 | **13** | 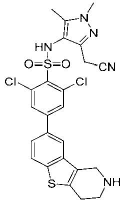 | 546.1 |
| **14** | 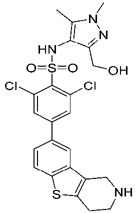 | 537.1 | **15** | 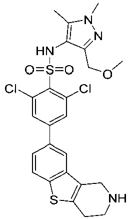 | 551.1 |
| **16** | 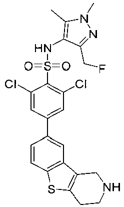 | 539.1 | **17** | 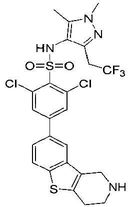 | 589.0 |
| **18** | 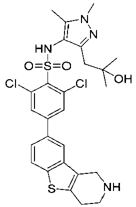 | 579.1 | **19** | 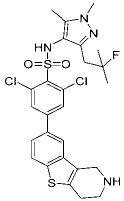 | 581.1 |
| **20** | 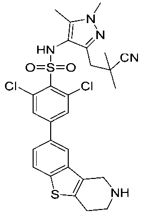 | 588.1 | **21** | 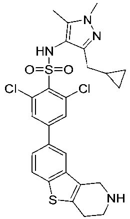 | 561.1 |
| **22** | 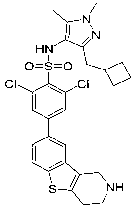 | 575.1 | **23** | 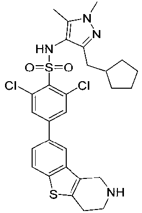 | 589.1 |
| **24** | 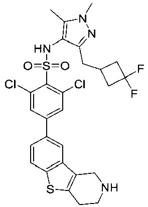 | 611.1 | **25** | 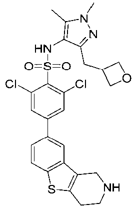 | 577.1 |
| **26** | 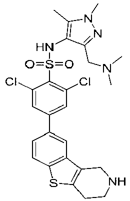 | 564.1 | **27** | 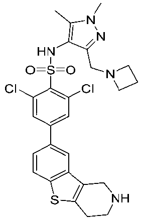 | 576.1 |
| **28** | 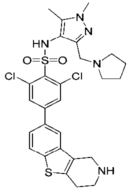 | 590.1 | **29** | 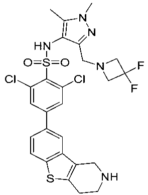 | 612.1 |
| **30** | 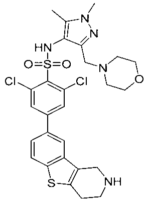 | 606.1 | **31** | 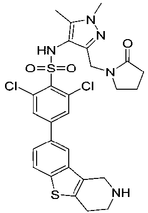 | 604.1 |
| **32** | 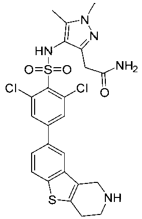 | 564.1 | **33** | 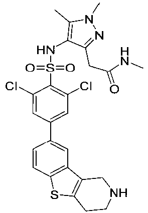 | 578.1 |
| **34** | 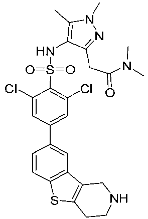 | 592.1 | **35** | 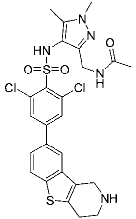 | 578..1 |

### Example 36-44: Synthesis of Compounds 36-44

Using **intermediate 2** as the starting material, a substitution reaction with the corresponding substituted benzenesulfonyl chloride is carried out, followed by coupling with **1-1.** The target **compounds 36-44** can be obtained by a synthetic method similar to that described in **Example 1.**

| Compound | Structure | ESI-MS: [M+H]⁺ | Compound | Structure | ESI-MS: [M+H]⁺ |
|---|---|---|---|---|---|
| **36** | 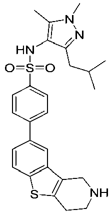 | 495.2 | **37** | 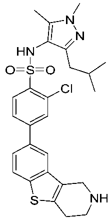 | 529.1 |
| **38** | 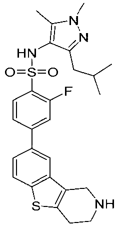 | 513.2 | **39** | 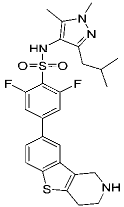 | 531.2 |
| **40** | 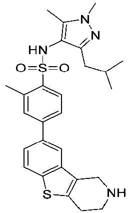 | 509.2 | **41** | 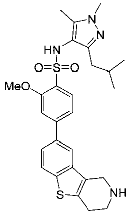 | 525.2 |
| **42** | 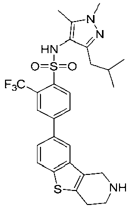 | 563.2 | **43** | 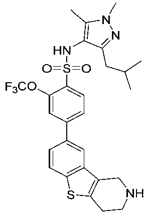 | 579.2 |
| **44** | 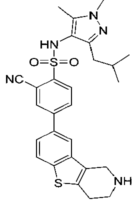 | 520.2 | | | |

### Example 45-62: Synthesis of Compounds 45-62

Using 1,3,5-trimethyl-1*H*-pyrazol-4-amine, **Intermediate 1** and **intermediate 2** as starting materials, a substitution reaction with the corresponding substituted benzenesulfonyl chloride is carried out, followed by coupling with the corresponding boronic acid or boronic ester of **intermediates 38-43.** The target **compounds 45-62** can be obtained by a synthetic method similar to that described in **Example 1.**

| Compound | Structure | ESI-MS: [M+H]⁺ | Compound | Structure | ESI-MS: [M+H]⁺ |
|---|---|---|---|---|---|
| **45** | 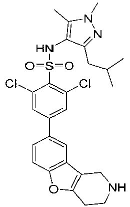 | 547.1 | **46** | 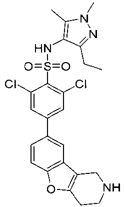 | 519.1 |
| **47** | 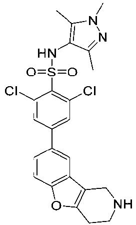 | 505.1 | **48** | 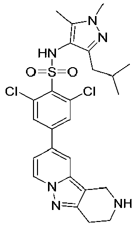 | 547.1 |
| **49** | 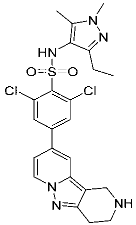 | 519.1 | **50** | 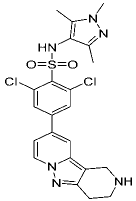 | 505.1 |
| **51** | 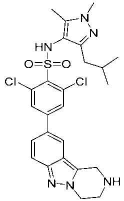 | 547.1 | **52** | 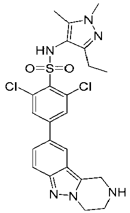 | 519.1 |
| **53** | 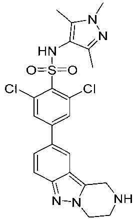 | 505.1 | **54** | 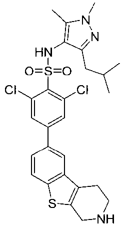 | 563.1 |
| **55** | 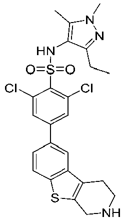 | 535.1 | **56** | 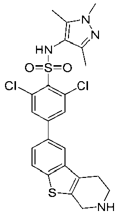 | 521.1 |
| **57** | 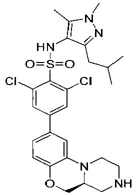 | 564.2 | **58** | 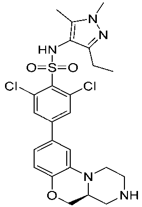 | 536.1 |
| **59** | 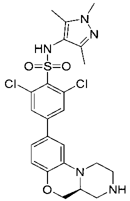 | 522.1 | **60** | 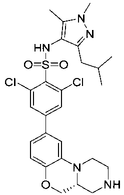 | 564.2 |
| **61** | 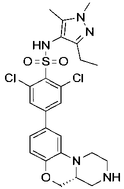 | 536.1 | **62** | 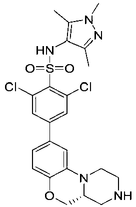 | 522.1 |

### Example 63: Synthesis of 2,6-dichloro-4-(3,4,6,7,8,9-hexahydrothieno[3,2-c:4,5-c']dipyridine-2(1H)-yl)-N-(3-isobutyl-1,5-dimethyl-1H-pyrazol-4-yl)benzenesulfonamide (Compound 63)

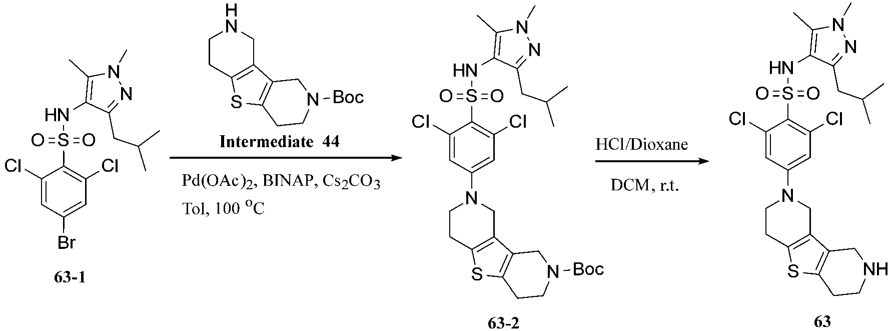

Using a synthetic approach similar to that described in **Example 1, 63-1** can be prepared, ESI-MS m/z: 454.0 [M+H]⁺.

### Step 1: Synthesis of 63-2

**63-1** (200 mg, 0.44 mmol) and Intermediate 44 (130 mg, 0.44 mmol) were dissolved in toluene (20 mL). Pd(OAc)₂ (10 mg, 0.04 mmol), BINAP (56 mg, 0.09 mmol), and Cs₂CO₃ (287 mg, 0.88 mmol) were added. The mixture was stirred at 100 °C and monitored by LC-MS until the starting material was consumed. Water (30 mL) was added, and the mixture was extracted with EA (30 mL*3). The combined organic phases were dried, concentrated, and purified by column chromatography (PE/EA = 10/1 to 1/1) to afford **63-2** (91 mg, 31% yield) as a yellow solid. ESI-MS m/z: 668.2 [M+H]⁺.

### Step 2: Synthesis of 63

**63-2** (91 mg, 0.14 mmol) was dissolved in DCM (5 mL). HCl/dioxane (4 M, 1 mL) was added, and the reaction mixture was stirred at room temperature. The reaction was monitored by LC-MS until the starting material was consumed. The mixture was neutralized to slightly basic with saturated aqueous sodium bicarbonate and extracted with EA (20 mL*3). The combined organic phases were dried, concentrated, and purified by prep-HPLC to afford 63 (47 mg, 61% yield) as a yellow solid.

¹H NMR (400 MHz, DMSO-*d*₆): δ 7.35 (s, 1H), 7.16 (s, 2H), 4.30-4.25 (m, 2H), 3.84-3.80 (m, 2H), 3.75-3.70 (m, 2H), 3.60-3.57 (m, 3H), 3.08-3.04 (m, 2H), 2.85-2.79 (m, 2H), 2.74-2.68 (m, 2H), 1.99-1.95 (m, 5H), 1.79-1.73 (m, 1H), 0.71 (d, *J =* 6.7 Hz, 6H); ESI-MS m/z: 568.1 [M+H]⁺.

### Example 64-71: Synthesis of Compounds 64-71

Using 1,3,5-trimethyl-1*H*-pyrazol-4-amine, **Intermediate 1** and **intermediate 2** as starting materials, a substitution reaction with the corresponding substituted benzenesulfonyl chloride is carried out, followed by coupling with **intermediates 44-46.** The target **compounds 64-71** can be obtained by a synthetic method similar to that described in **Example 63.**

| Compound | Structure | ESI-MS: [M+H]⁺ | Compound | Structure | ESI-MS: [M+H]⁺ |
|---|---|---|---|---|---|
| **64** | 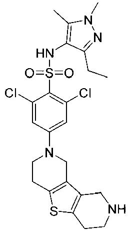 | 540.1 | **65** | 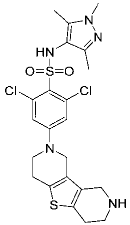 | 526.1 |
| **66** | 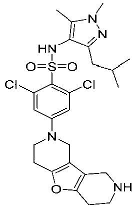 | 552.2 | **67** | 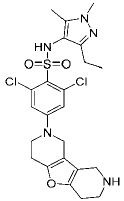 | 524.1 |
| **68** | 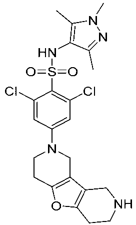 | 510.1 | **69** | 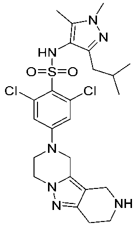 | 552.2 |
| **70** | 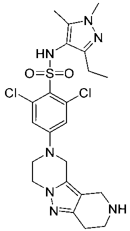 | 524.1 | **71** | 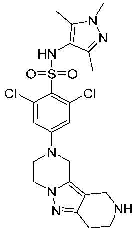 | 510.1 |

### Example 72: Synthesis of 2,6-dichloro-N-(3-isobutyl-1,5-dimethyl-1H-pyrazol-4-yl)-4-(2-methyl-1,2,3,4-tetrahydrobenzo[4,5]thieno[3,2-c]pyridin-8-yl)benzenesulfonamide (Compound 72)

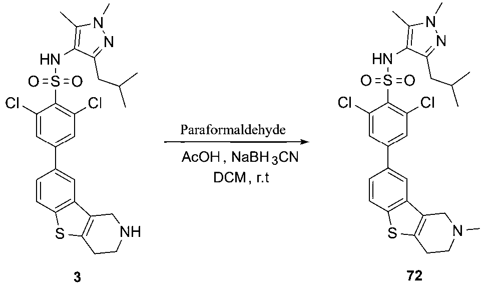

### Step 1: Synthesis of 72

**3** (50 mg, 0.09 mmol) was dissolved in DCM (5 mL). Paraformaldehyde (13 mg, 0.44 mmol), AcOH (5 mg, 303 µmol), and NaBH₃CN (28 mg, 0.44 mmol) were added. The reaction mixture was stirred at room temperature and monitored by LC-MS until the starting material was consumed. The mixture was extracted with DCM (10 mL*3), and the combined organic phases were dried, concentrated, and purified by prep-HPLC to afford **72** (30 mg, 58% yield) as a yellow solid.

¹H NMR (400 MHz, DMSO-*d*₆): δ 8.25 (s, 1H), 8.11-8.07 (m, 3H), 8.04 (d, *J =* 8.5 Hz, 1H), 7.75 (dd, *J =* 8.5, 1.7 Hz, 1H), 4.10 (s, 2H), 3.61 (s, 3H), 3.13 (t*, J =* 5.8 Hz, 2H), 2.89-2.83 (m, 2H), 2.24 (s, 3H), 2.00 (s, 3H), 1.95 (d, *J =* 7.2 Hz, 2H), 1.77-1.68 (m, 1H), 0.69 (d, *J =* 6.6 Hz, 6H); ESI-MS m/z: 577.1 [M+H]⁺.

### Example 73-79: Synthesis of Compounds 73-79

Using a synthetic approach similar to that described in **Example 72,** the target **compounds 73-79** can be prepared.

| Compound | Structure | ESI-MS: [M+H]⁺ | Compound | Structure | ESI-MS: [M+H]⁺ |
|---|---|---|---|---|---|
| **73** | 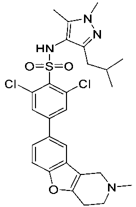 | 561.1 | **74** | 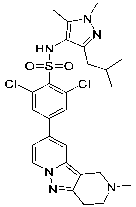 | 561.2 |
| **75** | 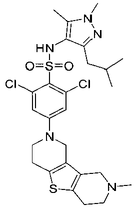 | 582.2 | **76** | 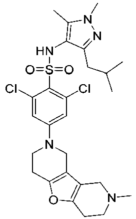 | 566.2 |
| **77** | 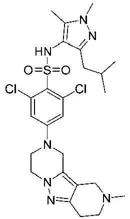 | 566.2 | **78** | 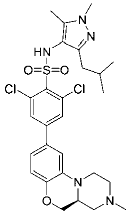 | 578.2 |
| **79** | 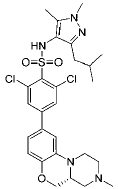 | 578.2 | | 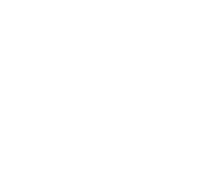 | |

### Example 80-137: Synthesis of Compounds B1-B34, B37-B45, B48-B56, B59-B64

Using 1,3,5-trimethyl-1*H*-pyrazol-4-amine, **Intermediate 1,** intermediate 2, **intermediate 12, intermediate 16, intermediate 20, intermediate 35,** and **intermediate 36** as starting materials, a substitution reaction with the corresponding substituted benzenesulfonyl chloride is carried out, followed by coupling with the corresponding boronic acid or boronic ester of **intermediates 37-39.** The target **compounds B1-B34, B37-B45, B48-B56,** and **B59-B64** can be obtained by a synthetic method similar to that described in **Example 1.**

| Compound | Structure | ESI-MS: [M+H]⁺ | Compound | Structure | ESI-MS: [M+H]⁺ |
|---|---|---|---|---|---|
| **B1** | 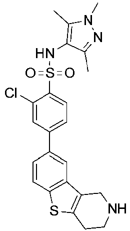 | 487.1 | **B2** | 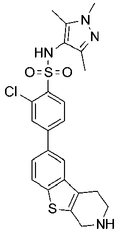 | 487.1 |
| **B3** | 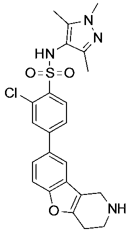 | 471.1 | **B4** | 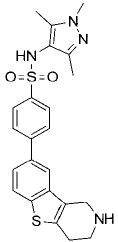 | 453.1 |
| **B5** | 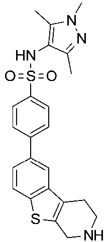 | 453.1 | **B6** | 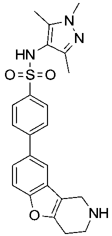 | 437.2 |
| **B7** | 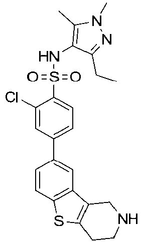 | 501.1 | **B8** | 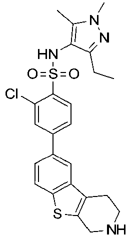 | 501.1 |
| **B9** | 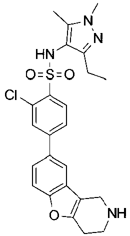 | 485.1 | **B10** | 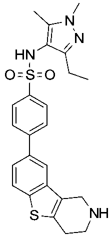 | 467.2 |
| **B11** | 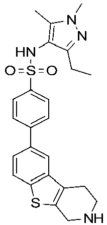 | 467.2 | **B12** | 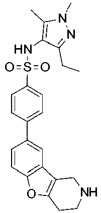 | 451.2 |
| **B13** | 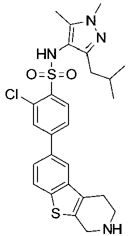 | 529.2 | **B14** | 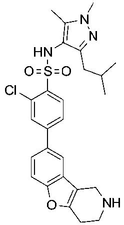 | 513.2 |
| **B15** | 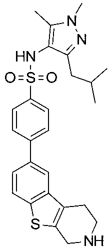 | 495.2 | **B16** | 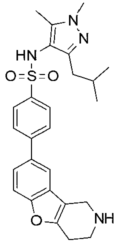 | 479.2 |
| **B17** | 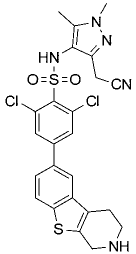 | 546.1 | **B18** | 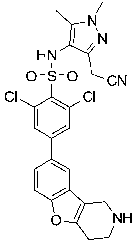 | 530.1 |
| **B19** | 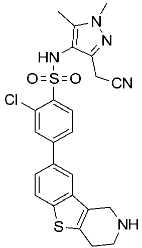 | 512.1 | **B20** | 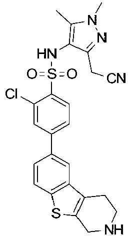 | 512.1 |
| **B21** | 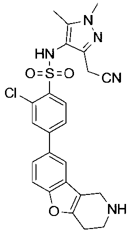 | 496.1 | **B22** | 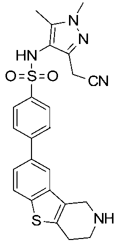 | 478.1 |
| **B23** | 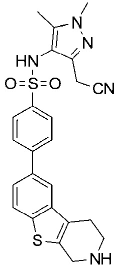 | 478.1 | **B24** | 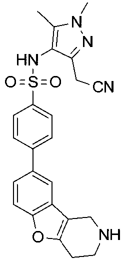 | 462.2 |
| **B25** | 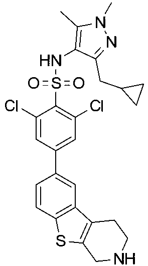 | 561.1 | **B26** | 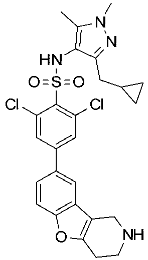 | 545.1 |
| **B27** | 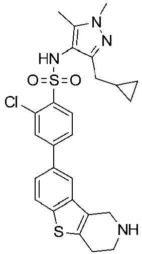 | 527.1 | **B28** | 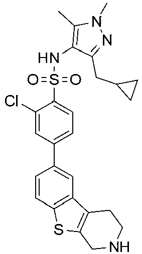 | 527.1 |
| **B29** | 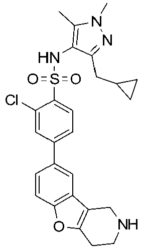 | 511.2 | **B30** | 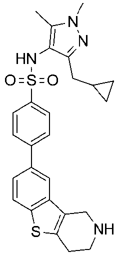 | 493.2 |
| **B31** | 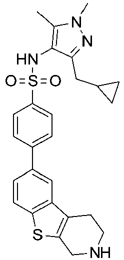 | 493.2 | **B32** | 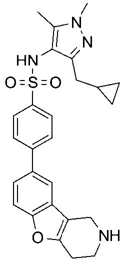 | 477.2 |
| **B33** | 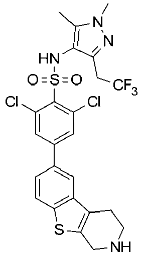 | 589.1 | **B34** | 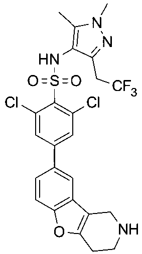 | 573.1 |
| **B37** | 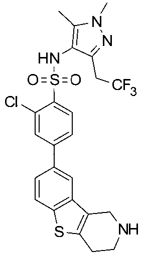 | 555.1 | **B38** | 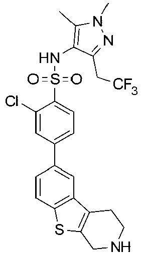 | 555.1 |
| **B39** | 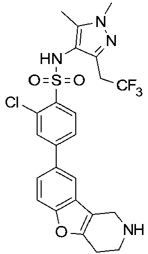 | 539.1 | **B40** | 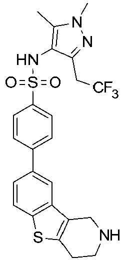 | 521.1 |
| **B41** | 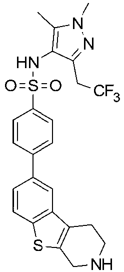 | 521.1 | **B42** | 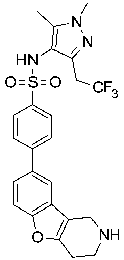 | 505.2 |
| **B43** | 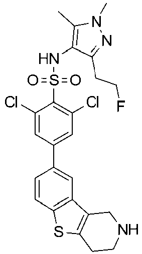 | 553.1 | **B44** | 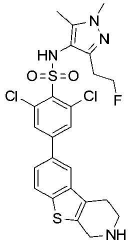 | 553.1 |
| **B45** | 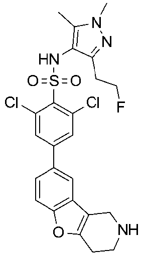 | 537.1 | **B48** | 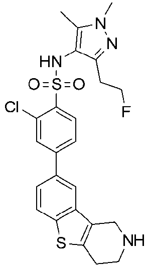 | 519.1 |
| **B49** | 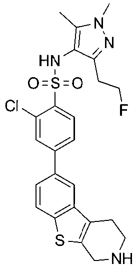 | 519.1 | **B50** | 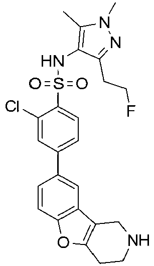 | 503.1 |
| **B51** | 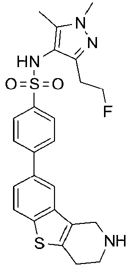 | 485.2 | **B52** | 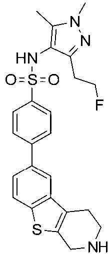 | 485.2 |
| **B53** | 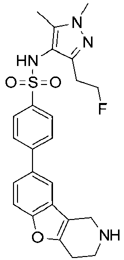 | 469.2 | **B54** | 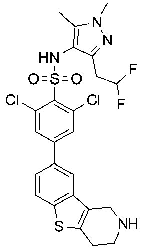 | 571.1 |
| **B55** | 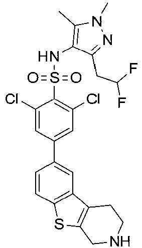 | 571.1 | **B56** | 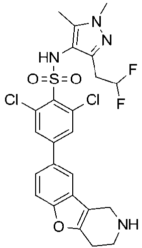 | 555.1 |
| **B59** | 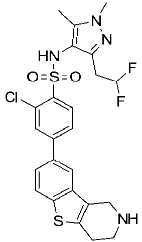 | 537.1 | **B60** | 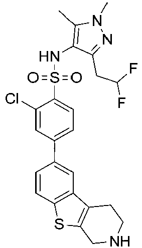 | 537.1 |
| **B61** | 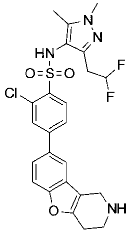 | 521.1 | **B62** | 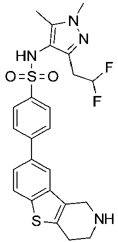 | 503.1 |
| **B63** | 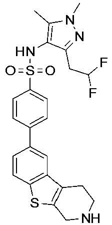 | 503.1 | **B64** | 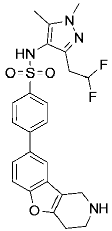 | 487.2 |

### Example 138-143: Synthesis of Compounds B35-B36, B46-B47, B57-B58

Using **Intermediate 16, intermediate 35,** and **intermediate 36** as starting materials, a substitution reaction with the corresponding substituted benzenesulfonyl chloride is carried out, followed by coupling with **intermediates 44-45.** The target **compounds B35-B36, B46-B47,** and **B57-B58** can be obtained by a synthetic method similar to that described in **Example 63.**

| Compound | Structure | ESI-MS: [M+H]⁺ | Compound | Structure | ESI-MS: [M+H]⁺ |
|---|---|---|---|---|---|
| **B35** | 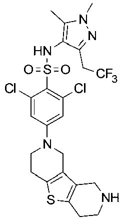 | 594.1 | **B36** | 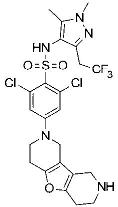 | 578.1 |
| **B46** | 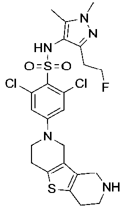 | 558.1 | **B47** | 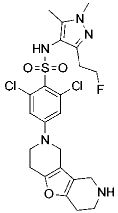 | 542.1 |
| **B57** | 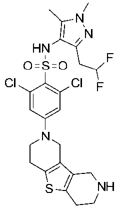 | 576.1 | **B58** | 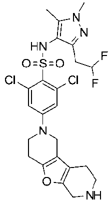 | 560.1 |

### Example 144: Screening of Compound Inhibitory Activity on NMT1 Enzyme Catalysis

This study evaluated the ability of the compounds of the invention to inhibit NMT1 catalytic activity *in vitro.* The experiment used a FI (Fluorescence Intensity) analysis method, characterizing NMT1 inhibitors under conditions of 1.25 µM myristoyl-CoA (Lithium salt, Sigma, M4414). In this experiment, a synthesized peptide with the sequence H-Gly-Ser-Asn-Lys-Ser-Lys-Pro-Lys-NH₂, HSPP60SRC (Nanjing Peptide Industry, NJP23953), was used as the reaction substrate. The final concentration of the NMT1 enzyme was 6 nM, the final concentration of CPM (Reactive thiol fluorescence probe, Invitrogen, D346) was 1 µM, the final concentration of HSPP60 SRC was 20 µM, and the final concentration of DMSO was 0.5%.

Compounds were dissolved in DMSO to obtain 10 mM stock solutions. Dose-response solutions were prepared with a top endpoint compound concentration of 3 mM, followed by three-fold serial dilutions in DMSO, totaling ten data points. Using an ECHO acoustic liquid handler (BECKMAN, ECHO 655 system), 0.05 µL of the diluted compound solutions was transferred to a 384-well assay plate (Corning, CLS4514). Then, 2.5 µL of the NMT1 enzyme working solution was added to the 384-well assay plate, followed by incubation at 25 °C for 10 minutes. Subsequently, 5 µL of the CPM and myristoyl-CoA working solution was added, the plate was centrifuged at 1000 rpm for 1 minute, and then 2.5 µL of the HSPP60 SRC working solution was added to initiate the reaction. The reaction was incubated at 25 °C for 45 minutes. The FI signal (em: 320 nm; ex: 405 nm) was detected using an HTS high-throughput screening multifunctional microplate reader (BMG, PHERA star FSX).

The inhibition rate of the compounds on NMT1 enzyme catalytic activity was calculated using the following formula: Inhibition Percentage (%) = 100 * (Mean of DMSO group - Mean of Compound group) / (Mean of DMSO group - Mean of Blank control group). The IC₅₀ curve of the compounds was fitted using the software XLfit 5.5.0 according to the nonlinear regression equation. Table 1 provides the inhibitory activity of the compounds of the invention on NMT1 enzyme catalysis.

### Example 145: Screening of Compound Anti-proliferative Activity against MV-4-11 Cells

This study utilized a cell proliferation assay to analyze the cytotoxicity of MV-4-11 human monocytic leukemia cells after three days of exposure to NMT1 inhibitors. The MV-4-11 cell line was purchased from Nanjing Cobuilder Biotechnology Co., Ltd., and cultured in IMDM medium (Viva cell) in an incubator (Thermo) at 37 °C with 5% CO₂. In this experiment, NMT1 inhibitors were dissolved in DMSO to obtain 600 µM stock solutions. Dose-response solutions were prepared with a top endpoint compound concentration of 3 µM, followed by three-fold serial dilutions in DMSO, totaling eight data points. The final concentration of DMSO was 0.5%.

MV-4-11 cells were seeded into a white 96-well plate, 80 µL of cell suspension per well, containing 6000 MV-4-11 cells. The cell plate was placed in the CO₂ incubator for overnight culture. Then, 20 µL of NMT1 inhibitor solutions at different concentrations were added to the wells. The 96-well plate was placed back into the incubator for three days. Another cell plate was prepared, and the signal was read on the day of drug addition to serve as the maximum value (Max value in the equation below) for data analysis. To this cell plate, 25 µL of cell viability chemiluminescence detection reagent was added per well, followed by incubation at room temperature for 10 minutes to stabilize the luminescence signal. A multi-label analyzer was used for reading.

The raw data were converted into inhibition rates using the equation (Sample - Min) / (Max - Min) * 100%. The IC₅₀ values were then determined by fitting a four-parameter curve (using the "log(inhibitor) vs. response - Variable slope" model in GraphPad Prism). Min: wells treated with 0.5% DMSO; Max: Day 0 wells. Table 1 provides the inhibitory activity of the compounds of the invention on MV-4-11 cell proliferation.

**Table 1: Compound Inhibitory Activity against NMT1 Enzyme (IC₅₀) and Anti-proliferative Activity against MV-4-11 Cell line (IC₅₀)**

| Compound | NMT1 (IC₅₀, nM) | MV-4-11 (IC₅₀, nM) | Compound | NMT1 (IC₅₀, nM) | MV-4-11 (IC₅₀, nM) |
|---|---|---|---|---|---|
| **1** | 3.44 | 11.12 | **B18** | 4.85 | 37.02 |
| **2** | 3.09 | 5.43 | **B19** | 3.07 | 21.73 |
| **3** | 3.45 | 15.03 | **B20** | 3.56 | 34.89 |
| **4** | 3.64 | 25.76 | **B21** | - | 98.25 |
| **13** | 3.21 | 19.30 | **B25** | 2.62 | 10.36 |
| **14** | 7.89 | 145.02 | **B26** | 2.87 | 11.45 |
| **15** | 4.73 | 78.61 | **B27** | 2.08 | 9.68 |
| **16** | 4.67 | 58.45 | **B29** | 3.60 | 17.45 |
| **17** | 5.62 | 62.47 | **B30** | 2.59 | 12.31 |
| **21** | 3.58 | 26.92 | **B34** | 5.03 | 61.79 |
| **45** | 5.38 | 43.69 | **B35** | 1.97 | 7.48 |
| **46** | 3.96 | 18.75 | **B36** | 2.75 | 26.17 |
| **47** | 4.17 | 23.10 | **B37** | 2.21 | 16.58 |
| **48** | 4.20 | 36.67 | **B39** | 4.76 | 40.15 |
| **49** | 3.64 | 16.83 | **B40** | 2.70 | 11.29 |
| **50** | 3.79 | 21.86 | **B43** | 1.79 | 3.42 |
| **54** | 4.73 | 37.95 | **B44** | 2.08 | 9.27 |
| **55** | 3.97 | 26.19 | **B45** | 1.94 | 9.51 |
| **56** | 4.21 | 29.72 | **B46** | 2.03 | 3.61 |
| **57** | 8.76 | 143.58 | **B48** | 1.89 | 3.26 |
| **60** | 7.95 | 152.39 | **B49** | 2.37 | 19.48 |
| **63** | 3.35 | 16.73 | **B50** | 3.95 | 17.89 |
| **64** | 2.97 | 7.36 | **B51** | 2.51 | 6.05 |
| **65** | 3.51 | 21.08 | **B52** | 3.88 | 17.33 |
| **69** | 3.75 | 21.94 | **B53** | 4.02 | 27.16 |
| **70** | 3.46 | 15.23 | **B54** | 1.79 | 5.45 |
| **71** | 3.79 | 25.88 | **B55** | 3.72 | 21.15 |
| **B1** | 2.67 | 14.36 | **B56** | 3.49 | 19.64 |
| **B3** | 3.10 | 21.94 | **B57** | 2.95 | 11.84 |
| **B4** | 2.94 | 15.75 | **B58** | 2.08 | 8.97 |
| **B6** | 3.76 | 23.32 | **B59** | 1.93 | 5.30 |
| **B7** | 2.93 | 9.82 | **B60** | - | 62.77 |
| **B9** | 2.52 | 13.46 | **B61** | 5.21 | 27.01 |
| **B10** | 2.39 | 10.07 | **B62** | 2.47 | 7.48 |
| **B12** | 2.98 | 17.83 | **B63** | 3.67 | 20.36 |
| **B14** | 3.06 | 25.88 | **B64** | 4.08 | 30.17 |
| **B16** | 3.84 | 32.41 | **PCLX-001** | 6.72/6.46* | 75.35/74.31* |

| | | | | | |
|---|---|---|---|---|---|
| "-" means not test; "*" represents the experimental results of two independent tests. | | | | | |

The reference compound PCLX-001 is compound DDD86481 disclosed in patent WO2010026365A1.

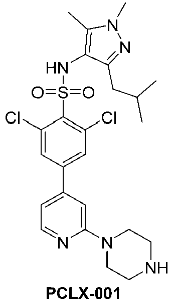

It can be seen from the data in the above table that the compounds of the present invention exhibit stronger inhibitory activity against the catalytic activity of NMT1 enzyme than the control compound PCLX-001, and also showed more potent anti-proliferative activity against MV-4-11 cell line.

### Example 146: Evaluation of In Vivo Plasma Concentrations in Mice Following Oral Administration

This study evaluated the plasma concentrations of the compounds of the present invention in mice. ICR mice were procured from JOINN Laboratories (Suzhou) Co., Ltd. At the initiation of dosing, the animals were 6-8 weeks old and weighed 18-25 g. Mice were housed in transparent plastic cages (400 mm*240 mm*200 mm). The animal facility maintained a 12-hour light/12-hour dark cycle (lights on 07:00-19:00) using fluorescent lighting. The dark period may be intermittently interrupted as required for study-related activities. Ambient temperature and relative humidity were controlled within 20-26 °C and 40-70%, respectively. Animals were fed certified rodent chow and provided with drinking water filtered and sterilized through an ultrapure water system. All animals had ad libitum access to water throughout the study. Animals were fasted for at least 12 hours prior to dosing, and food was restored 4 hours post-dosing.

Prior to dosing, body weights were recorded, and healthy animals with similar weights were selected and randomly assigned to groups (n = 3 per group) based on body weight. The compounds were formulated in a 0.5% aqueous carboxymethyl cellulose (CMC) solution and administered orally at a single dose of 10 mg/kg. Blood samples (≥0.2 mL) were collected via venipuncture at 1 h and 4 h post-dose into EDTA-K₂ anticoagulant tubes. Tubes were gently inverted to mix, immediately placed in an ice-water bath, and rapidly centrifuged at 4,000 rpm for 10 min at 4 °C to separate plasma. Plasma was stored at -70 °C until analysis.

Calibration curve and QC plasma samples: Working solutions for the calibration curve and QC samples were prepared using 80% methanol in water (Thermo Fisher) as the diluent. To prepare plasma calibration standards and QC samples, 5 µL of each working solution was added to 45 µL of blank plasma and mixed. To the prepared calibration and QC samples, 300 µL of internal standard solution (tolbutamide at 8 µg/mL in acetonitrile, Thermo Fisher) was added. After vortex mixing for 1 min, samples were centrifuged at 15,400 g for 10 min at 4 °C (Eppendorf 5810 centrifuge). The supernatant was collected and analyzed by liquid chromatography-tandem mass spectrometry (LC-MS/MS; AB Sciex API 5000).

Plasma sample pretreatment: After thawing at room temperature, 50 µL of each plasma sample was mixed with 300 µL of the internal standard solution (tolbutamide at 8 µg/mL in acetonitrile, Thermo Fisher). Following vortex mixing for 1 min, mixtures were centrifuged at 15,400 g for 10 min at 4 °C (Eppendorf 5810 centrifuge). The supernatant was collected and analyzed by LC-MS/MS (AB Sciex API 5000).

Raw chromatograms, concentrations, and accuracy data were generated using Analyst software version 1.6.3. Means and standard errors of the mean (SEM) were calculated using Microsoft Excel 2007. The concentration-time profiles of the compounds in plasma samples from each group are presented in Table 2.

**Table 2. Plasma concentrations of the compounds of the present invention at different time points (Mean ± SEM)**

| Compound ID | 1 h Concentration (ng/mL) | 4 h Concentration (ng/mL) | 8 h Concentration (ng/mL) |
|---|---|---|---|
| **1** | 1820 ± 493 | 1353 ±15 | 723 ± 363 |
| **2** | 2887 ± 858 | 1957 ± 190 | 915 ± 97 |
| **3** | 2407 ± 21 | 1980 ± 417 | 1141 ± 551 |
| **13** | 1893 ± 196 | 623 ± 109 | 334 ± 83 |
| **17** | 5100 ± 2695 | 4080 ± 1974 | 3000 ± 1015 |
| **21** | 1900 ± 199 | 1114 ± 196 | 679 ± 220 |
| **45** | 2973 ± 855 | 3257 ± 876 | 1957 ± 467 |
| **54** | 3317 ± 1198 | 2307 ± 211 | 981 ± 343 |
| **B18** | 2490 ± 1205 | 1673 ± 467 | 1035 ± 157 |
| **B26** | 4630 ± 2015 | 3377 ± 575 | 2150 ± 206 |
| **B35** | 2267 ± 1084 | 1270 ± 384 | 684 ± 239 |
| **B39** | 9763 ± 410 | 8533 ± 1166 | 6727 ± 1446 |
| **B43** | 3217 ± 650 | 1713 ± 224 | 1573 ± 104 |
| **B45** | 5040 ± 1822 | 3803 ± 621 | 3727 ± 1277 |
| **B48** | 2963 ± 340 | 1400 ± 240 | 447 ± 71 |
| **B52** | 3633 ± 990 | 2183 ± 185 | 504 ± 71 |
| **B53** | 2480 ± 626 | 1024 ± 361 | 399 ± 264 |
| **B59** | 1870 ± 389 | 846 ± 698 | 149 ± 114 |
| **B64** | 1663 ± 366 | 795 ± 127 | 260 ± 63 |

As shown in the table above, following oral administration to mice, the compounds of the present invention exhibited favorable oral absorption properties and achieved high plasma concentrations.

### Example 147: Evaluation of In Vivo Anti-Tumor Efficacy of the Compounds of the Present Invention

To evaluate the effect of the compounds of the present invention on tumor growth, an MV-4-11 human myelomonocytic leukemia xenograft mouse model was established. Female BALB/c nude mice were procured from Shanghai Jihui Laboratory Animal Care Co., Ltd. Upon arrival, the mice were 6-7 weeks old and weighed 18-22 g. They were housed in specific pathogen-free (SPF) individually ventilated cages (IVC) at a density of 2-6 mice per cage, with ad libitum access to standard diet and water. The housing environment was maintained at 20 - 26 °C (68 - 79 °F) with a relative humidity of 40-70%, under a 12-hour light/dark cycle (lights on from 05:00 to 17:00). The study commenced after a one-week acclimatization period.

Human myelomonocytic leukemia MV-4-11 cells (ATCC, CRL-9591) were maintained in suspension culture using IMDM medium (Gibco, 12440-053) supplemented with 10% heat-inactivated fetal bovine serum (FBS; Gibco, 10099-141C) at 37 °C in a 5% CO₂ atmosphere. Cells were passaged 2-3 times per week. Upon reaching the exponential growth phase, cells were harvested, counted, and inoculated subcutaneously into the right dorsal flank of female nude mice. Each mouse received a 200 µL suspension containing 8 × 10⁶ MV-4-11 cells (100 µL cell suspension + 100 µL Matrigel basement membrane matrix, Corning, 354234). Thirteen days post-inoculation, when subcutaneous tumor volumes reached 100 - 300 mm³ , tumor-bearing mice with appropriate morphology and uniform size were selected. Using stratified randomization, mice were assigned to experimental groups (n = 5 per group). Oral administration (PO, once daily [QD]) was initiated on the day of grouping, with all compounds of the present invention dosed at 30 mg/kg. During the dosing period, tumor diameters were measured twice weekly using digital calipers, and body weights were recorded. In the posttreatment observation period, tumor diameters and body weights were measured once weekly. Tumor volume (V) was calculated using the formula: V = 0.5 *a * b² , where a represents the long diameter and b represents the short diameter of the tumor.

The anti-tumor efficacy of the compounds was evaluated using the tumor growth inhibition percentage (TGI%), calculated by the formula: TGI(%) = [1 - (TVi - TV0) / (TVVi - TVV0)] *100%, where TV0 and TVV0 represent the mean tumor volumes on day 0 for the treatment and control groups, respectively, and TVi and TVVi represent the mean tumor volumes on day i for the treatment and control groups, respectively. Data were plotted using GraphPad Prism software. Tumor volume and body weight data are expressed as mean ± SEM and plotted as a function of time.

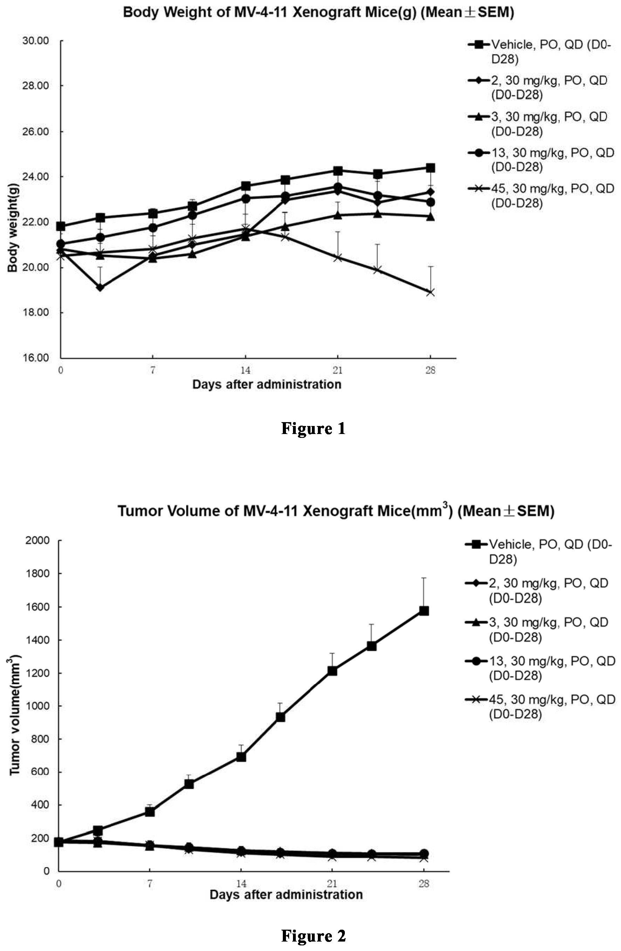

Tumor volume results are shown in Figure 2. Daily oral administration of compounds 2, 3, 13, and 45 significantly inhibited the growth of MV-4-11 tumors and induced tumor regression.

Body weight results are shown in Figure 1. Compound 2 caused a slight decrease in body weight during the early phase of dosing, which subsequently returned to normal. Compound 45 showed a slight weight loss in the later phase, but it did not exceed 10%. Compounds 3 and 13 showed no weight loss throughout the study. Overall, the compounds of the present invention demonstrated good tolerability.

In conclusion, the compounds of the present invention exhibit excellent anti-tumor efficacy and are well-tolerated.

All references mentioned in the present disclosure are cited as references in this application, just as each reference is cited separately. In addition, it should be understood that after reading the above lecture content of the present disclosure, those skilled in the art can make various changes or modifications to the present disclosure, and these equivalent forms also fall within the scope of the claims attached to the present disclosure.

## Claims

1. A compound having a structure shown in Formula (1), or an isomer, a polymorph, a pharmaceutically acceptable salt, a hydrate, or a solvate thereof: 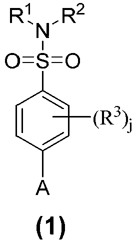 wherein:
A is selected from the group consisting of: : 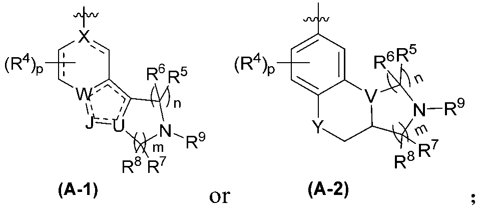
wherein, the dashed bond represents a chemical bond or absent; and 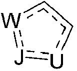 is an aromatic ring;
X is selected from the group consisting of: CH or N, when X is N, the dashed bond attached to X is absent;
U is selected from the group consisting of: CH or N;
W is selected from the group consisting of: CH or N;
J is selected from the group consisting of: O, S or NH;
V is selected from the group consisting of: CH or N;
Y is selected from the group consisting of: O, S, NH or N(C₁₋₃ alkyl);
m is 1 or 2;
n is 1 or 2;
p is 1, 2 or 3;
R⁴, R⁵, R⁶, R⁷ and R⁸ are each independently selected from the group consisting of: H, halogen, CN, C₁₋₆ alkyl, C₃₋₆ cycloalkyl, C₁₋₆ haloalkyl or C₁₋₆ alkoxyl;
R⁹ is selected from the group consisting of: H or C₁₋₃ alkyl;
j is 1, 2, 3 or 4;
R¹ is selected from the group consisting of: H, C₁₋₃ alkyl or C₁₋₃ haloalkyl;
R² is a 5-7-membered heteroaryl, the heteroaryl can be further substituted by 1, 2, or 3 R^{2a}, R^{2a} is selected from the group consisting of: cyano, NO₂, -OR^{a}, -C(=O)R^{a}, -NR^{c}R^{d}, - C(=O)NR^{c}R^{d}, -NR^{b}C(=O)NR^{c}R^{d}, -NR^{b}C(=O)R^{a}, C₁₋₆ alkyl, C₂₋₆ alkenyl, C₂₋₆ alkynyl, C₃₋₆ cycloalkyl, or 4-7-membered heterocycloalkyl; the alkyl, alkenyl, alkynyl, cycloalkyl, and heterocycloalkyl can be optionally substituted by one or more R^{2b};
R^{2b} is selected from the group consisting of: halogen, CN, NO₂, OH, SH, -OR^{a}, -NR^{c}R^{d}, -C(=O)NR^{c}R^{d}, -NR^{b}C(=O)NR^{c}R^{d}, -NR^{b}C(=O)R^{a}, -NR^{b}S(=O)²R^{a}, -C(=O)R^{a}, -OC(=O)R^{a}, - C(=O)OR^{b}, -NR^{b}C(=O)OR^{b}, -OC(=O)NR^{c}R^{d}, C₁₋₆ alkyl, C₂₋₆ alkenyl, C₂₋₆ alkynyl, C₁₋₆ alkoxyl, C₃₋₆ cycloalkyl or 4-7-membered heterocycloalkyl; the alkyl, alkenyl, alkynyl, alkoxy, cycloalkyl and heterocycloalkyl can be optionally substituted by one or more R;
or two R^{2b} substituents on the same atom jointly form =O;
R^{a} is independently selected from the group consisting of: C₁₋₆ alkyl, C₂₋₆ alkenyl, C₂₋₆ alkynyl, C₁₋₆ alkoxyl, C₁₋₆ haloalkyl, hydroxy-substituted C₁₋₆ alkyl, amino-substituted C₁₋₆ alkyl, C₃₋₆ cycloalkyl or 4-7-membered heterocycloalkyl; the alkyl, alkenyl, alkynyl, alkoxyl, cycloalkyl and heterocycloalkyl can be optionally substituted by one or more R;
R^{b} is independently selected from the group consisting of: H, C₁₋₆ alkyl, C₂₋₆ alkenyl, C₂₋₆ alkynyl, C₁₋₆ alkoxyl, C₁₋₆ haloalkyl, hydroxy-substituted C₁₋₆ alkyl, amino-substituted C₁₋₆ alkyl, C₃₋₆ cycloalkyl or 4-7-membered heterocycloalkyl; the alkyl, alkenyl, alkynyl, alkoxyl, cycloalkyl and heterocycloalkyl can be optionally substituted by one or more R;
R^{c} and R^{d} are each independently selected from the group consisting of: H, C₁₋₆ alkyl, C₂₋₆ alkenyl, C₂₋₆ alkynyl, C₁₋₆ alkoxyl, C₁₋₆ haloalkyl, hydroxy-substituted C₁₋₆ alkyl, amino-substituted C₁₋₆ alkyl, C₃₋₆ cycloalkyl or 4-7-membered heterocycloalkyl; the alkyl, alkenyl, alkynyl, alkoxyl, cycloalkyl and heterocycloalkyl can be optionally substituted by one or more R;
or R^{c} and R^{d} together with the atoms to which they are connected form a 4-7-membered heterocycloalkyl; the heterocycloalkyl can be optionally substituted by one or more R;
each R is independently selected from the group consisting of: halogen, CN, OH, NH₂, NHCH₃, N(CH₃)₂, -C(=O)NH₂, -C(=O)NHCH₃, -C(=O)N(CH₃)₂, C₁₋₆ alkyl, C₁₋₆ alkoxyl, C₁₋₆ haloalkyl, hydroxy-substituted C₁₋₆ alkyl, cyano-substituted C₁₋₆ alkyl, amino-substituted C₁₋₆ alkyl or C₃₋₆ cycloalkyl;
each R³ is independently selected from the group consisting of: H, halogen, CN, C₁₋₆ alkyl, C₃₋₆ cycloalkyl, C₁₋₆ haloalkyl or C₁₋₆ alkoxyl.

2. The compound according to claim 1, or an isomer, a polymorph, a pharmaceutically acceptable salt, a hydrate, or a solvate thereof, wherein, the compound of formula (1) has a structure shown in (2-1), (2-2) or (2-3) as follows: 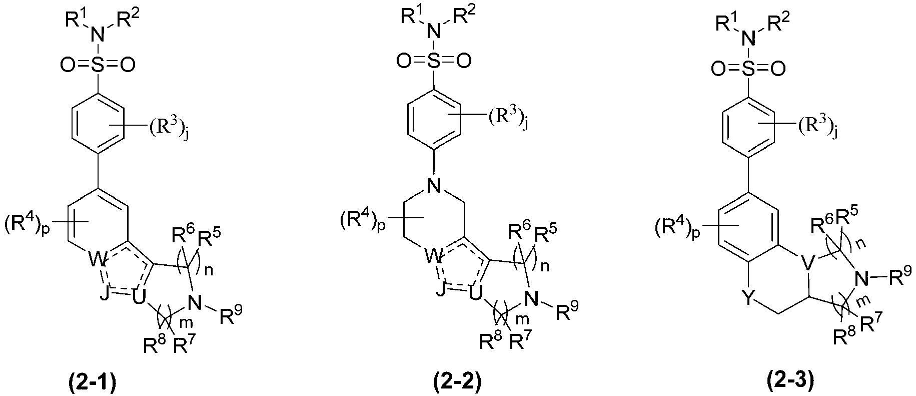 wherein, the definitions of each group are as described in claim 1.

3. The compound according to claim 1-2, or an isomer, a polymorph, a pharmaceutically acceptable salt, a hydrate, or a solvate thereof, wherein, R¹ selected from the group consisting of: H, Me, Et, ⁱ⁻Pr, CHF₂ or CH₂CF₃.

4. The compound according to claim 1-2, or an isomer, a polymorph, a pharmaceutically acceptable salt, a hydrate, or a solvate thereof, wherein R² is 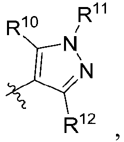 wherein, R¹⁰ is selected from H or C₁₋₆ alkyl, R¹¹ is selected from H or C₁₋₆ alkyl, R¹² is the R^{2a} as described in claim 1.

5. The compound according to claim 1-2, or an isomer, a polymorph, a pharmaceutically acceptable salt, a hydrate, or a solvate thereof, wherein, R² is 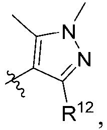 wherein R¹² is the R^{2a} as described in claim 1.

6. The compound according to claim 1-2, or an isomer, a polymorph, a pharmaceutically acceptable salt, a hydrate, or a solvate thereof, wherein, R² is selected from the group consisting of:

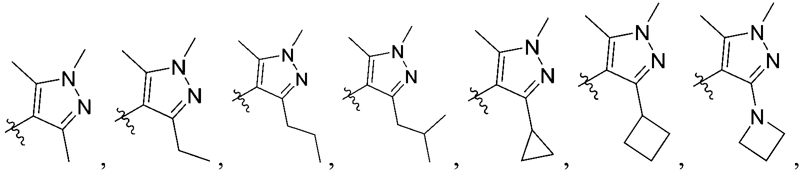

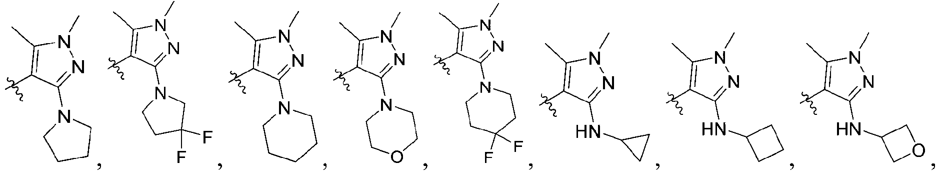

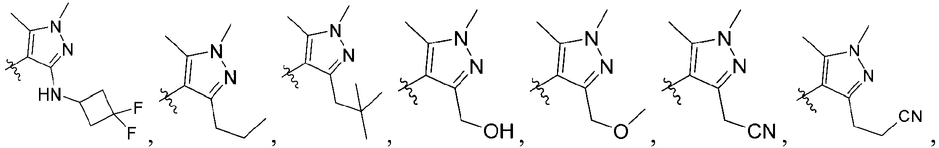

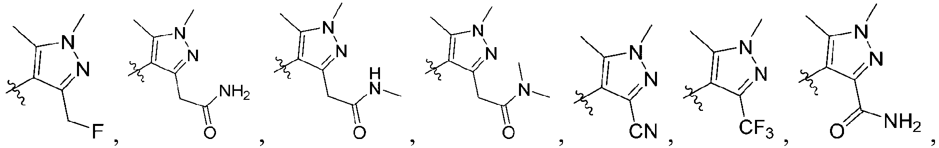

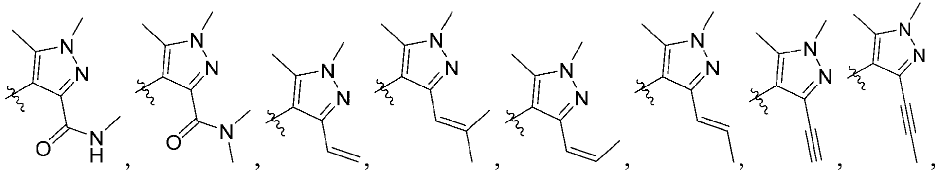

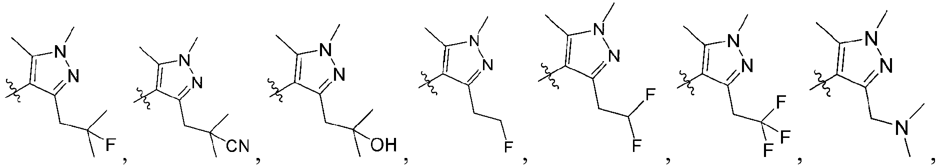

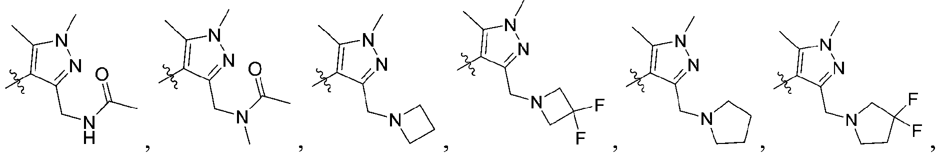

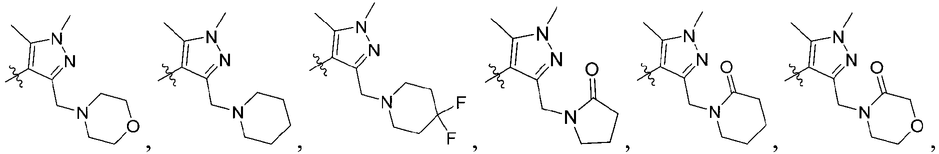

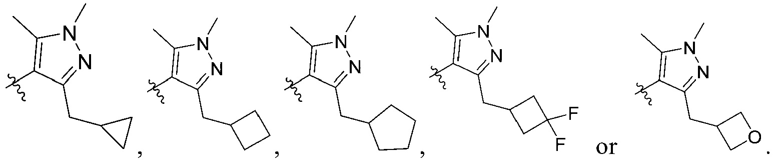

7. The compound according to claim 1-2, or an isomer, a polymorph, a pharmaceutically acceptable salt, a hydrate, or a solvate thereof, wherein, R³, R⁴, R⁵, R⁶, R⁷ and R⁸ are each independently selected from the group consisting of: H, halogen, CN, Me, Et, CF₃, cyclopropyl, OMe, OEt or OCF₃.

8. The compound according to claim 1-2, or an isomer, a polymorph, a pharmaceutically acceptable salt, a hydrate, or a solvate thereof, wherein, R⁹ is selected from the group consisting of: H or Me.

9. The compound according to claim 1-2, an isomer, a polymorph, or a pharmaceutically acceptable salt thereof, wherein the compound has a structure selected from the group consisting of:

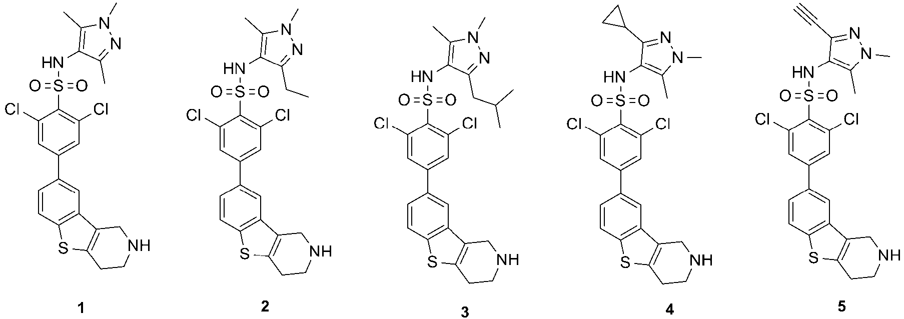

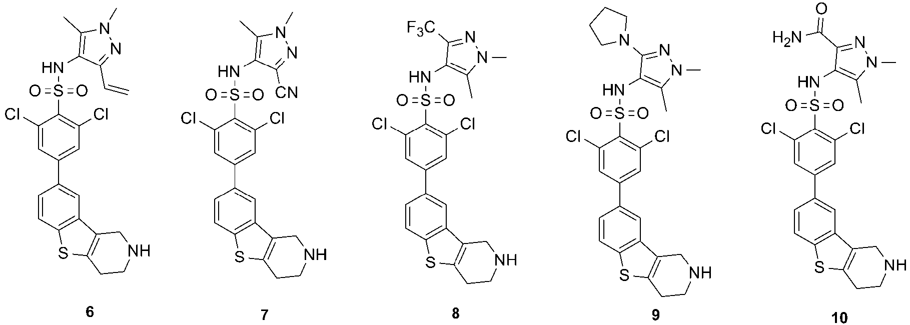

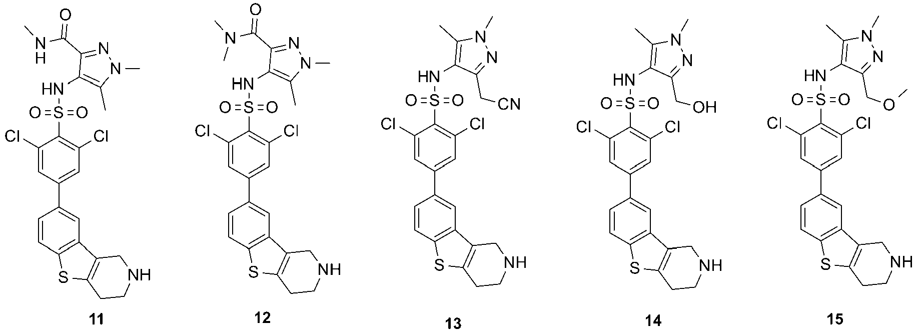

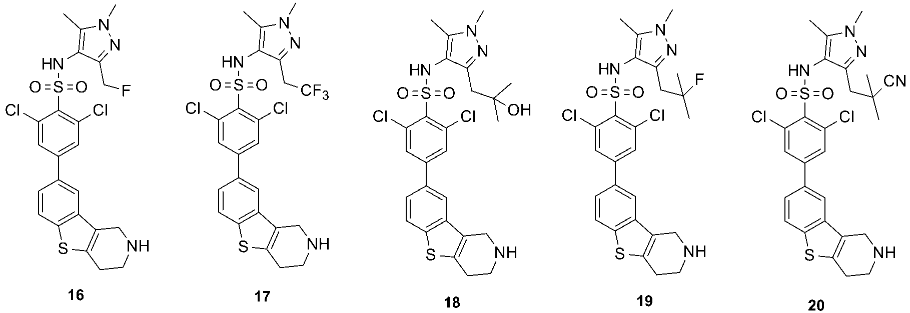

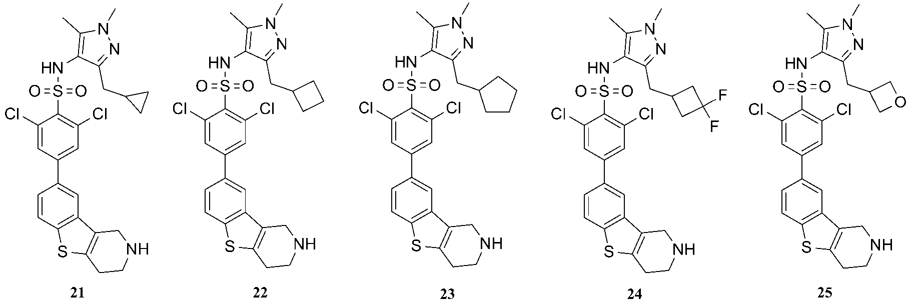

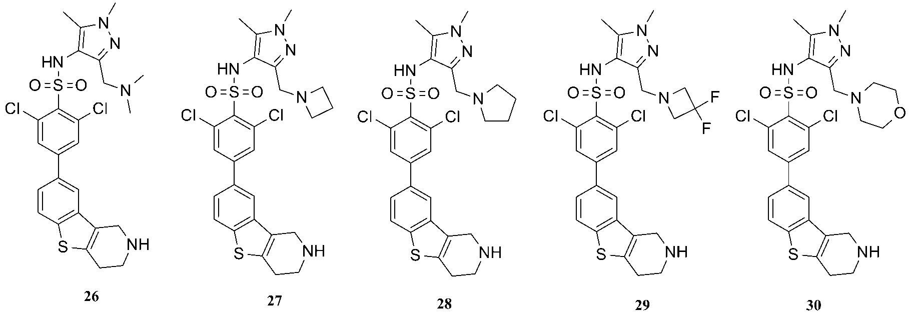

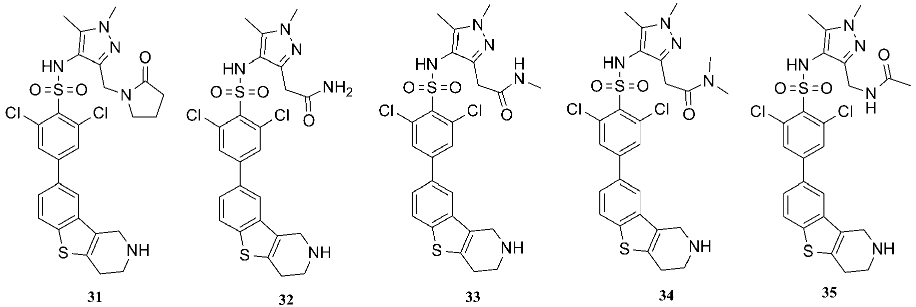

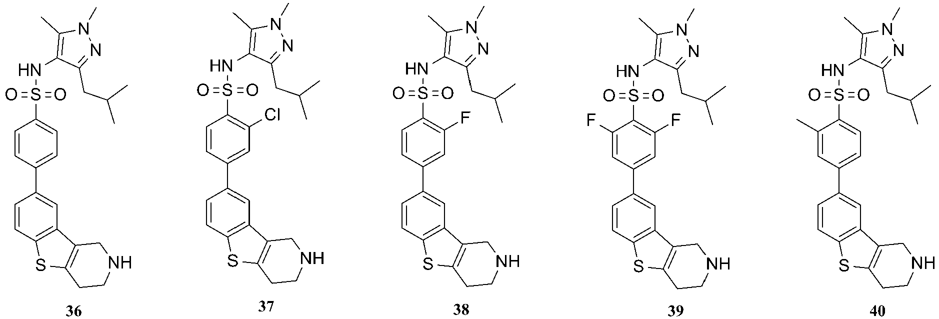

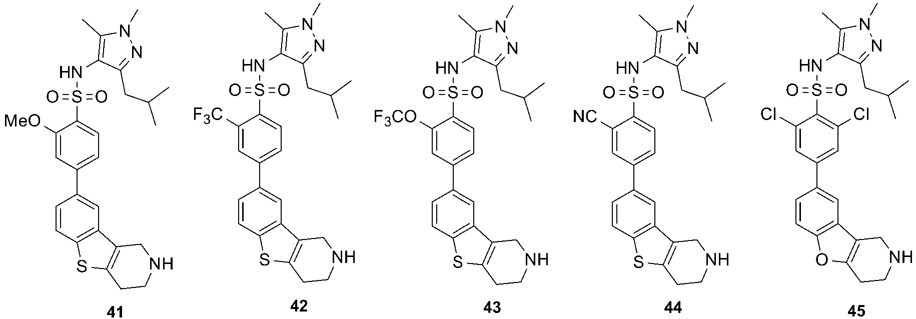

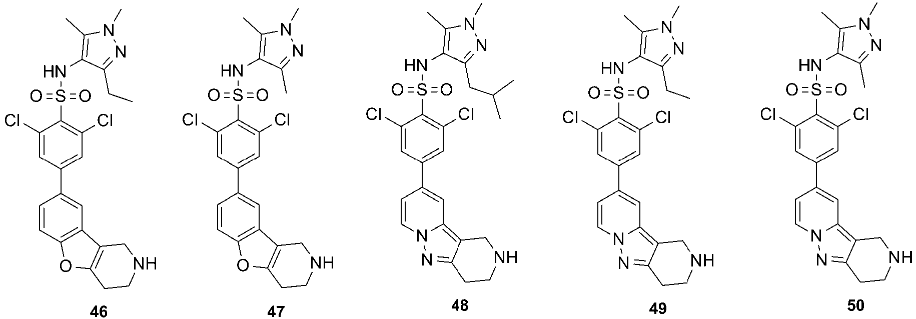

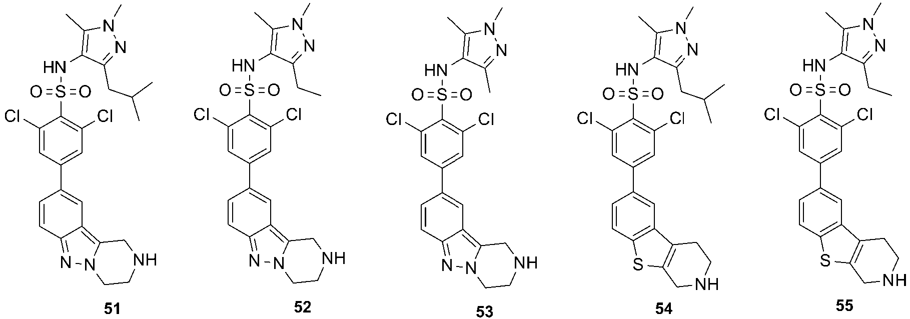

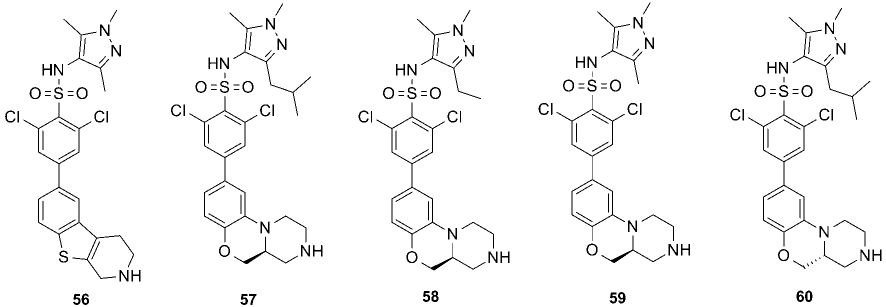

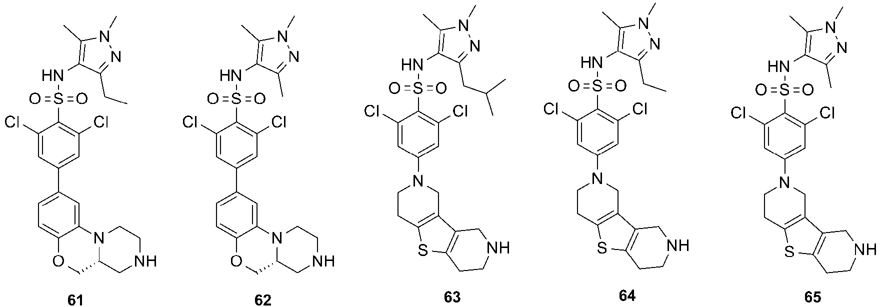

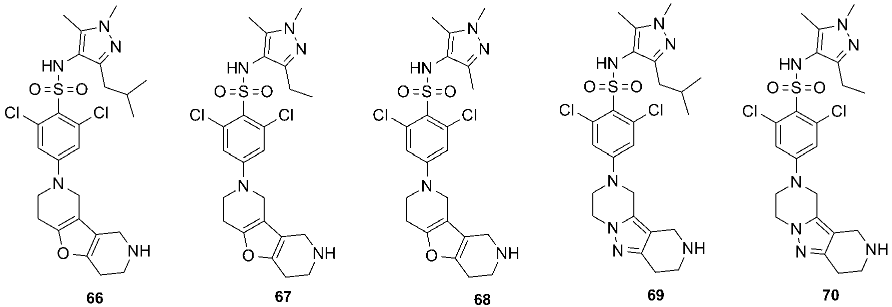

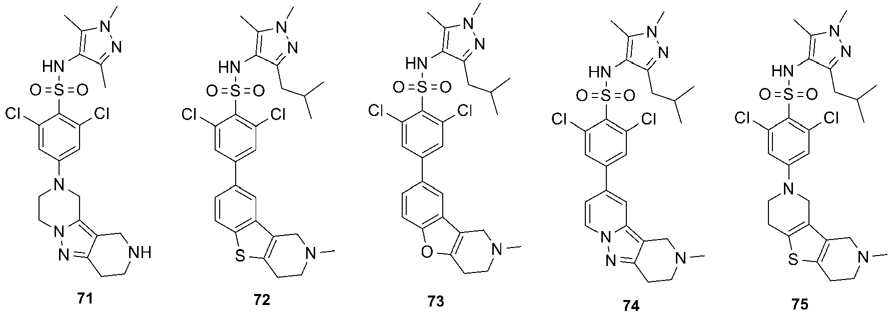

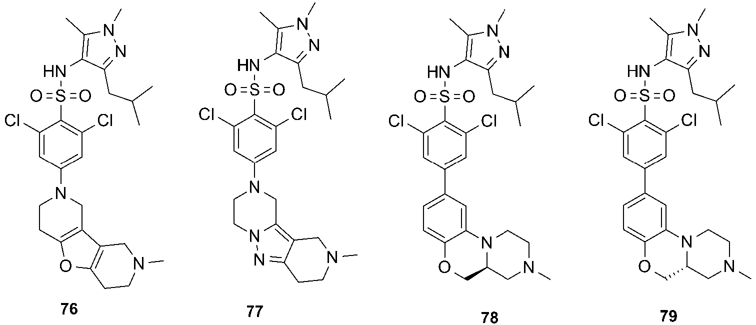

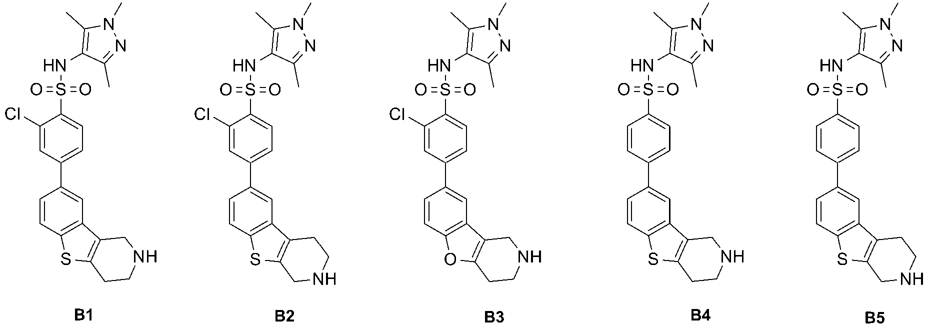

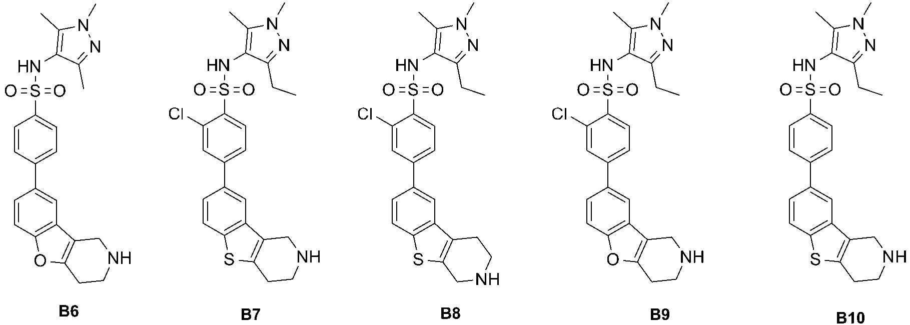

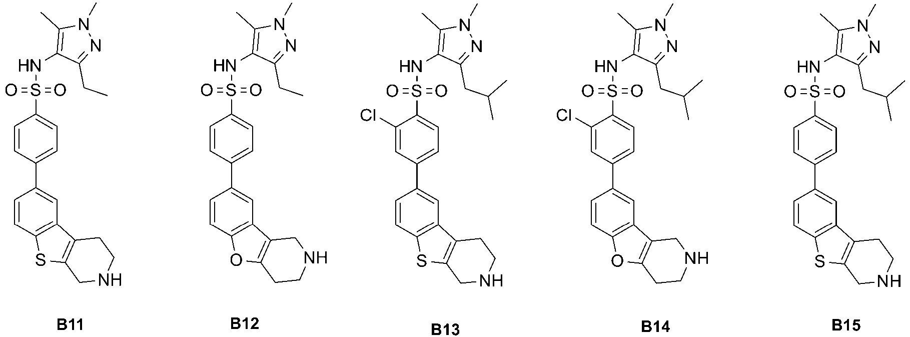

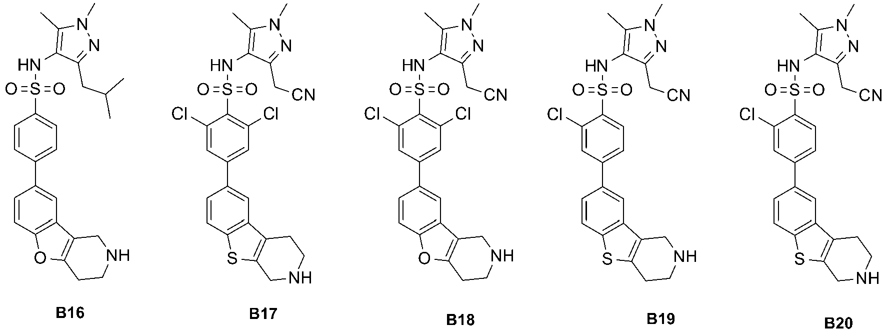

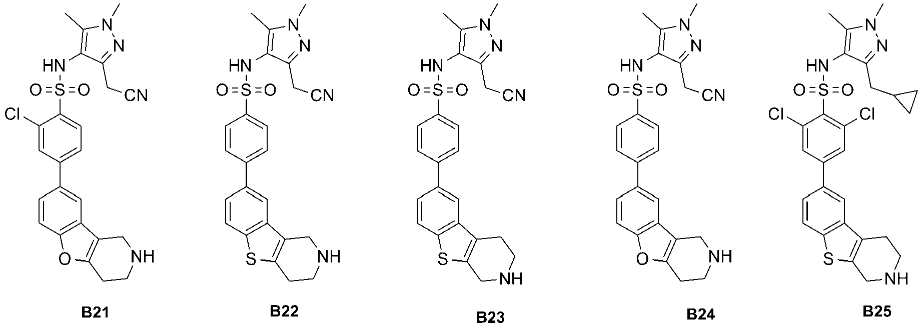

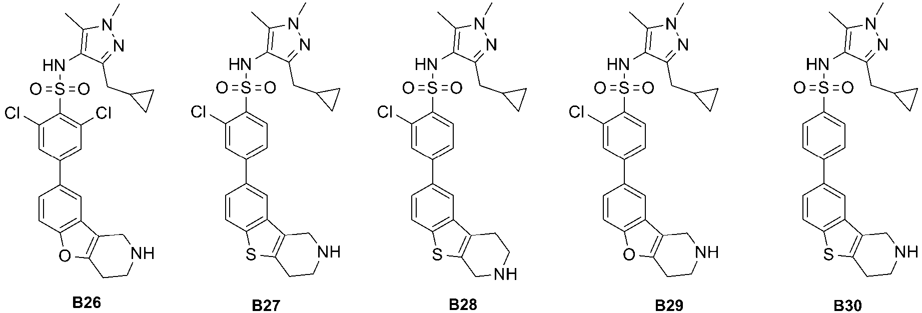

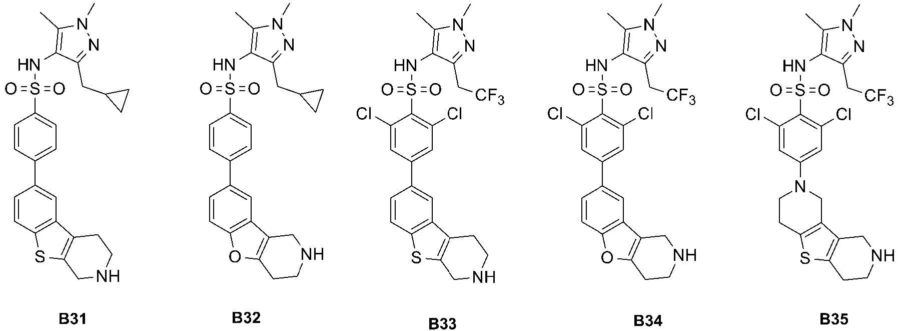

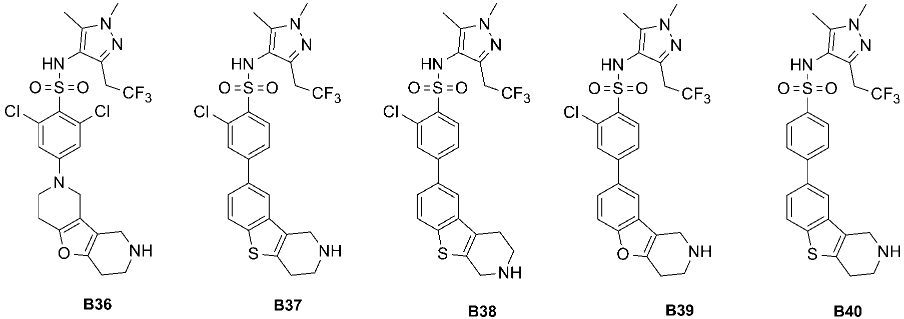

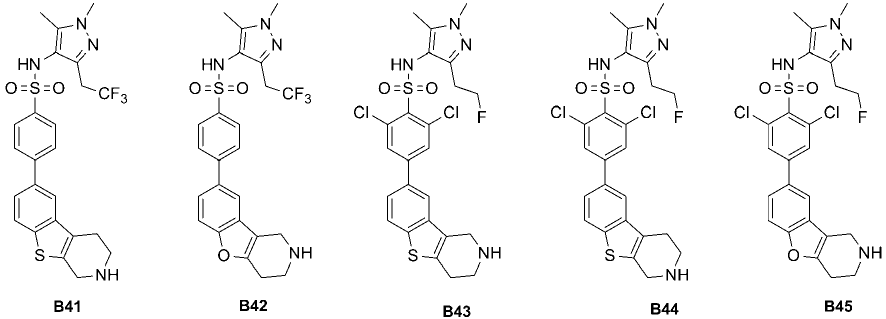

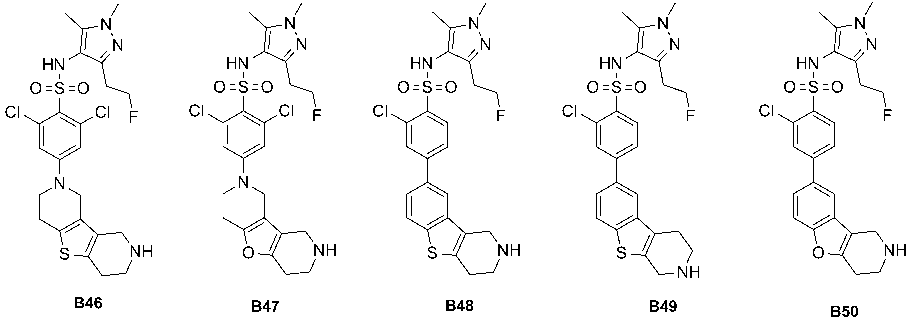

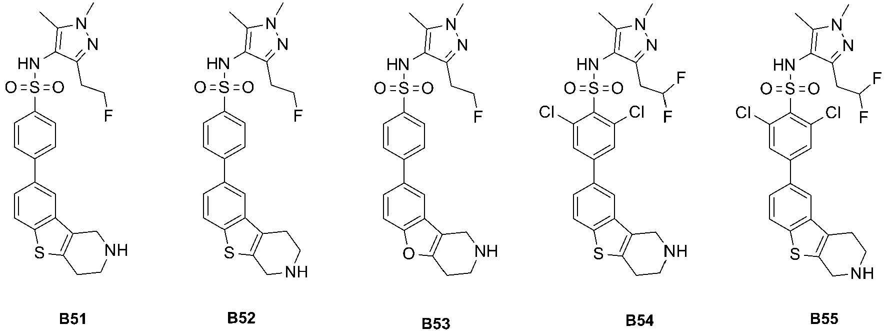

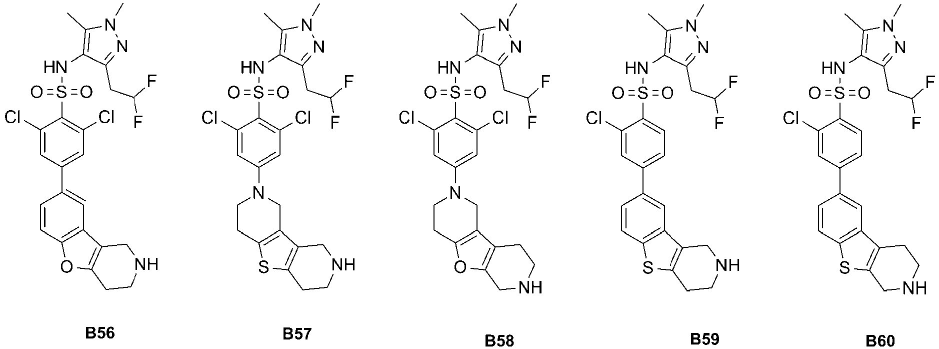

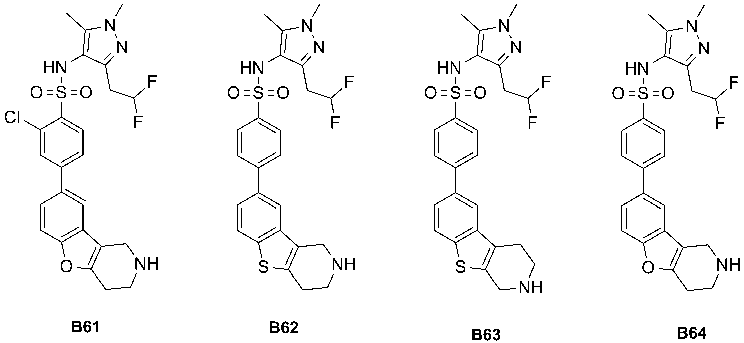

10. A pharmaceutical composition for treating, regulating, and/or preventing a NMT-mediated related disease, wherein, the pharmaceutical composition comprises a pharmaceutically acceptable excipient or carrier, and the compound as an active ingredient according to any one of claims 1-9, or an isomer, a polymorph, a pharmaceutically acceptable salt, a hydrate, or a solvate thereof.

11. Use of the compound according to any one of claims 1-9, or an isomer, a polymorph, a pharmaceutically acceptable salt, a hydrate or a solvate thereof, or the pharmaceutical composition according to claim 10, in preparing a pharmaceutical composition for treating, regulating and/or preventing a NMT-mediated related disease.

12. A method for treating, regulating, and/or preventing a NMT-mediated related disease, comprising the steps of: administering to an individual in need with a compound according to any one of claims 1-9, or an isomer, a polymorph, a pharmaceutically acceptable salt, a hydrate, or a solvate, or a pharmaceutical composition according to claim 10.
